(19) Europäisches Patentamt
European Patent Office
Office européen des brevets

(11) Veröffentlichungsnummer: **0 592 791 A2**

(12) # EUROPÄISCHE PATENTANMELDUNG

(21) Anmeldenummer: **93113311.0**

(22) Anmeldetag: **20.08.93**

(51) Int. Cl.[5]: **C07K 5/06**, C07K 5/08, C07K 5/10, C07K 7/06, C07D 265/38, C07D 279/22, C07D 279/30, C07D 311/82, C07D 333/12, A61K 37/02, A61K 31/33

(30) Priorität: **31.08.92 CH 2725/92**

(43) Veröffentlichungstag der Anmeldung: **20.04.94 Patentblatt 94/16**

(84) Benannte Vertragsstaaten: **AT BE CH DE DK ES FR GB GR IE IT LI LU MC NL PT SE**

(71) Anmelder: **F. HOFFMANN-LA ROCHE AG**
**Postfach 3255**
**CH-4002 Basel(CH)**

(72) Erfinder: **Bannwarth, Wilhelm**
**36 Parker Place**
**Upper Saddle River, NJ 07458(US)**
Erfinder: **Gerber, Fernand**
**10 rue du chêne**
**F-68680 Niffer(FR)**
Erfinder: **Grieder, Alfred**
**Heidengässli 2**
**CH-4450 Sissach(CH)**
Erfinder: **Knierzinger, Andreas**
**Burenweg 30**
**CH-4127 Birsfelden(CH)**
Erfinder: **Müller, Klaus**
**Grellingerstrasse 5**
**CH-4142 Münchenstein(CH)**
Erfinder: **Obrecht, Daniel**
**Wasgenring 101**
**CH-4055 Basel(CH)**
Erfinder: **Trzeciak, Arnold**
**Talstrasse 54**
**D-7860 Schopfheim(DE)**

(74) Vertreter: **Bertschinger, Christoph, Dr. et al**
**Grenzacherstrasse 124**
**P.O. Box 3255**
**CH-4002 Basel (CH)**

(54) **Tricyclische Verbindungen und deren Peptid Derivate.**

(57) Beschrieben werden Verbindungen der Formel

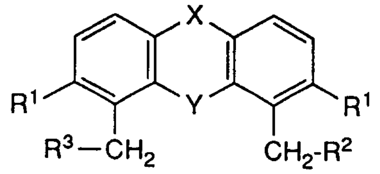

I

worin X eine Gruppe der Formel

EP 0 592 791 A2

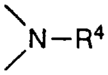

(a)

oder

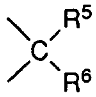

(b)

;

Y Sauerstoff oder Schwefel;

$R^1$ Wasserstoff oder niederes Alkoxy;

$R^2$ geschütztes Amino, Amino oder einen Rest der Formel -NH-$R^7$ (c);

$R^3$ Carboxyl, funktionell abgewandeltes Carboxyl oder einen Rest der Formel -CO-$R^8$ (d);

$R^4$ niederes Alkyl, Aryl, Aryl-niederes Alkyl, Wasserstoff oder Acyl;

$R^5$ und $R^6$ je niederes Alkyl, Aryl oder Aryl-niederes Alkyl; und

$R^7$ und $R^8$ je einzeln oder zusammen einen Rest einer Aminosäure oder eine Kette von bis zu 20 Aminosäureresten bedeuten, wobei der Aminosäurerest bzw. die Aminosäurereste geschützt sein kann bzw. sein können und wobei das Molekül insgesamt höchstens 20 Aminosäurereste enthält,
sowie Salze davon.

Diejenigen Verbindungen der Formel I und deren Salze , worin $R^2$ einen Rest der Formel (c), $R^3$ einen Rest der Formel (d) und $R^7$ und $R^8$ zusammen einen Rest einer Aminosäure oder eine Kette von bis zu 20 Aminosäureresten bedeuten, insbesondere solche, worin der Aminosäurerest bzw. die Kette von Aminosäureresten keine Schutzgruppe(n) enthält, sind wertvolle Hilfsmittel ("Research Tools"), um biologisch aktive Peptidsequenzen zu ermitteln ; sie sind aber auch potentiell als Heilmittel geeignet. Die übrigen Verbindungen der Formel I und deren Salze sind wertvolle Zwischenprodukte.

Die vorliegende Erfindung betrifft tri- und tetracylische Verbindungen, und zwar in erster Linie Verbindungen der allgemeinen Formel

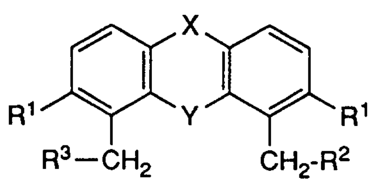

I

worin X eine Gruppe der Formel

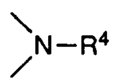

oder

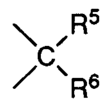

;

(a) (b)

Y Sauerstoff oder Schwefel;

$R^1$ Wasserstoff oder niederes Alkoxy;

$R^2$ geschütztes Amino, Amino oder einen Rest der Formel $-NH-R^7$ (c);

$R^3$ Carboxyl, funktionell abgewandeltes Carboxyl oder einen Rest der Formel $-CO-R^8$ (d);

$R^4$ niederes Alkyl, Aryl, Aryl-niederes Alkyl, Wasserstoff oder Acyl;

$R^5$ und $R^6$ je niederes Alkyl, Aryl oder Aryl-niederes Alkyl; und

$R^7$ und $R^8$ je einzeln oder zusammen einen Rest einer Aminosäure oder eine Kette von bis zu 20 Aminosäureresten bedeuten, wobei der Aminosäurerest bzw. die Aminosäurereste geschützt sein kann bzw. sein können und wobei das Molekül insgesamt höchstens 20 Aminosäurereste enthält,
sowie Salze davon.

Die Verbindungen der allgemeinen Formel I und deren Salze sind neu. Diejenigen, worin $R^2$ einen Rest der Formel (c), $R^3$ einen Rest der Formel (d) und $R^7$ und $R^8$ zusammen einen Rest einer Aminosäure oder eine Kette von bis zu 20 Aminosäureresten bedeuten, d.h. Verbindungen der allgemeinen Formel,

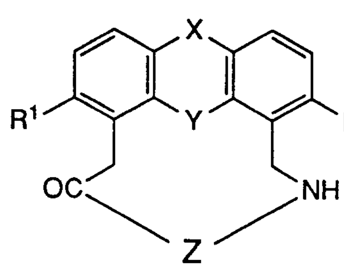

Ia

worin X, Y und $R^1$ obige Bedeutung besitzen und Z einen Rest einer Aminosäure oder eine Kette von bis zu 20 Aminosäureresten bedeutet,
und Salze davon, insbesondere solche, worin der Aminosäurerest bzw. die Kette von Aminosäureresten keine Schutzgruppe(n) enthält, sind wertvolle Hilfsmittel, um biologisch aktive Peptidsequenzen zu ermitteln und stellen somit sogenannte "Research Tools" dar; sie sind aber auch potentiell als Heilmittel geeignet. Die übrigen Verbindungen der Formel I und deren Salze sind wertvolle Zwischenprodukte.

Gegenstand der vorliegenden Erfindung sind die Verbindungen der allgemeinen Formel I und Salze davon als solche, deren Herstellung und Zwischenprodukte zu deren Herstellung, ferner die Verwendung von Verbindungen der allgemeinen Formel Ia und von Salzen davon als Research Tools und als Arzneimittel bzw. zur Herstellung von Arzeimitteln, weiterhin Verbindungen der allgemeinen Formel Ia und Salze davon zur Anwendung als therapeutische Wirkstoffe, sowie Arzneimittel, enthaltend eine Verbindung der allgemeinen Formel Ia oder ein Salz davon und die Herstellung solcher Arzneimittel.

Der Ausdruck " niederes Alkyl" umfasst geradkettige oder verzweigte gesättigte Kohlenwasserstoffreste mit bis zu 7, vorzugsweise bis zu 4 Kohlenstoffatomen, wie Methyl, Aethyl, n-Propyl, Isopropyl, n-Butyl, Isobutyl, tert. Butyl u. dgl. Der Ausdruck "niederes Alkoxy" umfasst Alkyloxygruppen im Sinne der obigen Umschreibung des Begriffs "niederes Alkyl". Der Ausdruck"Aryl" umfasst den Phenylrest und substituierte Phenylreste, insbesondere mono- oder disubstituierte Phenylreste, wobei als Substituenten in erster Linie niedere Alkyl- oder Alkoxygruppen oder Halogenatome in Frage kommen. Der Ausdruck "Halogen" bezeichnet die vier Formen Fluor, Chlor, Brom, und Jod, sofern nicht ausdrücklich anders angegeben. Der Ausdruck"Acyl" umfasst Reste von aliphatischen und aromatischen Carbonsäuren, in erster Linie einerseits niedere Alkanoylgruppen, wie Acetyl, Propionyl, Butyryl o. dgl., welche substituiert sein können, beispielsweise durch Carboxy oder niederes Alkoxycarbonyl, wie dies z.B. in 4-Carboxybutyryl, 4-Methoxycarbonylbutyryl o. dgl. der Fall ist, und andererseits die Benzoylgruppe und substituierte Benzoylgruppen, insbesondere mono- oder disubstituierte Benzoylgruppen, wobei als Substituenten in erster Linie niedere Alkyl- oder Alkoxygruppen oder Halogenatome in Frage kommen. Der Ausdruck "funktionell abgewandeltes Carboxyl" umfasst Reste der Formel $-COOR^9$ (e), worin $R^9$ niederes Alkyl, substituiertes niederes Alkyl, Aryl-niederes Alkyl, Aroyl-niederes Alkyl oder Allyl bedeuten kann; zweckmässigerweise bedeutet $R^9$ Methyl, tert.-Butyl, Phenacyl, Trimethylsilyläthyl, Trichloräthyl, Phenyl, Pentafluorphenyl, Benzyl, Allyl, o. dgl. Der Ausdruck "geschütztes Amino" umfasst einerseits Reste wie Phthalimido u. dgl. und anderseits Reste der Formel $-NH-R^{10}$ (f), worin $R^{10}$ Benzyloxycarbonyl ("Z"), tert.-Butyloxycarbonyl ("Boc"), 9-Fluorenylmethoxy-carbonyl ("Fmoc"), Allyloxycarbonyl("Alloc"), Trimethylsilyläthoxycarbonyl ("Teoc"), Trichloräthoxycarbonyl ("Tcc"), o-Nitrophenylsulfenyl ("Nps") u. dgl. bedeuten kann.

Als Aminosäurereste kommen in erster Linie solche in Betracht, welche sich von α-Aminosäuren ableiten, insbesondere von natürlichen α-Aminosäuren; die Aminosäurereste können nicht nur in der L-Form, sondern auch in der D- Form vorliegen, und sie können gegebenenfalls geschützt sein. Nachstehend folgt eine Liste von Aminosäuren, welche bzw. deren Reste für die Zwecke der vorliegenden Erfindung geeignet sind, wobei die Abkürzungen den einschlägigen IUPAC-Regeln (Biochemistry 11, 1726 (1972)) und den allgemeinen üblichen Gepflogenheiten entsprechen.

| | |
|---|---|
| $Ac_3c$ | 1-Aminocyclopropancarbonsäure |
| $Ac_4c$ | 1-Aminocyclobutancarbonsäure |
| $Ac_5c$ | 1-Aminocyclopentancarbonsäure |
| $Ac_6c$ | 1-Aminocyclohexancarbonsäure |
| $Ac_7c$ | 1-Aminocycloheptancarbonsäure |
| Aib | 2-Amino-2-methylpropionsäure |
| Ala | L-Alanin |
| D-Ala | D-Alanin |
| ß-Ala | ß-Alanin |
| Arg | L-Arginin |
| D-Arg | D-Arginin |
| Asn | L-Asparagin |
| D-Asn | D-Asparagin |
| Asp | L-Asparaginsäure |
| D-Asp | D-Asparaginsäure |
| D-Asp (ONa) | Natrium-D-aspartat |
| $C_3al$ | L-3-cyclopropyalanin |
| $C_4al$ | L-3-cyclobutylalanin |
| $C_5al$ | L-3-cyclopentylalanin |
| $C_6al$ | L-3-cyclohexylalanin |
| Cys | L-Cystein |
| D-Cys | D-Cystein |
| Glu | L-Glutaminsäure |
| D-Glu | D-Glutaminsäure |
| Gln | L-Glutamin |
| D-Gln | D-Glutamin |
| Gly | Glycin |
| His | L-Histidin |
| D-His | D-Histidin |
| Hyp | 4-Hydroxy-L-prolin |
| Ile | L-Isoleucin |
| alle | L-Alloisoleucin |

| | |
|---|---|
| D-Ile | D-Isoleucin |
| D-alle | D-Alloisoleucin |
| D-Itg | D-2-(Isothiazolyl)glycin |
| Leu | L-Leucin |
| D-Leu | D-Leucin |
| tert.-Leu | L-2-Amino-3,3-dimethylbuttersäure |
| D-tert.-Leu | D-2-Amino-3,3-dimethylbuttersäure |
| Lys | L-Lysin |
| D-Lys | D-Lysin |
| Lys (CHO) | $N^6$-Formyl-L-lysin |
| MeAla | N-Methyl-L-alanin |
| MeLeu | N-Methyl-L-leucin |
| MeMet | N-Methyl-L-methionin |
| Met | L-Methionin |
| D-Met | D-Methionin |
| Met(O) | L-Methionin-sulfoxid |
| D-Met(O) | D-Methionin-sulfoxid |
| $Met(O_2)$ | L-Methionin-sulfon |
| $D-Met(O_2)$ | D-Methionin-sulfon |
| Nal | L-3-(1-Naphthylalanin) |
| D-Nal | D-3-(1-Naphthylalanin) |
| Nle | L-Norleucin |
| D-Nle | D-Norleucin |
| Nva | L-Norvalin |
| D-Nva | D-Norvalin |
| Orn | L-Ornithin |
| D-Orn | D-Ornithin |
| Orn(CHO) | $N^5$-Formyl-L-ornithin |
| Phe | L-Phenylalanin |
| D-Phe | D-Phenylalanin |
| L-Phg | L-Phenylglycin |
| D-Phg | D-Phenylglycin |
| Pip | L-Pipecolinsäure |
| D-Pip | D-Pipecolinsäure |
| Pro | L-Prolin |
| D-Pro | D-Prolin |
| Sar | Sarcosin |
| Ser | L-Serin |
| D-Ser | D-Serin |
| Thr | L-Threonin |
| D-Thr | D-Threonin |
| Thz | L-Thiazolidin-4-carbonsäure |
| D-Thz | D-Thiazolidin-4-carbonsäure |
| Trp | L-Tryptophan |
| D-Trp | D-Tryptophan |
| D-Trp(CHO) | $N^{in}$-Formyl-D-tryptophan |
| D-Trp(O) | D-3-(2,3-Dihydro-2-oxoindol-3-yl)alanin |
| Tyr | L-Tyrosin |
| D-Tyr | D-Tyrosin |
| Tza | L-3-(2-Thiazolyl)alanin |
| D-Tza | D-3-(2-Thiazolyl)alanin |
| Tzg | L-2-(Thiazolyl)glycin |
| D-Tzg | D-2-(Thiazolyl)glycin |
| Val | L-Valin |
| D-Val | D-Valin |

Geeignete Schutzgruppe für Aminosäuren bzw. deren Reste sind beispielsweise

- für die Aminogruppe (wie sie z.B. auch in der Seitenkette von Lysin vorliegt)

| | |
|---|---|
| Z | Benzyloxycarbonyl |

Boc tert.- Butyloxycarbonyl
Fmoc 9-Fluorenylmethoxycarbonyl
Alloc Allyloxycarbonyl
Teoc Trimethylsilyläthoxycarbonyl
Tcc Trichloräthoxycarbonyl
Nps o-Nitrophenylsulfenyl;

- für die Carbonylgruppe (wie sie z.B. auch in der Seitenkette von Asparginsäure und Glutaminsäure vorliegt) durch Ueberführung in entsprechende Ester mit den Alkoholkomponenten
  tBu tert.-Butyl
  Bzl Benzyl
  Me Methyl
  Ph Phenyl
  Pac Phenacyl
  Allyl
  Trimethylsilyläthyl
  Trichloräthyl;
- für die Guanidingruppe (wie sie beispielsweise in der Seitenkette von Arginin vorliegt)
  Pmc 2,2,5,7,8,-Pentamethylchroman-6-sulfonyl
  Ts Tosyl
  Z Benzyloxycarbonyl;
- für die Hydroxygruppe (wie sie beispielsweise in der Seitenkette von Threonin und Serin vorliegt)
  tBu tert.-Butyl
  Bzl Benzyl
  Trityl;
- und für die Mercaptogruppe (wie sie beispielsweise in der Seitenkette von Cystein vorliegt)
  tBu tert.-Butyl
  Bzl Benzyl
  Trityl
  2-Methoxytrityl.

Wenn in Formel I oder Ia X eine Gruppe der Formel (a) bedeutet, dann bedeutet $R^4$ zweckmässigerweise Methyl, Aethyl, Hexyl, Benzyl, Wasserstoff, 4-Methoxycarbonylbutyryl oder 4-Carboxybutyryl; im Falle, dass $R^4$ eine Carboxylgruppe enthält, bedeutet $R^3$ in Formel I zweckmässigerweise nicht Carboxyl. Wenn X in Formel I oder Ia eine Gruppe der Formel (b) bedeutet, dann bedeuten $R^5$ und $R^6$ zweckmässigerweise beide Methyl.

Die beiden Symbole $R^1$ in Formel I und Ia bedeuten zweckmässigerweise beide Methoxy oder beide Aethoxy.

In Formel Ia kann das Symbol Z zweckmässigerweise bis zu 14 Aminosäurereste enthalten und beispielsweise folgende Bedeutungen haben:

-Arg-

-Ala-

-Ala-Ala-

-Arg-Gln-

-Arg-Ser-

-Gln-Arg-

-Glu-Arg-

-Lys-Glu-

-Ser-Arg-

-Thr-Gly-

-Tyr-Phe-

-Leu-D-Try-D-Asp--

-Gly-Arg-Gly-

-Ile-Tyr-Ala-

-Leu-Tyr-Asp-

-Ala-Thr-Val-Gly-

-Arg-Gly-Asp-Val-

-Gly-Asp-Gly-Gly-

-Gly-Gly-Ala-Gly-

-Val-Arg-Lys-Lys-

-Ala-Arg-Gly-Asp-Phe-Pro-

-Glu-Arg-Gly-Asp-Val-Tyr-

-Ile-Ala-Arg-Gly-Asp-Phe-Pro-Asp-

-Val-Ala-Ala-Phe-Leu-Ala-Leu-Ala-

-Arg-Ile-Ala-Arg-Gly-Asp-Phe-Pro-Asp-Asp-

-Ala-Arg-Ile-Ala-Arg-Gly-Asp-Phe-Pro-Asp-Asp-Arg-

Im Rahmen der vorliegenden Erfindung besonders bevorzugte Verbindungen der Formel Ia sind:

4,5-Cyclo[-acetyl-L-alanyl-L-arginyl-L-isoleucyl-L-alanyl-L-arginylglycyl-L-aspartyl-L-phenylalanyl-L-prolyl-L-aspartyl-L-aspartyl-L-arginylaminomethyl-]-3,6-dimethoxy-9,9-dimethylxanthen;

4,5-Cyclo[-acetyl-L-arginyl-L-isoleucyl-L-alanyl-L-arginyl-glycyl-L-aspartyl-L-phenylalanyl-L-prolyl-L-aspartyl-L-aspartylaminomethyl-]-3,6-dimethoxy-9,9-dimethyl-xanthen;

4,5-Cyclo[-acetyl-L-isoleucyl-L-alanyl-L-arginyl-glycyl-L-aspartyl-L-phenylalanyl-L-prolyl-L-aspartylaminomethyl-]-3,6-dimethoxy-9,9-dimethylxanthen;

4,5-Cyclo[-acetyl-L-alanyl-L-arginyl-glycyl-L-aspartyl-L-phenylalanyl-L-prolylaminomethyl-]-3,6-dimethoxy-9,9-dimethylxanthen;

4,5-Cyclo[-acetyl-L-arginyl-glycyl-L-aspartyl-L-phenylalanylaminomethyl-]-3,6-dimethoxy-9,9-dimethylxanthen;

4,5-Cyclo[-acetyl-L-arginyl-glycyl-L-aspartyl-L-valylaminomethyl-]-3,6-dimethoxy-9,9-dimethylxanthen;

10-Methyl-4,6-cyclo[-acetyl-L-arginyl-glycyl-L-aspartyl-L-valylaminomethyl-]phenothiazin;

4,6-Cyclo[-acetyl-L-arginyl-glycyl-L-aspartyl-L-valylaminomethyl-] -3,7-diäthoxy-10-aethyl-10H-dibenz-[b,e][1,4] oxazin;

10-Hexyl-3,7-dimethoxy-4,6-cyclo[-acetyl-L-arginyl-glycyl-L-asparty-L-valylaminomethyl-]phenothiazin; und

3,7-Dimethoxy-10-methyl-4,6-cyclo[-acetyl-L-arginyl-glycyl-L-asparty-L-valylaminomethyl-]phenothiazin.

Weitere bevorzugte Verbindungen der Formel Ia sind:

(S)-4,12-Dimethoxy-8-(3-guanidinopropyl)-17-methyl-1,15-imino-6,7,8,9,10,11-hexahydro-5H-dibenzol-[b,k][1,5,8]thiadiazacyclodecin-7,10-dion;

(S)-8-(3-Guanidinopropyl)-17-hexyl-1,15,imino-6,7,8,9,10,11-hexahydro-5H-dibenzo[b,k][1,5,8]-

thiadiazacyclodecin-7,10-dion;

(S)-8-(3-Guanidinopropyl)-17-methyl-1,15-imino-6,7,8,9,10,11-hexahydro-5H-dibenzo[b,k][1,5,8]-thiadiazacylodecin-7,10-dion;

10-Hexyl-3,7-dimethoxy-4,6-cyclo[-acetyl-L-glutaminyl-L-arginylamino-methyl-]phenothiazin;

10-Hexyl-3,7-dimethoxy-4,6-cyclo[acetyl-L-arginyl-L-glutaminylaminomethyl-]phenothiazin;

10-Hexyl-3,7-dimethoxy-4,6-cyclo[-acetyl-L-seryl-L-arginylaminomethyl]-phenothiazin;

10-Hexyl-3,7-dimethoxy-4,6-cyclo[-acetyl-L-arginyl-L-serylaminomethyl]-phenothiazin; und

10-Hexyl-3,7-dimethoxy-4,6-cyclo[acetyl-glycyl-L-arginylglycylaminomethyl-]phenothiazin.

Die Verbindungen der allgemeinen Formel I und deren Salze können erfindungsgemäss dadurch hergestellt werden, dass man

a) eine Verbindung der allgemeinen Formel

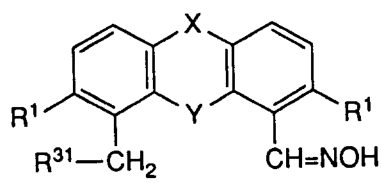

II

worin X,Y und $R^1$ obige Bedeutung besitzen und $R^{31}$ Carboxyl oder funktionell abgewandeltes Carboxyl bedeutet,

reduziert; oder

b) eine Verbindung der allgemeinen Formel

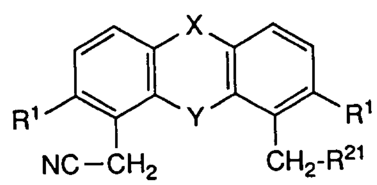

III

worin X,Y und $R^1$ obige Bedeutung besitzen und $R^{21}$ geschütztes Amino oder Amino bedeutet,

hydrolysiert; oder

c) in einer Verbindung der allgemeinen Formel

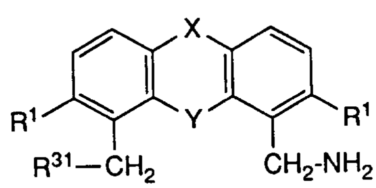

Ib

worin X,Y,$R^1$ und $R^{31}$ obige Bedeutung besitzen,

die Aminogruppe in eine geschützte Aminogruppe überführt; oder

d) in einer Verbindung der allgemeinen Formel

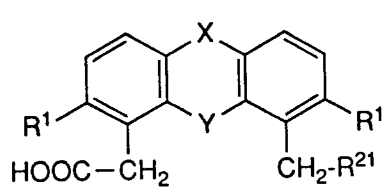

Ic

worin X,Y,$R^1$ und $R^{21}$ obige Bedeutung besitzen,

die Carboxylgruppe in eine funktionell abgewandelte Carboxylgruppe überführt; oder

e) in einer Verbindung der Formel

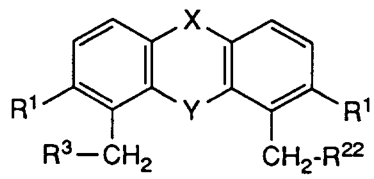

Id

worin X,Y,$R^1$ und $R^3$ obige Bedeutung besitzen und $R^{22}$ geschütztes Amino bedeutet, die Schutzgruppe abspaltet; oder

f) in einer Verbindung der allgemeinen Formel

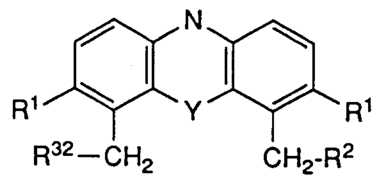

Ie

worin X,Y,$R^1$ und $R^2$ obige Bedeutung besitzen und $R^{32}$ funktionell abgewandeltes Carboxyl bedeutet, die funktionell abgewandelte Carboxyl-gruppe in die Carboxylgruppe überführt; oder

g) aus einer Verbindung der allgemeinen Formel

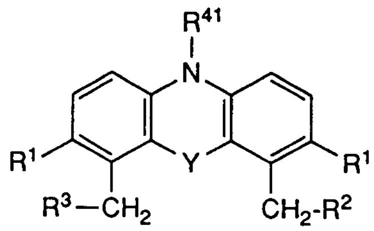

If

worin Y,$R^1$,$R^2$ und $R^3$ obige Bedeutung besitzen und $R^{41}$ eine abspaltbare Aralkylgruppe bedeutet, die abspaltbare Gruppe; entfernt oder

h) eine Verbindung der allgemeinen Formel

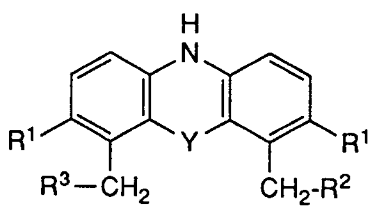

Ig

worin Y,$R^1$ ,$R^2$ und $R^3$ obige Bedeutung besitzen,
acyliert und erwünschtenfalls eine in der eingeführten Acylgruppe vorhandene Carboxylgruppe verestert; oder

i) eine Verbindung der allgemeinen Formel

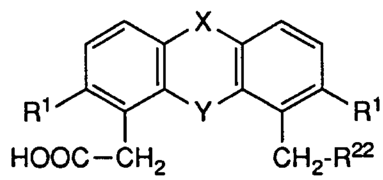

Ih

worin X,Y,$R^1$ und $R^{22}$ obige Bedeutung besitzen,
mit einer gegebenenfalls geschützten Aminosäure oder mit einer gegebenenfalls geschützten Kette von bis zu 20 Aminosäuren kuppelt; oder

j) eine Verbindung der allgemeinen Fomel

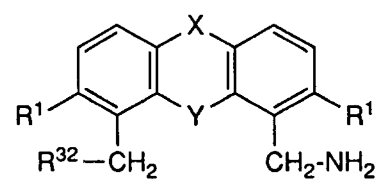

Ii

worin X,Y,$R^1$ und $R^{32}$ obige Bedeutung besitzen,
mit einer gegebenenfalls geschützten Aminosäure oder mit einer gegebenenfalls geschützten Kette von bis zu 20 Aminosäuren kuppelt; oder

k) eine Verbindung der allgemeinen Formel

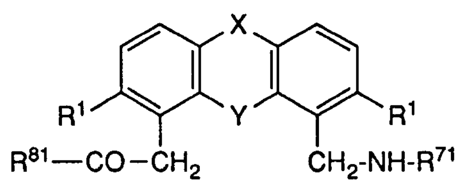

Ij

worin X,Y und $R^1$ obige Bedeutung besitzen und $R^{71}$ Wasserstoff, einen gegebenenfalls geschützten Rest einer Aminosäure oder eine gegebenenfalls geschützte Kette von Aminosäureresten und $R^{81}$ Wasserstoff, einen gegebenenfalls geschützten Rest einer Aminosäure oder eine gegebenenfalls geschütze Kette von Aminosäureresten bedeuten, wobei mindestens eines von $R^{71}$ und $R^{81}$ von Wasserstoff verschieden ist,
mit einer gegebenenfalls geschützten Aminosäure oder mit einer gegebenenfalls geschützten Kette von Aminosäuren kuppelt, wobei in den beiden Reaktionskomponenten insgesamt höchstens 20 Aminosäurereste vorhanden sind; oder

l) eine Verbindung der allgemeinen Formel

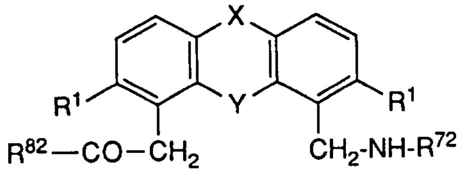

Ik

worin X,Y und $R^1$ obige Bedeutung besitzen und $R^{72}$ Wasserstoff, einen gegebenenfalls geschützten Rest einer Aminosäure oder eine gegebenenfalls geschützte Kette von bis zu 20 Aminosäureresten und $R^{82}$ Wasserstoff, einen gegebenenfalls geschützten Rest einer Aminosäure oder eine gegebenenfalls geschützte Kette von bis zu 20 Aminosäureresten bedeuten, wobei das Molekül mindestens einen und höchstens 20 Aminosäurereste enthält,
cyclisiert; oder

m) aus einer Verbindung der Formel I, welche mindestens einen geschützten Aminosäurerest enthält, die Schutzgruppe(n) abspaltet; oder

n) eine Verbindung der Formel I, welche ein basisches Zentrum enthält, mittels einer Säure bzw. eine Verbindung der Fomel I, welche ein saures Zentrum enthält, mittels einer Base in ein Salz überführt.

Die Reduktion einer Verbindung der Formel II gemäss Verfahrensvariante a) liefert Verbindungen der Formel I, worin $R^2$ Amino und $R^3$ Carboxyl oder funktionell abgewandeltes Carboxyl bedeuten. Sie erfolgt unter Anwendung von allgemein üblichen und jedem Fachmann geläufigen Methoden, zweckmässigerweise durch Hydrierung in Gegenwart eines geeigneten Katalysators, wie Palladiumkohle.

Die Hydrolyse einer Verbindung der Formel III gemäss Verfahrensvariante b) liefert Verbindungen der Formel I, worin $R^2$ geschütztes Amino oder Amino und $R^3$ Carboxyl bedeuten. Sie erfolgt ebenfalls unter Anwendung von allgemein üblichen und jedem Fachmann geläufigen Methoden, zweckmässigerweise unter stark sauren Bedingungen, z.B. durch Erhitzen mit konzentrierter Salzsäure, oder unter stark alkalischen Bedingungen, z.B. durch Erhitzen mit etwa 5-7 N Natronlauge o. dgl.

Verfahrensvariante c) liefert Verbindungen der Formel I, worin $R^2$ geschütztes Amino und $R^3$ Carboxyl oder funktionell abgewandeltes Carboxyl bedeuten. Die Verbindung der Formel Ib wird unter Anwendung von allgemein üblichen und jedem Fachmann geläufigen Methoden mit einem die gewünschte Schutzgruppe liefernden Mittel behandelt. So kann man zur Einführung einer tert.-Butyloxycarbonylgruppe (Boc) beispielsweise Di-tert.-butyldicarbonat verwenden, zur Einführung einer 9-Fluorenylmethoxycarbonylgruppe (Fmoc) beispielsweise N-(9- Fluorenylmethoxycarbonyl)-sucinimid, zur Einführung einer Benzyloxycarbonylgruppe (Z) beispielsweise Chlorkohlensäurebenzylester u.s.w.

Verfahrensvariante d) liefert Verbindungen der Fomel I, worin $R^2$ geschütztes Amino oder Amino und $R^3$ funktionell abgewandeltes Carboxyl bedeuten. Hierzu wird eine Verbindung der Formel Ic unter Anwendung von allgemein üblichen und jedem Fachmann geläufigen Methoden entsprechend verestert. So kann man zur Herstellung eines Methylesters beispielsweise Diazomethan verwenden, zur Herstellung eines Benzylesters Benzylbromid in Gegenwart einer geeigneten Base, wie 1,8-Diazabicyclo-[5.4.0]undec-7-en (DBU), oder Benzylalkohol in Gegenwart eines geeigneten Dehydratisierungsmittels, wie Dicyclohexylcarbodiimid, und einer geeigneten Base, wie 4-Dimethylaminopyridin, u.s.w.

Gemäss Verfahrensvariante e) erhält man Verbindungen der Formel I, worin $R^2$ Amino bedeutet. Hierzu wird aus einer Verbindung der Formel Id unter Anwendung von allgemein üblichen und jedem Fachmann geläufigen Methoden die Schutzgruppe abgespalten. So kann man zur Spaltung einer Phthalimidgruppe beispielsweise Hydrazinhydrat verwenden, zur Abspaltung einer tert.-Butyloxycarbonylgruppe (Boc) beispielsweise Trifluoressigsäure u.s.w.

Verfahrensvariante f) liefert Verbindungen der Formel I, worin $R^3$ Carboxyl bedeutet, und sie erfolgt unter Anwendung von allgemein üblichen und jedem Fachmann geläufigen Methoden zur Spaltung eines Esters. So kann die Spaltung eines niederen Alkylesters, z.B. eines Methylesters, zweckmässigerweise durch alkalische Hydrolyse erfolgen, beispielsweise mittels Kalium- oder Natriumhydroxyd in einem Gemisch von Wasser und dem entsprechenden Alkohol, die Spaltung eines Benzylesters zweckmässigerweise durch katalytische Hydrierung, z.B. in Gegenwart von Palladiumkohle, u.s.w.

Gemäss Verfahrensvariante g) erhält man Verbindungen der Formel I, worin X einen Rest der Formel (a) und $R^4$ Wasserstoff bedeuten. Die Abspaltung in der Formel If durch das Symbol $R^{41}$ wiedergegebenen Gruppe erfolgt unter Anwendung von allgemein üblichen und jedem Fachmann geläufigen Methoden. Zweckmässigerweise handelt es sich bei der abspaltbaren Gruppe um eine Benzylgruppe, und diese kann beispielsweise durch katalytische Hydrierung abgespalten werden, wobei als Hydrierungskatalysator in erster Linie Palladiumkohle in Betracht zu ziehen ist.

Verfahrensvariante h) liefert Verbindungen der Formel I , worin X einen Rest der Formel (a) und $R^4$ Acyl bedeuten. Die Acylierung der Verbindung der Formel Ig erfolgt unter Anwendung von allgemein üblichen und jedem Fachmann geläufigen Methoden durch Behandlung mit einem geeigneten, den gewünschten Acylrest liefernden Acylierungsmittel. So eignen sich beispielsweise zur Einführung eines Acetylrestes Acetylchlorid oder Acetanhydrid, zur Einführung eines Benzoylrestes oder eines substituierten Benzoylrestes Benzoylchlorid bzw.ein entsprechend substituiertes Benzoylchlorid, zur Einführung eines durch Carboxy substituierten niederen Alkanoylrests ein entsprechendes Dicarbonsäureanhydrid ( zur Einführung eines 4-Carboxybutyrylrests also Glutarsäureanhydrid) u.s.w.

Die Acylierung erfolgt in Anwesenheit einer Base, wobei je nach der Natur der zu acylierenden Verbindung der Formel Ig und des Acylierungsmittels anorganische Basen, wie Natriumcarbonat, Natriumhydroxyd o.dgl. und/ oder organische Basen, wie Triäthylamin, 4-Dimethylaminopyridin o. dgl. in Frage kommen.

Die Veresterung einer in der eingeführten Acylgruppe vorhandenen Carboxylgruppe kann nach allgemein üblichen und jedem Fachmann geläufigen Methoden erfolgen, z.B. mittels Methyljodid in Gegenwart

einer Base, wie 1,8-Diazabicyclo[5.4.0]undec-7-en.

Verfahrensvariante i) liefert Verbindungen der Formel I, worin $R^2$ geschütztes Amino, $R^3$ einen Rest der Formel (d) und $R^8$ einen gegebenenfalls geschützten Rest einer Aminosäure oder eine gegebenenfalls geschützte Kette von bis zu 20 Aminosäureresten bedeuten; Verfahrensvariante j) liefert Verbindungen der Formel I, worin $R^3$ funktionell abgewandeltes Carboxyl, $R^2$ einen Rest der Formel (c) und $R^7$ einen gegebenenfalls geschützten Rest einer Aminosäure oder eine gegebenenfalls geschützte Kette von bis zu 20 Aminosäureresten bedeuten; Verfahrensvariante k) liefert Verbindungen der Formel I, worin $R^2$ einen Rest der Formel (c), $R^3$ einen Rest der Formel (d) und $R^7$ und $R^8$ je einen gegebenenfalls geschützten Rest einer Aminosäure oder eine gegebenenfalls geschützte Kette von Aminosäureresten bedeuten, wobei das Molekül insgesamt höchstens 20 Aminosäurereste enthält; und Verfahrensvariante l) liefert Verbindungen der Formel I, worin $R^2$ einen Rest der Formel (c), $R^3$ einen Rest der Formel (d) und $R^7$ und $R^8$ zusammen einen gegebenfalls geschützten Rest einer Aminosäure oder eine gegebenfalls geschützte Kette von bis zu 20 Aminosäureresten bedeuten.

Zur Durchführung dieser Verfahrensvarianten werden in der Peptidchemie allgemein übliche und jedem Fachmann geläufige Methoden verwendet. Dabei kann durchaus auch die Methode der Festphasensynthese verwendet werden, falls im gewünschten Produkt einer der Aminosäurereste terminal oder in der Seitenkette oder die durch das Symbol X bezeichnete Gruppe eine Carboxylgruppe enthält; als Träger eignen sich beispielsweise p-Hydroxymethyl-phenoxy-polystyrolharz, 4-(2',4'-Dimethoxyphenyl-hydroxymethyl)phenoxy-polystrolharz u. dgl.

Geht man gemäss Verfahrensvariante i) von einer Verbindung der Formel Ih aus, so kuppelt man diese mit einer Aminosäurekomponente mit N-terminaler freier Aminogruppe und C-terminaler geschützter Carboxylgruppe. Geht man gemäss Verfahrensvariante j) von einer Verbindung der Formel Ii aus, so kuppelt man diese mit einer Aminosäurekomponente mit N-terminaler geschützter Aminogruppe und mit C-terminaler freier Carboxylgruppe oder mit einem aktivierten Derivat davon. Die in Verfahrensvariante k) als Ausgangsprodukte verwendeten Verbindungen der Formel Ij enthalten je nach der Natur der Reste $R^{71}$ und $R^{81}$ eine freie Aminogruppe oder eine freie Carboxylgruppe; in ersterem Fall kuppelt man sie mit einer Aminosäurekomponente mit N-terminaler geschützter Aminogruppe und mit C-terminaler freier Carboxylgruppe oder mit einem aktivierten Derivat davon, und in letzterem Fall mit einer Aminosäurekomponente mit N-terminaler freier Aminogruppe und mit C-terminaler geschützter Carboxylgruppe. Bei der Cyclisation einer Verbindung der Formel Ik gemäss Verfahrensvariante l) werden eine freie Carboxylgruppe oder ein aktiviertes Derivat davon und eine freie Aminogruppe unter Bildung einer Amidbindung miteinander gekuppelt.

Um diese Kupplung herbeizuführen, können alle möglichen in der Peptidchemie gebräuchlichen Aktivierungsreagenzien verwendet werden, wie z.B. O-Benzotriazol-1-yl-N,N,N',N'-tetramethyl-uroniumhexafluorphosphat (HBTU), O-(1,2-Dihydro-2-oxo-1-pyridyl)-N,N,N',N'-tetramethyl-uroniumtetrafluoroborat (TPTU), 2-(1H-Benzotriazol-1-yl)-1.1,3,3-tetramethyluroni-um-tetrafluoroborat (TBTU), 1-Hydroxybenzotriazol (HOBT) in Kombination mit N,N-Dicyclohexylcarbodiimid u. dgl.

Soll gemäss Verfahrensvariante i) bzw. j) bzw. k) eine Verbindung der Formel Ih bzw. Ii bzw. Ij mit einer gegebenenfalls geschützten Kette von Aminosäureresten gekuppelt werden, so kann dies auch stufenweise erfolgen.

Durch Abspaltung der Schutzgruppe(n) aus einer mindestens einen geschützten Aminosäurerest enthaltenden Verbindung der Formel I erhält man gemäss Verfahrensvariante m) eine entsprechende Verbindung der Formel I, welche 1-20 Aminosäurereste enthält, worin der Aminosäurerest bzw. die Aminosäurerste keine Schutzgruppe(n) enthält bzw. enthalten. Die Abspaltung der Schutzgruppe(n) erfolgt nach in der Peptidchemie allgemein üblichen und jedem Fachmann geläufigen Methoden, wobei natürlich jeweils die Natur der Schutzgruppe(n) in Betracht zu ziehen ist. So können die weiter oben genannten Schutzgruppen beispielsweise wie folgt abgespalten werden:

| | |
|---|---|
| Z : | Katalytische Hydrierung in Gegenwart von Pd/ C in einem niederen Alkanol, wie Methanol oder Aethanol. |
| Boc: | Mittels Trifluoressigsäure/ Methylenchlorid (1:1) oder mittels gesättigter Chlorwasserstofflösung in Essigsäureäthylester. |
| Fmoc : | Mittels Piperidin oder 1,8-Diazabicyclo[5.4.0]undec-7-en in Dimethylformamid. |
| Alloc : | Mittels Palladium-tetrakis-triphenylphosphin in Tetrahydrofuran/Dimethylsulfoxid / 0,1 N Salzsäure. |
| Teoc : | Mittels Caesiumfluorid oder Tetrabutylammoniumfluorid in Dimethylformamid o.dgl. |
| Tcc : | Mittels Zink in Eisessig oder Methanol. |
| Nps : | Mittels Natrium- oder Kaliumrhodanid in leicht saurem Milieu. |

| | |
|---|---|
| $^{t}$Bu : | Mittels Trifluoressigsäure/ Methylenchlorid (1:1). |
| Bzl : | Durch katalytische Hydrierung in Gegenwart von Pd / C in einem niederen Alkanol, wie Methanol oder Aethanol. |
| Me: | Mittels Lithiumhydroxid in Tetrahydrofuran/Methanol/Wasser (3:1:1). |
| Ph : | Mittels Natriumperoxyd bei pH 10,5. |
| Pac : | Mittels Zink in Eisessig oder Methanol oder mittels Natriumthiophenolat in Dimethylformamid. |
| Allyl : | Mittels Palladium-bis-triphenylphosphin-dichlorid und Tributylzinnhydrid oder mittels Palladium-tetrakis-triphenylphosphin in Tetrahydrofuran/ Dimethylsulfoxyd/0,5 N Salzsäure. |
| Trimethylsilyläthyl : | Mittels Caesiumfluorid oder Tetrabutylammoniumfluorid in Dimethylformamid o. dgl. |
| Trichloräthyl: | Mittels Zink in Eisessig oder Methanol. |
| Pmc : | Mittels wässriger Trifluoressigsäure. |
| Ts : | Mittels Natrium in flüssigem Ammoniak oder flüssigem Fluorwasserstoff. |

In analoger Weise kann eine an einem p-Hydroxymethylphenoxypolystyrolharz, an einem 4-(2',4'-Dimethoxyphenyl-hydroxymethyl)phenoxypolystyrolharz o.dgl. durch Festphasensynthese hergestellte Verbindung der Formel I, welche einen Aminosäurerest enthält, der terminal oder in der Seitenkette eine Carboxylgruppe aufweist, oder in welcher die durch das Symbol X bezeichnete Gruppe eine Carboxylgruppe enthält, von dem Trägerharz abgespalten werden, beispielsweise mittels "Field's Reagens", d.h. einer Mischung aus 82,5% Trifluoressigsäure, 5% Phenol. 5% Wasser, 5% Thioanisol und 2,5% 1,2-Aethandithiol.

Gemäss Verfahrensvariante n) kann eine Verbindung der Formel I, welche ein basisches bzw. ein saures Zentrum enthält, mittels einer Säure bzw. einer Base in ein Salz übergeführt werden, was nach allgemein üblichen und jedem Fachmann geläufigen Methoden erfolgen kann. Als Säuren kann man hier für anorganische Säuren, wie Salzsäure, Schwefelsäure, Phosphorsäure o. dgl., oder organische Säuren, wie Trifluoressigsäure, Methansulfonsäure, p-Toluolsulfonsäure o.dgl., verwenden und als Base anorganischen Basen, wie Kaliumhydroxyd, Natriumhydroxyd o.dgl., oder organische Basen, wie Triätlylamin, Dimethylaminopyridin o. dgl.

Die für Verfahrensvarianten a) und b) benötigten Ausgangsprodukte sind neu und ebenfalls Gegenstand der vorliegenden Erfindung. Sie können gemäss nachfolgendem Reaktionsschema hergestellt werden, worin $X^1$ einen Rest der Formel (a), worin $R^4$ niederes Alkyl, Aryl oder Aryl-niederes Alkyl bedeutet, oder einen Rest der Formel (b) bedeutet; die beiden Symbole $R^{11}$ je niederes Alkyl oder zusammen niederes Alkylen bedeuten; $R^{12}$ niederes Alkyl, Aryl oder Aryl-niederes Alkyl bedeutet; $R^{13}$ eine Schutzgruppe, wie tert,-Butyl-dimethyl-silyl (TBDMS), tert.-Butyl-diphenylsilyl (TBDPS), 2-Methoxyäthoxymethyl (Mem) o. dgl., bedeutet; $R^{14}$ Wasserstoff oder eine Schutzgruppe, wie tert.-Butyloxycarbonyl (Boc), 9-Fluorenylmethoxycarbonyl (Fmoc), o. dgl. bedeutet; Phth die Phthalimidgruppe bedeutet; und Y und $R^1$ obige Bedeutung besitzen.

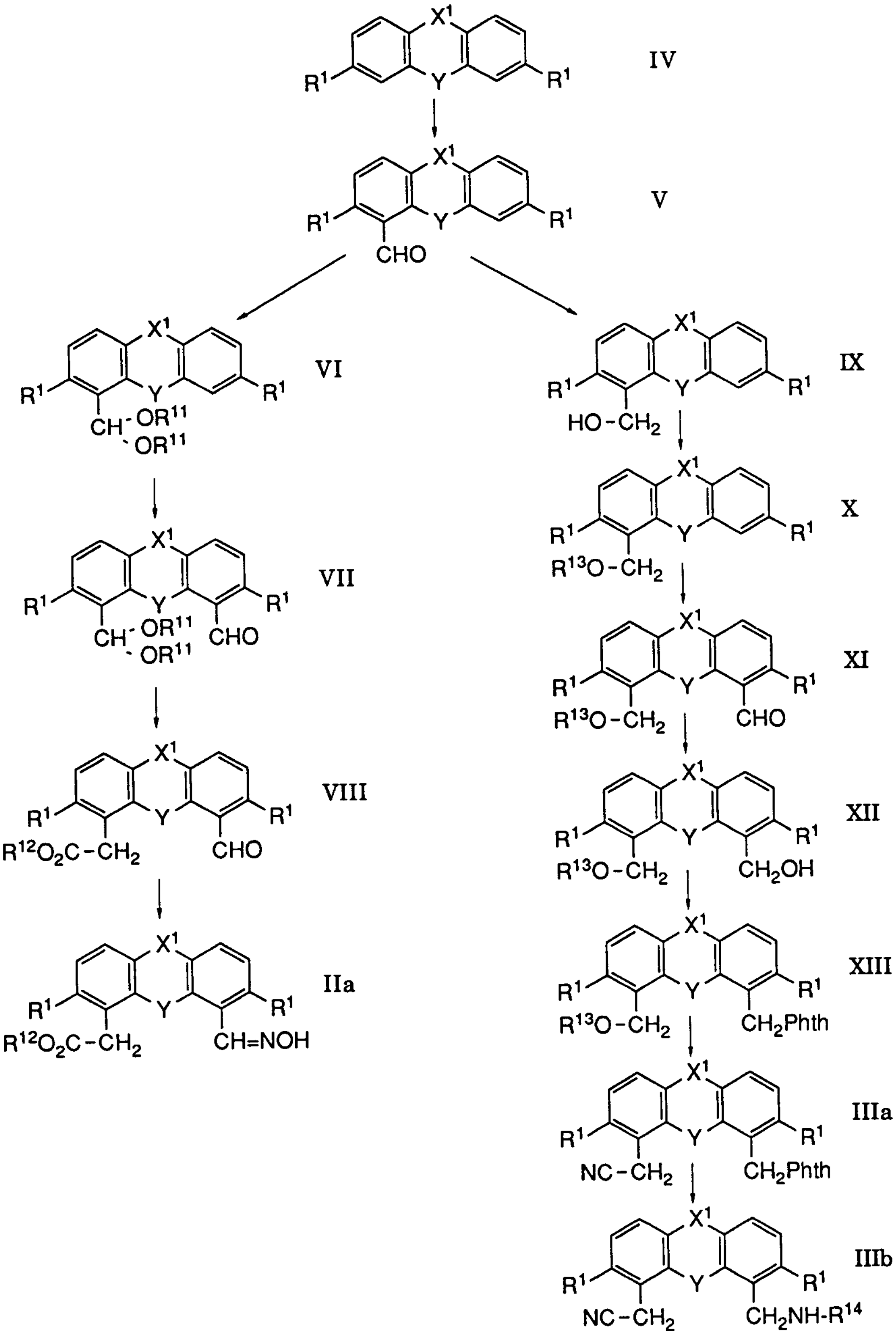

IV

V

VI

IX

VII

X

XI

VIII

XII

IIa

XIII

IIIa

IIIb

Die Verbindung der Formell IV sind bekannt oder, in Analogie zur Herstellung der bekannten Verbindungen, nach allgemein üblichen und jedem Fachmann geläufigen Methoden leicht herstellbar; zudem enthalten einige der nachfogenden Beispiele detaillierte Angaben über die Herstellung von gewissen bisher noch nicht beschriebenen Verbindungen der Formel IV.

Die einzelnen Stufen gemäss vorstehendem Reaktionsschema werden nachstehend näher erläutert.

IV → V

Die Verbindung der Formel IV wird zweckmässigerweise mit etwas mehr als der aequimolaren Menge von Butyllithium in Aether/ Hexan umgesetzt. Zur Einführung der Formylgruppe dient zweckmässigerweise N-Formylpiperidin.

V → VI

Die Formylgruppe wird in üblicher Weise in eine Acetalgruppe übergeführt, zweckmässigerweise mittels Orthoameisäuretriäthylester o. dgl.

VI → VII

Die Verbindung der Formel VI wird zweckmässigerweise mit etwas mehr als der aequimoleren Menge von Butyllithium in Aether/ Tetrahydrofuran/ Hexan umgesetzt. Zur Einführung der Formylgruppe dient wiederum zweckmässigerweise N-Fomylpiperdin.

VII → VIII

Die Verbindung der VII wird zweckmässigerweise in Gegenwart einer Base, wie Triton B, mit Methyl (methylthiomethyl)sulfoxid umgesetzt. Das entstandene Produkt wird dann zweckmassigerweise mit einer Lösung von Salzsäure in einem Alkohol der Formel $R^{12}$ -OH behandelt. In der erhaltenen Verbindung ist die Formylgruppe aus der Acetylgruppe der Verbindung der Formel VII entstanden und die Estergruppe ($R^{12}O_2C\text{-}CH_2$-) aus der Formylgruppe der Verbindung der Formel VII.

VIII → IIA

Der Aldehyd der Formel VII wird zweckmässigerweise mittels Hydroxylaminhydrochlorid in Gegenwart von Natriumacetat in das entsprechende Oxim übergeführt.

V → IX

Die Reduktion der Formyl- zur Hydroxymethylgruppe erfolgt zweckmässigerweise mittels Lithiumborhydrid in Tetrahydrofuran.

IX → X

Die Hydroxymethylgruppe wird in üblicher Weise geschützt, beispielsweise mit tert.-Butyldimethylchlorsilan/ Imidazol, mit tert.-Butyldiphenylsilylchlorid / Träthylamin / Dimethylaminopyridin, mit 2-Methoxyethoxymethylchlorid / Butyllithium u.s.w.

X → XI

Die Formylierung erfolgt analog wie für die Stufe VI → VII beschrieben.

XI → XII

Die Reduktion der Formyl- zur Hydroxymethylgruppe erfolgt analog wie für Stufe V → IX beschrieben.

XII → XIII

Der Ersatz der Hydroxygruppe durch die Phthalimidgruppe erfolgt zweckmässigerweise nach der von Mitsunobu (Synthesis 1981, 1) beschriebenen Methode mittels Phthalimid / Triphenylphosphin/ Azodicarbonsäuredimethylester.

XIII → IIIa

Zweckmässigerweise wird die Verbindung der Formel XIII zunächst in die entsprechende Brommethylverbindung übergeführt; wenn $R^{13}$ Methoxyäthoxyäthyl bedeutet, erfolgt dies zweckmässigerweise mittels Bromwasserstoff, und wenn $R^{13}$ tert.-Butyldimethylsilyl oder tert.-Butyldiphenylsilyl bedeutet, zweckmässigerweise mittels Bortribromid. Die Brommethylverbindung wird dann zweckmässigerweise mit Natriumcyanid umgesetzt.

IIIa → IIIb

Durch Spaltung der Phthalimidgruppe, z.B. mittels Hydrazinhydrat und nachfolgender Hydrolyse oder Verseifung, erhält man eine Verbindung der Formel IIIb, worin $R^{14}$ Wasserstoff bedeutet. Diese kann dann erwünschtenfalls nach allgemein üblichen Methoden in eine entsprechende Verbindung der Formel IIIb, worin $R^{14}$ eine Schutzgruppe, wie Boc, Fmoc o. dgl., bedeutet, übergeführt werden.

Erwünschtenfalls kann in einer Verbindung der Formel VII, VIII, IIa, XI, XIII, IIIa oder IIIb, worin X einen Rest der Formel (a) und $R^4$ eine leicht abgespaltbare Aralkylgruppe bedeutet, diese Gruppe abgespalten und erwünschtenfalls durch eine Acylgruppe ersetzt werden, wonach eine in der eingeführten Acylgruppe vorhandene Carbonylgruppe erwünschtenfalls verestert werden kann. Weiterhin kann in einer Verbindung der Formel VIII oder IIa eine Esterspaltung (erwünschtenfalls gefolgt von einer Veresterung der entstandenen Carbonsäure mit einem anderen Alkohol) oder eine Umesterung erfolgen.

Die Verbindungen der Formeln V bis XIII sind neu und ebenfalls Gegenstand der vorliegenden Erfindung.

Die Verbindungen der Formel Ia sind Aminosäurederivate, in welchen die Aminosäure bzw. die Aminosäuresequenz bezüglich ihrer konformativen Flexibilität stark eingeschränkt ist bzw. sind. Im speziellen liegen in Verbindungen der Formel Ia, worin Z eine Kette von 2 oder 4 Aminosäureresten bedeutet, die Aminosäuresequenzen bevorzugt in einer ß-Turn bzw. ß-Turn ähnlichen Konformation vor (vgl. G.D. Rose, L. Gierasch, J.A. Smith, Advances in Protein Chemistry 1985, 37, 1-109 und P.Y. Chou, G.D. Fasman, J. Mol. Biol. 1977, 115, 135- 175).

Die Verbindungen der Formel Ia, insbesondere solche, worin der Aminosäurerest bzw. die Kette von Aminosäureresten keine Schutzgruppe(n) enthält, eignen sich als Mimetica von exponierten Regionen von Proteinen, um deren Rolle bezüglich der Wechselwirkungen mit anderen Proteinen (Rezeptoren, Enzyme o. dgl.) aufzuklären. Insbesondere können so mittels Verbindungen der Formel Ia Aminosäuresequenzen mit biologischer Aktivität ermittelt werden. Sie eignen sich demnach als Forschungshilfsmittel ("Research Tools"), um biologisch aktive Peptidsequenzen bereitzustellen. Ausgehend von Proteinen, deren dreidimensionale Struktur bekannt ist, können exponierte schlaufenförmige Peptidsequenzen ermittelt werden. Derartige Peptidsequenzen können dann zu pharmakologisch wertvollen Verbindungen führen. Beispielsweise sind Verbindungen der Formel Ia, worin Z die Sequenz -Arg-Gly-Asp-Phe- oder Arg-Gly-Asp-Val- ist oder enthält, als Fibrinogen-Antagonisten wirksam, d.h. sie hemmen die Bindung von Fibrinogen an den Fibrinogenrezeptor der Blutplättchen (Glykoprotein IIb/ IIIa), was wie folgt nachgewiesen werden kann.

Das Glykoprotein IIb/ IIIa wird aus Triton X- 100 Extrakten von menschlichen Blutplättchen gewonnen und durch Lectin-Affinitätschromatographie (Analytical Biochemistry 151, 1985, 169-177) und Chromatographie an einer Arg-Gly-Asp-Ser-Affinitätssäule (Science 231, 1986, 1559-62) gereinigt. Das so erhaltene Rezeptorprotein wird an Mikrotiterplatten gebunden. Die spezifische Bindung von Fibrinogen an den immobilisierten Rezeptor wird mit Hilfe eines ELISA-Systems ("enzyme-linked immunosorbent assay") bestimmt. Als $IC_{50}$ wird diejenige Konzentration einer Testsubstanz bezeichnet, welche benötigt wird, um die Hemmung von Fibrinogen an den immobilisierten Rezeptor um 50% zu hemmen. Die nachfolgende Tabelle enthält für verschiedene Verbindungen der Formel Ia, welche das oben erwähnte strukturelle Kriterium erfüllen, Angaben darüber, um welchen Faktor sich deren $IC_{50}$ von derjenigen unterscheiden ("relative $IC_{50}$"), welche für das als Standard mituntersuchte L-Arginylglycyl-L-aspartyl-L-Serin ermittelt wurde (für welches die relative $IC_{50}$ als 1.0000 angegeben ist).

| Testsubstanz | relative $IC_{50}$ |
|---|---|
| A | 0.0124 |
| B | 0.0062 |
| C | 0.0077 |
| D | 0.1553 |
| E | 0.0483 |
| F | 0.68 |
| G | 1.46 |

| Testsubstanz | relative $IC_{50}$ |
|---|---|
| H | 0.62 |
| I | 7.51 |
| Standard | 1.0000 |

A: 4,5-Cyclo[-acetyl-L-alanyl-L-arginyl-L-isoleucyl-L-alanyl-L-arginyl- glycyl-L-aspartyl-L-phenylalanyl-L-prolyl-L-aspartyl-L-aspartyl-L-arginylaminomethyl-]-3,6-dimethoxy-9,9-dimethylxanthen

B: 4,5-Cyclo[-acetyl-L-arginyl-L-isoleucyl-L-alanyl-L-arginylglycyl-L-aspartyl-L-phenylalanyl-L-prolyl-L-aspartyl-L-aspartylaminomethyl-]-3,6-dimethoxy-9,9-dimethylxanthen

C: 4,5-Cyclo[-acetyl-L-isoleucyl-L-alanyl-L-arginyl-glycyl-L-aspartyl-L-phenylalanyl-L-prolyl-L-aspartylaminomethyl-]-3,6-dimethoxy-9,9-dimethyl-xanthen

D: 4,5-Cyclo[-acetyl-L-alanyl-L-arginyl-glycyl-L-aspartyl-L-phenylalanyl-L-prolylaminomethyl-]-3,6-dimethoxy-9,9-dimethylxanthen

E: 4,5-Cyclo[-acetyl-L-arginyl-glycyl-L-aspartyl-L-phenylalanylamino-methyl-]-3,6-dimethoxy-9,9-dimethylxanthen

F: 4,5-Cyclo[-acetyl-L-arginyl-glycyl-L-aspartyl-L-valylaminomethyl-]-3,6-dimethoxy-9,9-dimethylxanthen

G: 10-Methyl-4,6-cyclo[-acetyl-L-arginyl-glycyl-L-aspartyl-L-valylamino-methyl-]phenothiazin

H: 4,6-Cyclo[-acetyl-L-arginyl-glycyl-L-asparagyl-L-valylaminomethyl-] -3,7-diäthoxy-10-aethyl-10H-dibenz[b,e][1,4] oxazin

I: 10-Hexyl-3,7-dimethoxy-4,6-cyclo[-acetyl-L-arginyl-glycyl-L-aspartyl-L-valylaminomethyl-]phenothiazin

Aus dem Obigen geht hervor, dass die Verbindungen der Formel Ia, insbesondere solche, worin der Aminosäurerest bzw. die Kette von Aminosäureresten keine Schutzgruppe(n) enthält, nicht nur als "Research Tools" verwendet werden können, sondern auch potentiell als Arzneimittel geeignet sind, wobei der jeweilige therapeutische Verwendungszweck in erster Linie von der Natur, der Anzahl und der Reihenfolge der im Molkül vorhandenen Aminosäurereste abhängt. So können beispielsweise die vorstehend abgehandelten, als Fibrinogen-Antagonisten wirksamen Verbindungen A bis I und pharmazeutisch anwendbare Salze davon zur Verhinderung der Entstehung von Blutplättchenthromben und damit zur Bekämpfung bzw. Verhütung von Krankheiten, wie Thrombose, Hirnschlag, Herzinfarkt, Entzündung und Arteriosklerose verwendet werden.

Somit sind, wie eingangs erwähnt, Arzneimittel, enthaltend eine Verbindung der Formel Ia oder ein pharmazeutisch anwendbares Salz davon und einen pharmazeutisch anwendbaren Hilfsstoff, ebenfalls Gegenstand der vorliegenden Erfindung, weiterhin auch ein Verfahren zur Herstellung derartiger Arzneimittel, welches dadurch gekennzeichnet ist, dass man eine oder mehrere Verbindungen der Formel Ia oder pharmazeutisch anwendbare Salze davon und erwünschtenfalls einen oder mehrere andere therapeutisch wertvolle Stoffe zusammen mit einem oder mehreren pharmazeutisch anwendbaren Hilfsstoffen in eine galenische Darreichungsform bringt. Die Arzneimittel können enteral, z.B. oral in Form von Tabletten, Lacktabletten, Dragées, Hart- und Weichgelatinekapseln, Lösungen, Emulsionen oder Suspensionen, oder rektal, z.B. in Form von Suppositoren, oder als Spray verabreicht werden. Die Verabreichung kann aber auch patenteral, z.B. in Form von Injektionslösungen, erfolgen.

Zur Herstellung von Tabletten, Lacktabletten, Dragées und Hartgelatinekapseln kann der Wirkstoff bzw. können die Wirkstoffe mit pharmazeutisch inerten, anorganischen oder organischen Excipientien vermischt werden. Als solche Excipientien kann man für Tabletten, Dragées und Hartgelatinekapseln z.B. Lactose. Maisstärke oder Derivate davon, Talk, Stearinsäure oder deren Salze, verwenden. Für Weichgelatinekapseln

eignen sich als Excipientien z.B. vegetabile Oele, Wachse, Fette, halbfeste und flüssige Polyole; je nach Beschaffenheit des Wirkstoffes bzw. der Wirkstoffe sind jedoch bei Weichgelatinekapseln überhaupt keine Excipientien erforderlich. Zur Herstellung von Lösungen und Sirupen eignen sich als Excipientien z.B. Wasser, Polyole, Saccharose, Invertzucker und Glukose, für Injektionslösungen eignen sich z.B. Wasser, Alkohole, Polyole, Glycerin und vegetabile Oele, und für Suppositorien eignen sich z.B. natürliche oder gehärtete Oele, Wachse, Fette und halbflüssige oder flüssige Polyole. Die pharmazeutischen Präparate können daneben noch Konservierungsmittel, Lösungsvermittler, Färbemittel, Aromatisierungsmittel, Salze zur Veränderung des osmotischen Druckes, Ueberzugsmittel oder Antioxidantien enthalten.

Schliesslich sind Gegenstand der Erfindung auch die Verwendung von Verbindungen der Formel Ia und von Salzen davon als "Research Tools" zur Ermittlung von biologisch aktiven Peptidsequenzen sowie die Verwendung von Verbindungen der Formel Ia und von pharmazeutisch anwendbaren Salzen davon als Heilmittel bzw. zur Herstellung von Arzneimitteln, beispielsweise die Verwendung der weiter vorne abgehandelten, als Fibrinogen-Antagonisten wirksamen Verbindungen A bis I und von pharmazeutisch anwendbaren Salzen davon zur Verhinderung der Entstehung von Blutplättchenthromben bzw. zur Herstellung entsprechender Arzneimittel.

In den nachfolgenden Beispielen, welche die Erfindung näher erläutern, ihren Umfang aber in keiner Weise beschränken sollen, sind sämtliche Temperaturen in Celsiusgraden angegeben.

Beispiel 1.1.1.a

Eine Lösung von 10,8 g (40 mMol) 3,6-Dimethoxy-9,9-dimethylxanthen [hergestellt nach Coll. Czech. Chem. Commun. 24 (1959), 1061] in absolutem Aether wurde auf 0 bis -5° abgekühlt. Unter Rühren wurden 50 ml einer 1,6M Butyllithiumlösung (in Hexan) zugetropft. Nach 15 Stunden wurden 9,04 g (80 mMol) N-Formylpiperidin zugetropft, und anschliessend wurde das Gemisch 1 Stunde bei 0° sowie 2 Stunden bei Raumtemperatur gerührt. Danach wurde das Reaktionsgemisch auf saures (pH 1) Eiswasser gegossen, worauf zweimal mit je 200 ml Aether extrahiert wurde. Die vereinigten Extrakte wurden mit Eiswasser neutral gewaschen, über $Na_2SO_4$ getrocknet und eingeengt. Das verbleibende Oel wurde an Kieselgel mit Hexan/Essigester gereinigt und danach aus t-Butylmethyläther/Hexan kristallisiert. Es wurden 7,1 g 3,6-Dimethoxy-9,9-dimethylxanthen-4-carboxaldehyd vom Smp. 84,5-86° erhalten; IR 2966, 1688, 1637, 1605, 1568, 1515, 1486 $cm^{-1}$.

Beispiel 1.1.1.b

Eine Lösung von 24,15 g 3,6-Dimethoxy-9,9-dimethylxanthen-4-carboxaldehyd in 50 ml absolutem Aethanol wurde mit 50 ml Orthoameisensäuretriäthylester versetzt. Nach Zugabe von 100 mg p-Toluolsulfonsäure-monohydrat wurde das Gemisch 1 Stunde unter Rückfluss gekocht, dann abgekühlt und auf eiskalte Natriumbicarbonatlösung gegossen, worauf zweimal mit je 200 ml Aether extrahiert wurde. Die vereinigten Extrakte wurden mit Wasser neutral gewaschen, über $Na_2SO_4$ getrocknet und eingeengt. Der Rückstand wurde an Kieselgel mit Hexan/Essigester gereinigt, wobei 19,6 g 3,6-Dimethoxy-9,9-dimethylxanthen-4-carboxaldehyd-dimethylacetal als hellgelbes Oel erhalten wurden, welches langsam kristallisierte; Smp. 60-62°, IR 2972, 1606, 1572, 1490 $cm^{-1}$.

Beispiel 1.1.1.c

4,46 g (12 mMol) 3,6-Dimethoxy-9,9-dimethylxanthen-4-carboxaldehyddiäthylacetal wurden in einem Gemisch von 43 ml Aether und 10 ml THF gelöst. Die erhaltene Lösung wurde auf -10° abgekühlt und tropfenweise mit 11,25 ml (18 mMol) einer 1,6M Butyllithiumlösung (in Hexan) versetzt. Nach 6 Stunden wurden 2,17 g (19,2 mMol) N-Formylpiperidin zugegeben. Nach einer weiteren Stunde wurde das Gemisch auf Eiswasser gegossen, worauf mit Aether extrahiert wurde. Die Extrakte wurden mit Eiswasser gewaschen, über $Na_2SO_4$ getrocknet und eingeengt. Der Rückstand wurde aus Hexan/Aether kristallisiert, wobei 3,6-Dimethoxy-9,9-dimethylxanthen-4,5-dicarboxaldehyd-4-diäthylacetal vom Smp. 144-145° erhalten wurde; IR 1685, 1624, 1603, 1568, 1465, 1390 $cm^{-1}$.

Beispiel 1.1.1.d

Eine Lösung von 2,15 g (5,37 mMol) 3,6-Dimethoxy-9,9-dimethylxanthen-4,5-dicarboxaldehyd-4-diäthylacetal in 15 ml THF wurde zuerst mit 0,8 g (6,44 mMol) Methyl(methylthiomethyl)sulfoxid und danach mit 0,63 ml einer Triton-B Lösung (35% in Methanol) versetzt. Das Gemisch wurde 3 Stunden unter Rückfluss

erhitzt, dann abgekühlt und schliesslich auf Eiswasser gegossen, worauf mit 2 * 50 ml Aether extrahiert wurde. Die Extrakte wurden mit Wasser neutral gewaschen über Magnesiumsulfat getrocknet und eingeengt. Der verbleibende Rückstand (2,66 g) wurde mit 30 ml einer methanolischen Salzsäurelösung (20%) versetzt. Das Gemisch wurde 3 Stunden bei Raumtemperatur gerührt und dann auf eiskalte gesättigte Natriumbicarbonatlösung gegossen, worauf mit 2 * 50 ml Aether extrahiert wurde. Die ätherische Phase wurde mit Wasser neutral gewaschen über Magnesiumsulfat getrocknet und eingeengt. Der Rückstand wurde an Kieselgel mit Hexan/Essigsäureäthylester chromatographiert und danach aus Hexan/Essigsäureäthylester umkristallisiert, wobei 1,05 g 3,6-Dimethoxy-9,9-dimethyl-5-formylxanthen-4-essigsäuremethylester vom Smp. 142-143,5° erhalten wurden; IR 2964, 1730, 1681, 1625, 1602, 1573 $cm^{-1}$.

Beispiel 1.1.1.e

5 g (13,5 mMol) 3,6-Dimethoxy-9,9-dimethyl-5-formylxanthen-4-essigsäuremethylester wurden in einem Gemisch von 75 ml Methanol, 75 ml THF und 20 ml Wasser gelöst. Nach Zugabe von 1,66 g Natriumacetat und 1,4 g Hydroxylaminhydrochlorid wurde über Nacht bei Raumtemperatur gerührt und dann mit 200 ml Wasser verdünnt. Der ausgefallene Feststoff wurde abfiltriert, mit Wasser gewaschen und getrocknet, wobei 4,95 g 3,6-Dimethoxy-9,9-dimethyl-5-[(hydroximino)methyl]xanthen-4-essigsäuremethylester als weisses Pulver vom Smp. 234-236° erhalten wurden; IR 1743, 1629, 1604, 1582, 1498 $cm^{-1}$.

Beispiel 1.1.1.f

4 g (10,4 mMol) 3,6-Dimethoxy-9,9-dimethyl-5-[(hydroximino)methyl]-xanthen-4-essigsäuremethylester wurden in 100 ml Methanol suspendiert, worauf 60 ml einer 20%igen methanolischen Salzsäurelösung zugegeben wurden. Das Gemisch wurde 2 Stunden bei Raumtemperatur stehen gelassen, dann mit 1 g Palladiumkohle (10%) versetzt und 20 Stunden unter Wasserstoff gerührt. Danach wurde der Katalysator abfiltriert, und das Filtrat wurde zur Trockene eingedampft. Das als Rückstand verbleibende Rohprodukt wurde aus Methanol/Aether umkristallisiert, wobei 2,95 g farbloses 5-Aminomethyl-3,6-dimethoxy-9,9-dimethylxanthen-4-essigsäuremethylester-hydrochlorid vom Smp. 261-262° erhalten wurden; IR 3233, 2967, 2838, 1724, 1606 und 1495 $cm^{-1}$.

Beispiel 1.1.2.

Eine Lösung von 1 g (2,45 mMol) 5-Aminomethyl-3,6-dimethoxy-9,9-dimethylxanthen-4-essigsäuremethylester-hydrochlorid in 30 ml Dioxan wurde mit 15 ml Wasser verdünnt und bei 0° mit 1N Natronlauge auf pH 9 gestellt. Danach wurde tropfenweise eine Lösung von 0,8 g (3,7 mMol) Di-tert-butyldicarbonat in 2 ml Dioxan zugegeben. Das Gemisch wurde 2 Stunden bei 0° gerührt und dann auf Eiswasser gegossen, worauf dreimal mit Methylenchlorid extrahiert wurde. Die vereinigten Extrakte wurden zuerst mit Wasser und dann mit gesättigter Kochsalzlösung gewaschen, über Natriumsulfat getrocknet und eingeengt, wobei als Rückstand 1,47 g 5-[(1-tert-Butylformamido)methyl]-3,6-dimethoxy-9,9-dimethylxanthen-4-essigsäuremethylester als farbloses Oel verblieben; IR 3462, 3425, 2971, 2838, 1741, 1714, 1605, 1495 $cm^{-1}$.

Beispiel 1.1.3.

Eine Lösung von 4 g (8,48 mMol) 5-[(1-tert-Butoxyformamido)methyl]-3,6-dimethoxy-9,9-dimethylxanthen-4-essigsäuremethylester in 80 ml Methanol wurde mit 25 ml 1N-Kaliumhydroxidlösung versetzt. Das Gemisch wurde 2 Stunden auf 50° erwärmt, dann mit weiteren 9 ml 1N-Kaliumhydroxidlösung versetzt, hierauf 5 Stunden bei 50° nachgerührt und dann abgekühlt. Das pH wurde mit 2N Salzsäure auf 1 eingestellt, worauf dreimal mit je 180 ml Methylenchlorid extrahiert wurde. Die vereinigten organischen Extrakte wurden mit verdünnter Kochsalzlösung gewaschen über Magnesiumsulfat getrocknet und vom Lösungsmittel befreit, wobei als Rückstand 3,3 g 5-[(1-tert-Butoxyformamido)methyl]-3,6-dimethoxy-9,9-dimethylxanthen-4-essigsäure als farbloses Pulver verblieben; IR 2970, 1714, 1629, 1606, 1494 $cm^{-1}$.

Beispiel 1.1.4.

Eine Lösung von 3 g (6,55 mMol) 5-[(1-tert-Butoxyformamido)methyl]-3,6-dimethoxy-9,9-dimethylxanthen-4-essigsäure in 65 ml Methylenchlorid wurde auf 0° gekühlt und mit 1,1 ml (10,62 mMol) Benzylalkohol versetzt. Nach Zugabe von 20 mg 4-Dimethylaminopyridin wurde eine Lösung von 1,5 g (7,27 mMol) Dicyclohexylcarbodiimid in 16 ml Methylenchlorid zugetropft, worauf das Gemisch auf Raumtemperatur

erwärmt und 3 Stunden nachgerührt wurde. Der ausgefallene Dicyclohexylharnstoff wurde abfiltriert und mit wenig Methylenchlorid gewaschen. Das Filtrat wurde mit Natriumbicarbonatlösung und Wasser gewaschen, über Natriumsulfat getrocknet und eingeengt. Der verbleibende Rückstand wurde an Kieselgel mit Hexan/Essigsäureäthylester chromatographiert, wobei 2,9 g 5-[(1-tert-Butoxyformamido)methyl]-3,6-dimethoxy-9,9-dimethylxanthen-4-essigsäurebenzylester als farbloses Pulver vom Smp. 139-141° erhalten wurden; IR 3443, 2972, 2930, 1724, 1605, 1498 $cm^{-1}$.

Beispiel 1.1.5.

2,9 g (5,29 mMol) 5-[(1-tert-Butoxyformamido)methyl]-3,6-dimethoxy-9,9-dimethylxanthen-4-essigsäurebenzylester wurden bei 0° in einer Mischung von 10 ml Trifluoressigsäure und 5 ml Wasser aufgenommen. Nach einer halben Stunde wurden die Lösungsmittel entfernt, worauf der Rückstand mit 20 ml Aether versetzt wurde. Der erhaltene Feststoff wurde abfiltriert, mit Aether gewaschen und über Magnesiumsulfat getrocknet, wobei 2,25 g 5-Aminomethyl-3,6-dimethoxy-9,9-dimethylxanthen-4-essigsäurebenzylester-trifluoracetat als farbloses Pulver vom Smp. 169,5-171° erhalten wurden; IR 1716, 1662, 1631, 1604, 1538, 1494 $cm^{-1}$.

Beispiel 1.1.6.

Eine Lösung von 2 g (3,56 mMol) 5-Aminomethyl-3,6-dimethoxy-9,9-dimethylxanthen-4-essigsäurebenzylester-trifluoracetat in 70 ml Methanol wurde in Gegenwart von 200 mg Palladiumkohle (10%) bei Raumtemperatur unter Wasserstoff gerührt. Nach 30 Minuten wurde der Katalysator abfiltriert und das Filtrat eingeengt. Der Rückstand wurde mit Aether gewaschen und getrocknet, wobei 1,6 g 5-Aminomethyl-3,6-dimethoxy-9,9-dimethylxanthen-4-essigsäure-trifluoracetat vom Smp. 178-184° (Zers.) erhalten wurden; IR 1680, 1630, 1608, 1496 $cm^{-1}$ absorbiert.

Beispiel 1.1.7.

1,58 g (3,35 mMol) 5-Aminomethyl-3,6-dimethoxy-9,9-dimethylxanthen-4-essigsäure-trifluoracetat wurden zusammen mit 1,13 g (3,35 mMol) N-(9-Fluorenylmethoxycarbonyl)-succinimid in einem Gemisch von 7 ml Dioxan und 7 ml Wasser suspendiert, worauf bei 0° 0,9 ml (5,03 mMol) Aethyldiisopropylamin zugegeben wurden. Das Gemisch wurde 2 Stunden bei 0° und danach 4 Stunden bei Raumtemperatur gerührt. Der erhaltene Feststoff wurde abfiltriert, mit Wasser gewaschen, getrocknet und an Kieselgel mit Chloroform/Methanol chromatographiert. Die das gewünschte Produkt enthaltenden Fraktionen wurden vereinigt, mit verdünnter Kaliumhydrogensulfatlösungund danach mit Wasser gewaschen, über Magnesiumsulfat getrocknet und eingeengt, wobei 1,17 g 5-[(9-Fluorenylmethoxyformamido)methyl]-3,6-dimethoxy-9,9-dimethylxanthen-4-essigsäure als farbloses Pulver vom Smp. 134-136° erhalten wurden; IR 1727, 1703, 1606, 1495 $cm^{-1}$.

Beispiel 1.2.1.

Eine Lösung von 0,3 g (0,534 mMol) 5-Aminomethyl-3,6-dimethoxy-9,9-dimethylxanthen-4-essigsäurebenzylester-trifluoracetat in 60 ml Acetonitril wurde zuerst mit 91 mg (1,09 mMol) festem Natriumbicarbonat und dann mit 0,43 g (0,64 mMol) $N^{\alpha}$,$N^{G}$,$N^{G}$-Tris(benzyloxycarbonyl)-L-arginin-N-hydroxysuccinimidester versetzt. Das Gemisch wurde 21 Stunden bei Raumtemperatur gerührt und dann auf Eiswasser gegossen, worauf dreimal mit Methylenchlorid extrahiert wurde. Die vereinigten Extrakte wurden mit Wasser gewaschen, über Magnesiumsulfat getrocknet und eingeengt. Der Rückstand wurde zweimal mit Hexan/Essigester vorgewaschen und dann im gleichen Lösungsmittelgemisch über Kieselgel chromatographiert, wobei 0,45 g 5-[(N2-Benzyloxycarbonyl-N5-(benzyloxycarbonylamino-benzyloxycarbonylimino-methyl)-L-ornithylaminomethyl]-3,6-dimethoxy-9,9-dimethylxanthen-4-essigsäurebenzylester als farbloses amorphes Pulver erhalten wurden; IR 3388, 2958, 1720, 1676, 1607, 1495 $cm^{-1}$.

Beispiel 1.2.2.

Eine Lösung von 0,38 g 5-[N2-Benzyloxycarbonyl-N5-(benzyloxycarbonylamino-benzyloxycarbonylimino-methyl)-L-ornithylaminomethyl]-3,6-dimethoxy-9,9-dimethylxanthen-4-essigsäurebenzylester in 48 ml Trifluoräthanol wurde mit 77 mg Palladiumkohle (10%) versetzt und 2 Stunden unter Wasserstoff gerührt. Danach wurde der Katalysator abfiltriert, und das Filtrat wurde eingeengt. Der erhaltene Rückstand wurde mit Aether

gewaschen, und es wurden 0,2 g 5-L-Arginylaminomethyl-3,6-dimethoxy-9,9-dimethylxanthen-4-essigsäure erhalten; IR 3380-2830, 1656, 1604, 1557, 1492 $cm^{-1}$ absorbiert.

Beispiel 1.2.3.

Eine Lösung von 0,18 g (0,35 mMol) 5-L-Arginylaminomethyl-3,6-dimethoxy-9,9-dimethylxanthen-4-essigsäure in 72 ml Dimethylformamid wurde bei 0°, mit 48 mg trockenem Natriumbicarbonat und dann mit 0,26 g (0,68 mMol) O-Benzotriazol-1-yl-N,N,N',N'-tetramethyl-uroniumhexafluorphosphat behandelt. Die Reaktionslösung wurde 2,5 Stunden bei 0° gerührt und danach zur Trockene eingedampft. Der Rückstand wurde mit Wasser gewaschen und an Kieselgel mit Chloroform/Methanol/Wasser chromatographiert, wobei (S)-8-(3-Guanidinopropyl)-4,12-dimethoxy-17,17-dimethyl-1,15-methano-6,7,8,9,10,11-hexahydro-5H-dibenz-[b,k][1,5,8]oxadiazacyclo dodecin-7,10-dion-hexafluorphosphat erhalten wurden; IR 3405, 2963, 1661, 1535, 1493 $cm^{-1}$.

Beispiel 1.2.4.

Eine Lösung von 0,35 g 5-Aminomethyl-3,6-dimethoxy-9,9-dimethylxanthen-4-essigsäurebenzylester-trifluoracetat in 70 ml Acetonitril wurde auf 0° gekühlt und mit 0,15 g N-Benzyloxycarbonyl-L-alanin versetzt. In mehreren Portionen wurden insgesamt 0,36 g O-Benzotriazol-1-yl-N,N,N',N'-tetramethyluronium-hexafluorphosphat und 0,17 g Natriumbicarbonat zugegeben. Das Reaktionsgemisch wurde insgesamt 36 Stunden gerührt und dabei auf Raumtemperatur erwärmt, worauf mit Eiswasser verdünnt und mit Methylenchlorid extrahiert wurde. Die organische Phase wurde mit Wasser gewaschen, über Magnesiumsulfat getrocknet und eingeengt. Der Rückstand wurde mehrmals über Kieselgel in Chloroform/Methanol chromatographiert, wobei 0,33 g 5-[(N-Benzyloxycarbonyl-L-alanyl)aminomethyl]-3,6-dimethoxy-9,9-dimethylxan-then-4-essigsäurebenzylester als farbloser Schaum erhalten wurden; IR 1728, 1673, 1605, 1494 $cm^{-1}$.

Beispiel 1.2.5.

Eine Lösung von 0,3 g 5-[(N-Benzyloxycarbonyl-L-alanyl)aminomethyl]-3 ,6-dimethoxy-9,9-dimethylxan-then-4-essigsäurebenzylester in 85 ml Trifluoräthanol wurde in Gegenwart von Palladiumkohle (10%) 15 Minuten unter Wasserstoff gerührt. Der Katalysator wurde abfiltriert und mit Trifluoräthanol nachgewaschen. Filtrat und Waschlösung wurden zur Trockene gebracht, und der vereinigte Rückstand wurde über Kieselgel in Chloroform/Methanol/Wasser chromatographiert, wobei 0,18 g 5-(L-Alanylaminomethyl)-3,6-dimethoxy-9,9-dimethylxanthen-4-essigsäure als farbloser Schaum erhalten wurden; IR 3450-2400, 1679, 1606, 1575, 1493 $cm^{-1}$.

Beispiel 1.2.6.

Eine Lösung von 0,15 g 5-(L-Alanylaminomethyl)-3,6-dimethoxy-9,9-dimethylxanthen-4-essigsäure in 84 ml Dimethylformamid wurde auf 0° gekühlt und bei dieser Temperatur mit 49 mg Natriumbicarbonat sowie mit 0,26 g O-Benzotriazol-1-yl-N,N,N',N'-tetramethyluronium-hexafluorphosphatbehandelt. Das Gemisch wurde 5 Stunden bei 0° nachgerührt und anschliessend eingeengt. Der Rückstand wurde in Aether/Chloroform aufgenommen, worauf die erhaltene Lösung mit Wasser gewaschen wurde. Nach Entfernen des Lösungsmittels wurde das Rohprodukt über Kieselgel in Chloroform/Methanol chromatographiert, wobei 87 mg (S)-4,12-Dimethoxy-8,17,17-trimethyl-1,15-methano-6,7,8,9,10,11-hexahydro-5H-dibenz[b,k][1,5,8]-oxadiazacyclododecin-7,10-dion erhalten wurden; IR 3398, 3294 ,2966, 1660, 1603, 1530, 1491, 1463, 1419 $cm^{-1}$.

Beispiel 1.2.7.

Eine Lösung von 0,9 g (1,55 mMol) Calcium-(N2-Benzyloxycarbonyl-N6-tert-butoxycarbonyl-L-lysyl)-L-glutamat in 50 ml Dimethylformamid wurde auf 0° gekühlt, worauf zunächst 0,9 g (1,6 mMol) 5-Aminomethyl-3,6-dimethoxy-9,9-dimethylxanthen-4-essigsäurebenzylester-trifluoracetat, dann 0,91 g (2,4 mMol) O-Benzotriazol-1-yl-N,N,N',N'-tetramethyl-uroniumhexafluorphosphat und dann 0,48 g (4,8 mMol) N-Methylmorpholin zugegeben wurden. Das Gemisch wurde innerhalb von 15 Stunden auf Raumtemperatur erwärmt und dann eingeengt. Der Rückstand wurde in Chloroform aufgenommen, worauf mit 3 x 50 ml Wasser gewaschen und über Magnesiumsulfat getrocknet wurde. Die erhaltene Lösung wurde in Chloroform über Kieselgel filtriert, wobei 0,99 g 5-[(N2-Benzyloxycarbonyl-N6-tert-butoxycarbonyl-L-lysyl-5-O-tert-butyl-L-glutamyl)aminomethyl]-3,6-dimethoxy-9,9-dimethylxanthen-4-essigsäurebenzylester vom Smp. 132-135° erhalten wurde; IR 1707, 1668, 1605, 1525, 1496 $cm^{-1}$.

Beispiel 1.2.8.

Eine Lösung von 0,5 g (0,5 mMol) 5-[(N2-Benzyloxycarbonyl-N6-tertbutoxycarbonyl-L-lysyl-5-O-tert-butyl-L-glutamyl)aminomethyl]-3,6-dimethoxy-9,9-dimethylxanthen-4-essigsäurebenzylester wurde in 40 ml Methanol gelöst, worauf 100 mg Palladiumkohle (10%) zugegeben wurden und 4,5 Stunden unter Wasserstoff gerührt wurde. Nach Abfilrieren des Katalysators und Einengen des Filtrats verblieben 0,39 g 5-[(N6-tert-Butoxycarbonyl-L-lysyl-5-O-tert-butyl-L-glutamyl)aminomethyl]-3,6-dimethoxy-9,9-dimethylxanthen-4-essigsäure als farbloses Pulver vom Smp. 122-125°; IR 3395, 2973, 2935, 2600, 1713, 1682, 1606, 1521, 1494 $cm^{-1}$.

Beispiel 1.2.9.

Eine Lösung von 0,365 g 5-[(N6-tert-Butoxycarbonyl-L-lysyl-5-O-tertbutyl-L-glutamyl)aminomethyl]-3,6-dimethoxy-9,9-dimethylxanthen-4-essigsäure in 500 ml Dimethylformamid wurde auf 0° gekühlt, worauf zuerst 0,54 g O-Benzotriazol-1-yl-N,N,N',N'-tetramethyl-uroniumhexafluorphosphat und dann 0,2 g Natriumbicarbonat zugegeben wurden. Nach 15 Stunden wurde das Gemisch auf Raumtemperatur erwärmt und eingeengt. Das verbleibende Oel wurde in Chloroform aufgenommen, und die erhaltene Lösung wurde dreimal mit Wasser gewaschen, über Magnesiumsulfat getrocknet und eingeengt. Der erhaltene Rückstand wurde mehrmals in Chloroform/Methanol über Kieselgel chromatographiert, wobei 3-[(8S,11S)-11-(4-tert-Butoxycarbonylaminobutyl)-4,15-dimethoxy-20,20-dimethyl-7,10,13-trioxo-1,18-methano-5,6,7,8,9,10,11,12,13,14-decahydrodibenz[b,n][1,5,8,11]oxatriazacyclopentadecin-8-yl]propionsäure-tertbutylester als amorphes Pulver erhalten wurden; IR 3309, 2924, 2933, 1683, 1606, 1522, 1491 $cm^{-1}$.

Beispiel 1.2.10.

50 mg 3-[(8S,11S)-11-(4-tert-Butoxycarbonylaminobutyl)-4,15-dimethoxy-20,20-dimethyl-7,10,13-trioxo-1,18-methano-5,6,7,8,9,10,11,12,13,14-decahydro-dibenz[b,n][1,5,8,11]oxatriazacyclopentadecin-8-yl]-propionsäuretert-butylester wurden in einem Gemisch von Trifluoressigsäure und Methylenchlorid (1/1) gelöst. Die Lösung wurde 90 Minuten bei Raumtemperatur gerührt und dann eingeengt. Der Rückstand wurde in Chloroform/Methanol/Wasser an Kieselgel gereinigt, wobei 22 mg 3-[(8S,11S)-11-(4-Aminobutyl-4,15-dimethoxy-20,20-dimethyl-7,10,13-trioxa-1,18-methano-5,6,7,8,9,10,11,12,13,14-decahydrodibenz[b,n]-[1,5,8,11]oxatriazacyclopentadecin-8-yl]propionsäure erhalten wurden; IR 3419, 3304, 2961, 1671, 1605, 1531, 1493 $cm^{-1}$.

Beispiel 1.2.11.

Eine Lösung von 0,55 g (1,5 mMol) N-[(2S,3R)-2-Benzyloxycarbonyl-3-tert-butoxy-butyryl]-glycin in 75 ml Dimethylformamid wurde auf 0-5° gekühlt und zuerst mit 0,84 g (1,5 mMol) 5-Aminomethyl-3,6-dimethoxy-9,9-dimethylxanthen-4-essigsäurebenzylester-trifluoracetat, dann mit 0,85 g (2,25 mMol) O-Benzotriazol-1-yl-N,N,N',N'-tetramethyl-uroniumhexafluorphosphat und schliesslich mit 0,5 ml N-Methylmorpholin versetzt. Das erhaltene Gemisch wurde unter Rühren im Verlauf von 15 Stunden auf Raumtemperatur gebracht und danach vom Lösungsmittel befreit. Der Rückstand wurde in 100 ml Essigsäureäthylester aufgenommen, worauf die Lösung mit 2 x 50 ml Wasser gewaschen, über Natriumsulfat getrocknet und eingedampft wurde. Der als Rückstand verbliebene Schaum wurde an Kieselgel in Chloroform/Methanol chromatographiert, wobei 0,94 g 5-[(N-Benzyloxycarbonyl-O-tert-butyl-L-threonyl-glycyl)aminomethyl]-3,6-dimethoxy-9,9-dimethylxanthen-4-essigsäurebenzylester erhalten wurden; IR 3401, 2972, 1726, 1667, 1605, 1493 $cm^{-1}$.

Beispiel 1.2.12.

Eine Lösung von 0,67 g (0,84 mMol) 5-[(N-Benzyloxycarbonyl-O-tert-butyl-L-threonyl-glycyl)-aminomethyl]-3,6-dimethoxy-9,9-dimethylxanthen-4-essigsäurebenzylester in 100 ml Methanol wurde mit 140 mg Palladiumkohie (10%) versetzt und 1 Stunde unter Wasserstoff gerührt. Danach wurde der Katalysator abfiltriert, und das Filtrat wurde zur Trockene gebracht. Als Rückstand verblieben 0,46 g 5-[O-tert-Butyl-L-threonyl-glycyl)aminomethyl]-3,6-dimethoxy-9,9-dimethylxanthen-4-essigsäure als farbloses

amorphes Pulver; IR 3400-2500, 1675, 1606, 1578, 1531, 1493 $cm^{-1}$.

Beispiel 1.2.13.

Eine Lösung von 50 mg 5-[(O-tert-Butyl-L-threonyl-glycyl)aminomethyl]-3,6-dimethoxy-9,9-dimethylxanthen-4-essigsäure in 105 ml Dimethylformamid wurde auf 0° gekühlt, worauf zuerst 99 mg O-Benzotriazol-1-yl-N,N,N',N'-tetramethyl-uroniumhexafluorphosphat und dann 37 mg Natriumbicarbonat zugegeben wurden. Die erhaltene Lösung wurde über Nacht auf Raumtemperatur erwärmt und dann eingedampft. Der Rückstand wurde in Essigsäureäthylester/Wasser aufgenommen. Die organische Phase wurde abgetrennt, mit Wasser nachgewaschen, über Magnesiumsulfat getrocknet und vom Lösungsmittel befreit. Der verbleibende Rückstand wurde in Chloroform/Methanol über Kieselgel filtriert, wobei 49 mg (11S)-[(R)-11-(1-tert-Butoxyethyl)]-4,15-dimethoxy-20,20-dimethyl-1,18-methano-5,6,7,8,9,10,11,12,13,14-decahydro-dibenz[b,n]-[1,5,8,11]oxatriazacyclopentadecin-7,10,13-trion als schwach gelbes, amorphes Pulver erhalten wurden; IR 3426, 3306, 2972, 1684, 1653, 1605, 1527, 1492 $cm^{-1}$.

Beispiel 1.2.14.

Eine Lösung von 0,27 g (0,48 mMol) (11S)-[(R)-11-(1-tert-Butoxyethyl)]-4,15-dimethoxy-20,20-dimethyl-1,18-methano-5,6,7,8,9,10,11,12,13,14-decahydro-dibenz[b,n][1,5,8,11]-oxatriazacyclopentadecin-7,10,13-trion in 4 ml Methylenchlorid wurde auf 0° gekühlt und langsam mit 4 ml Trifluoressigsäure versetzt. Die erhaltene Lösung wurde auf Raumtemperatur erwärmt, 5 Stunden nachgerührt und zur Trockene eingedampft. Der Rückstand wurde in Chloroform/Methanol über Kieselgel chromatographiert, wobei 0,22 g (11S)-[(R)-11-(1-Hydroxyethyl)]-4,15-dimethoxy-20,20-dimethyl-1,18-methano-5,6,7,8,9,10,11,12,13,14-decahydro-dibenz[b,n][1,5,8,11]-oxatriazacyclopentadecin-7,10,13-trion als leicht gelblicher Schaum erhalten wurden; IR 3432, 3274, 2968, 2934, 1678, 1645, 1605, 1533, 1493 $cm^{-1}$. Eine Probe dieses Materials wurde aus Aethanol/Wasser umkristallisiert und zeigte dann einen Smp. von 194-196° (Zers.).

Beispiel 1.2.15.

Eine Lösung von 0,52 g (0,8 mMol) N-(9H-Fluoren-9-ylmethoxycarbonyl)-L-isoleucyl-O-tert-butyl-L-tyrosyl-L-alanin in 20 ml Dimethylformamid wurde auf 0° gekühlt und mit 0,45 g (0,8 mMol) 5-Aminomethyl-3,6-dimethoxy-9,9-dimethylxanthen-4-essigsäurebenzylester-trifluoracetat sowie mit 0,45 g (1,2 mMol) O-Benzotriazol-1-yl-N,N,N',N'-tetramethyluroniumhexafluorphosphat versetzt. Danach wurden 0,31 g (2,4 mMol) Aethyldiisopropylamin zugegeben, worauf das erhaltene Reaktionsgemisch innerhalb von 3 Stunden von 0° auf Raumtemperatur erwärmt und eingeengt wurde. Der verbleibende Rückstand wurde in Chloroform aufgenommen, worauf die erhaltene Lösung mit Wasser gewaschen und über Magnesiumsulfat getrocknet wurde. Nach Chromatographie über Kieselgel (Chloroform) wurden 0,77 g 3,6-Dimethoxy-9,9-dimethyl-5-[(N-(9H-fluoren-9-ylmethoxycarbonyl)-L-isoleucyl-O-tert-butyl-L-tyrosyl-L-alanyl)aminomethyl]-xanthen-4-essigsäurebenzylester vom Smp. 218-220° erhalten; IR 1728, 1662, 1606, 1503, 1492 $cm^{-1}$.

Beispiel 1.2.16.

Eine Lösung von 0,74 g (0,73 mMol) 3,6-Dimethoxy-9,9-dimethyl-5-[(N-(9H-fluoren-9-yl-methoxycarbonyl)-L-isoleucyl-O-tert-butyl-L-tyrosyl-L-alanyl)aminomethyl]xanthen-4-essigsäurebenzylester in 7 ml Dimethylformamid wurde bei 0° in Gegenwart von 0,7 ml Diäthylamin 1 Stunde gerührt. Das Reaktionsgemisch wurde zur Trockene gebracht. Der erhaltene Rückstand wurde in Chloroform/Methanol über Kieselgel chromatographiert, wobei 0,53 g 3,6-Dimethoxy-9,9-dimethyl-5-[(L-isoleucyl-O-tert-butyl-L-tyrosyl-L-alanyl)-aminomethyl]xanthen-4-essigsäurebenzylester erhalten wurden; IR 3292, 2970, 1742, 1672, 1655, 1627, 1605, 1536, 1502, 1499 $cm^{-1}$.

Beispiel 1.2.17.

Eine Lösung von 0,27 g 3,6-Dimethoxy-9,9-dimethyl-5-[(L-isoleucyl-O-tert-butyl-L-tyrosyl-L-alanyl)-aminomethyl]xanthen-4-essigsäurebenzylester in 15 ml Methanol wurde mit 50 mg Palladiumkohle (10%) versetzt und 3 Stunden unter Wasserstoff gerührt. Nach dem Abfiltrieren des Katalysators wurde das Lösungsmittel entfernt, und der Rückstand wurde mit Aether gewaschen. Es verblieben 0,24 g 3,6-Dimethoxy-9,9-dimethyl-5-[(L-isoleucyl-O-tert-butyl-L-tyrosyl-L-alanyl)aminomethyl]xanthen-4-essigsäure vom Smp. 146-149°; IR 1662, 1606, 1506, 1494 $cm^{-1}$.

Beispiel 1.2.18.

Eine Lösung von 0,24 g (0,32 mMol) 3,6-Dimethoxy-9,9-dimethyl-5-[(L-isoleucyl-O-tert-butyl-L-tyrosyl-L-alanyl)aminomethyl]xanthen-4-essigsäure in 480 ml Dimethylformamid wurde auf 0° gekühlt und zuerst mit 0,36 g (0,96 mMol) O-Benzotriazol-1-yl-N,N,N',N'-tetramethyl-uroniumhexafluorphosphat und dann mit 0,16 g (1,90 mMol) Natriumbicarbonat versetzt. Das Reaktionsgemisch wurde 3 Stunden bei 0° gerührt, auf Raumtemperatur erwärmt und eingeengt. Der Rückstand wurde in Chloroform aufgenommen, und die erhaltene Lösung wurde mit Wasser gewaschen, über Magnesiumsulfat getrocknet und konzentriert. Nach Chromatographie über Kieselgel (in Chloroform/Methanol) wurden 0,16 g 4,5-Cyclo-[acetyl-L-isoleucyl-O-tert-butyl-L-tyrosyl-L-alanylaminomethyl]-3,6-dimethoxy-9,9-dimethylxanthen als farbloses Pulver erhalten; IR 3406, 3328, 2971, 1664, 1605, 1506 $cm^{-1}$.

Beispiel 1.2.19.

Eine Lösung von 0,1 g (0,134 mMol) 4,5-Cyclo-[acetyl-L-isoleucyl-O-tert-butyl-L-tyrosyl-L-alanylaminomethyl]-3,6-dimethoxy-9,9-dimethylxanthenin 4 ml Methylenchlorid wurde auf 0° gekühlt, mit 0,5 ml Trifluoressigsäure versetzt und danach 5 Stunden bei 0° gerührt. Nach dem Erwärmen auf Raumtemperatur wurde das Gemisch eingeengt. Der verbleibende Rückstand wurde mit Aether gewaschen, wobei 91 mg 4,5-Cyclo-[acetyl-L-isoleucyl-L-tyrosyl-L-alanyl-aminomethyl]-3,6-dimethoxy-9,9-dimethylxanthen vom Smp. 193-196° (Zers.) erhalten wurden; IR 3331, 2965, 1662, 1608, 1515, 1498 $cm^{-1}$.

Beispiel 1.2.20.

Eine Lösung von 45 mg N-Benzyloxycarbonyl-L-valyl-L-alanyl-L-alanyl-L-phenylalanyl-L-leucyl-L-alanyl-L-leucyl-L-alanin in 5 ml Dimethylformamid wurde mit 27 mg 5-Aminomethyl-3,6-dimethoxy-9,9-dimethylxanthen-4-essigsäurebenzylester-trifluoracetat und 28 mg O-Benzotriazol-1-yl-N,N,N',N'-tetramethyl-uroniumhexafluorphosphat versetzt und danach auf 0° abgekühlt. Nach Zugabe von 16 mg Aethyldiisopropylamin wurde das Gemisch 15 Stunden unter Erwärmen auf Raumtemperatur nachgerührt. Der nach Entfernen des Lösungsmittels verbleibende Rückstand wurde nacheinander mit Wasser und Methanol gewaschen und an Kieselgel in Chloroform/Methanol chromatographiert, wobei 5-[(N-Benzyloxycarbonyl-L-valyl-L-alanyl-L-alanyl-L-phenylalanyl-L-leucyl-L-alanyl-L-leucyl-L-alanyl)-aminomethyl]-3,6-dimethoxy-9,9-dimethylxanthen-4-essigsäurebenzylester vom Smp. 287-290° erhalten wurde; IR 3284, 2959, 1632, 1532, 1500 $cm^{-1}$.

Beispiel 1.2.21.

100 mg 5-[(N-Benzyloxycarbonyl-L-valyl-L-alanyl-L-alanyl-L-phenylalanyl-L-leucyl-L-alanyl-L-leucyl-L-alanyl)aminomethyl]-3,6-dimethoxy-9,9-dimethylxanthen-4-essigsäurebenzylester wurden in 20 ml Hexafluorisopropanol gelöst, mit 60 mg Palladium auf Calciumcarbonat (10%) versetzt und 3 Stunden unter Wasserstoff gerührt. Nach dem Abfiltrieren des Katalysators wurde das Filtrat eingeengt. Der verbleibende Rückstand wurde mehrmals mit Methanol gewaschen und danach über Kieselgel in Chloroform/Methanol/Hexafluorisopropanol chromatographiert, wobei 65 mg 3,6-Dimethoxy-9,9-dimethyl-5-[(L-valyl-L-alanyl-L-alanyl-L-phenylalanyl-L-leucyl-L-alanyl-L-leucyl-L-alanyl)aminomethyl]xanthen-4-essigsäure erhalten wurden; IR 3393, 3288, 2963, 1685, 1636, 1531 $cm^{-1}$.

Beispiel 1.2.22.

Eine Lösung von 104 mg 3,6-Dimethoxy-9,9-dimethyl-5-[(L-valyl-L-alanyl-L-alanyl-L-phenylalanyl-L-leucyl-L-alanyl-L-leucyl-L-alanyl)aminomethyl]xanthen-4-essigsäure in 140 ml Dimethylformamid wurde auf 0° gekühlt und mit 106 mg O-Benzotriazol-1-yl-N,N,N',N'-tetramethyluroniumhexafluorphosphat sowie mit 40 mg Natriumbicarbonat versetzt. Das Gemisch wurde 25 Stunden unter Erwärmen auf Raumtemperatur nachgerührt. Nach Entfernen des Lösungsmittels wurde der Rückstand mehrmals über Kieselgel in Chloroform/Methanol chromatographiert. Nach Kristallisation aus Chloroform/Methanol verblieben 17 mg farbloses 4,5-Cyclo-[acetyl-L-valyl-L-alanyl-L-alanyl-L-phenylalayl-L-leucyl-L-alanylaminomethyl]-3,6-dimethoxy-9,9-dimethylxanthen vom Smp. 277-280°; IR 3322, 2958, 1650, 1523 $cm^{-1}$.

Beispiel 1.2.23.

Eine Lösung von 0,17g 5-Aminomethyl-3,6-dimethoxy-9,9-dimethylxanthen-4-essigsäurebenzylester-trifluoracetat und 0,14 g N-(9-Fluorenylmethoxycarbonyl)-4-O-tert-butyl-D-asparaginsäure in 5 ml Dimethylformamid wurde mit 0,11g O-Benzotriazol-1-yl-N,N,N'N'-tetramethyluroniumtetrafluoborat sowie mit 0.11 ml Diisopropyläthylamin versetzt. Das Gemisch wurde 1 Stunde bei Raumtemperatur gerührt und danach auf verdünnte Natriumbicarbonatlösung gegossen. Der entstandene Niederschlag wurde abfiltriert, mit Wasser, $KHSO_4/K_2SO_4$-Lösung und erneut mit Wasser gewaschen und bei 40° im Vakuum getrocknet, wobei 0,25g 5-[(N-(9-Fluorenylmethoxycarbonyl)-4-O-tert-butyl-D-asparty)aminomethyl]-3,6-dimethoxy-9,9-dimethylxanthen-4-essigsäurebenzylester erhalten wurden; MS: 841,5 $(M+H)^+$.

Beispiel 1.2.24.

Eine Lösung von 0,21g 5-[(N-(9-Fluorenylmethoxycarbonyl)-4-O-tertbutyl-D-aspartyl)aminomethyl]-3,6-dimethoxy-9,9-dimethylxanthen-4-essigsäurebenzylester in 8 ml Dimethylformamid wurde mit 2 ml Piperidin versetzt, 30 Minuten bei Raumtemperatur stehen gelassen und anschliessend im Vakuum eingeengt. Der verbleibende Rückstand wurde nacheinander mit 0,11 g N-(9-Fluorenylmethoxycarbonyl-D-tryptophan, 0,08g O-Benzotrial-1-yl-N,N,N',N'-tetramethyluroniumtetrafluorborat und 0,056 ml Diisopropyläthylamin versetzt. Das Gemisch wurde 2 Stunden bei Raumtemperatur gerührt und danach mit verdünnter Natriumbicarbonatlösung versetzt, wobei ein Niederschlag ausfiel. Der Niederschlag wurde abfiltriert und in Essigsäureäthylester gelöst. Die Lösung wurde nacheinander mit gesättigter Natriumbicarbonatlösung, $KHSO_4/K_2SO_4$-Lösung und gesättigter Kochsalzlösung gewaschen, über Natriumsulfat getrocknet und eingeengt. Der Rückstand wurde aus Aethanol/Wasser gefällt und bei 40° im Vakuum getrocknet, wobei 0,22g 5-[(N-(9-Fluorenylmethoxycarbonyl)-D-tryptophanyl-4-O-tert-butyl-D-aspartyl)aminomethyl]-3,6-dimethoxy-9,9-dimethylxanthen-4-essigsäurebenzylester erhalten wurden; MS: 1027.2 $(M+H)^+$.

Beispiel 1.2.25.

Eine Lösung von 0,19g 5-[(N-(9-Fluorenylmethoxycarbonyl)-D-tryptophanyl-4-O-tert-butyl-D-aspartyl)-aminomethyl]-3,6-dimethoxy-9,9-dimethylxanthen-4-essigsäurebenzylester in 8 ml Dimethylformamid wurde mit 2 ml Piperidin versetzt, worauf das Gemisch 30 Minuten bei Raumtemperatur stehen gelassen und dann eingeengt wurde. Der Rückstand wurde mit Hexan unter Zusatz von wenig Aether gewaschen und in 3 ml Dimethylformamid gelöst. Die Lösung wurde mit 0,09g N-(Benzyloxycarbonyl)-L-leucin-N-hydroxysuccinimidester versetzt und 3 Stunden bei Raumtemperatur gerührt, worauf verdünnte Natriumbicarbonatlösung zugegeben wurde. Der Niederschlag wurde abfiltriert, mit Wasser, $KHSO_4/K_2SO_4$-Lösung und erneut mit Wasser gewaschen und in 10 ml Methanol gelöst. Die erhaltene Lösung wurde in Gegenwart von 16 mg Palladiumkohle (10%) 4 Stunden unter Wasserstoff gerührt. Der Katalysator wurde abfiltriert, und das Filtrat wurde vom Lösungsmittel befreit. Der Rückstand wurde in 10 ml Dimethylformamid aufgenommen und mit 0,06 ml Diisopropyläthylamin versetzt. Die erhaltene Lösung wurde innerhalb von 30 Minuten unter Rühren zu einer Lösung von 0,13 g O-Benzotriazol-1-yl-N,N,N',N'-tetramethyluroniumtetrafluorborat in 50 ml Dimethylformamid gegeben. Das Gemisch wurde noch 2 Stunden bei Raumtemperatur nachgerührt und danach in verdünnte Natriumbicarbonatlösung eingetropft. Der ausgefallene Niederschlag wurde abfiltriert, mit $KHSO_4/K_2SO_4$-Lösung nachgewaschen, im Vakuum getrocknet und in einem Gemisch von 9,5 ml Trifluoressigsäure und 0,5 ml Wasser aufgenommen. Die Lösung wurde 1 Stunde bei Raumtemperatur stehen gelassen und dann vom Lösungsmittel befreit. Der Rückstand wurde mittels HPLC (C-18 Phase, Gradient Wasser/Aethanol mit 0,1% Trifluoressigsäure) gereinigt, wobei 34 mg 4,5-Cyclo-[acetyl-L-leucyl-D-tryptophanyl-D-aspartylaminomethyl]-3,6-dimethoxy-9,9-dimethylxanthen erhalten wurden; IR 3407, 2958, 1660, 1524 und 1493 $cm^{-1}$; MS: 754.2 $(M+H)^+$.

Beispiel 1.2.26.

Eine Lösung von 0,25 g 5-Aminomethyl-3,6-dimethoxy-9,9-dimethylxanthen-4-essigsäurebenzylester-trifluoracetet in 3 ml Dimethylformamid wurde nacheinander mit 0,36 g $N^{\alpha}$-Benzyloxycarbonyl-$N^{G}$-(2,2,5,7,8-pentamethylchroman-6-sulfonyl)-L-arginylglycyl-4-O-tert-butyl-L-aspartyl-L-valin, 0,17 g O-Benzotriazol-1-yl-N,N,N',N'-tetramethyluroniumhexafluorphosphat und 0,17 ml Diisopropyläthylamin versetzt. Nach 30-minütigem Rühren wurde das Reaktionsgemisch in verdünnte Natriumbicarbonatlösung gegossen. Der erhaltene Niederschlag wurde abfiltriert, mit Wasser gewaschen und im Vakuum getrocknet, wobei 0,5 g 5-[($N^{\alpha}$-Benzyloxycarbonyl-$N^{G}$-(2,2,5,7,8-pentamethylchroman-6-sulfonyl)-L-arginylglycyl-4-O-tert-butyl-L-aspartyl-L-valyl)aminomethyl]-3,6-dimethoxy-9,9-dimethylxanthen-4-essigsäurebenzylester erhalten wurden; MS: 1331.6 $(M+H)^+$.

Beispiel 1.2.27.

Eine Lösung von 0,47 g 5-[$N^\alpha$-Benzyloxycarbonyl-$N^G$-(2,2,5,7,8-pentamethylchroman-6-sulfonyl)-L-arginylglycyl-4-O-tert-butyl-L-asparty-L-valyl)aminomethyl]-3,6-dimethoxy-9,9-dimethylxanthen-4-essigsäurebenzylester in 25 ml Trifluoräthanol wurde in Gegenwart von Palladiumkohle (10%) 2 Stunden unter Wasserstoff gerührt. Danach wurde der Katalysator abfiltriert, und das Filtrat wurde zur Trockene gebracht. Der Rückstand wurde zusammen mit 0,34 ml Diisopropyläthylamin in 20 ml Dimethylformamid aufgenommen. Die erhaltene Lösung wurde innerhalb von 20 Minuten zu einer Lösung von 0,66 g O-Benzotriazol-1-yl-N,N,N',N'-tetramethyluroniumhexafluorphosphat in 50 ml Dimethylformamid getropft, worauf 1 Stunde bei Raumtemperatur nachgerührt wurde. Anschliessend wurde der grösste Teil des Lösungsmittels im Vakuum entfernt, worauf das erhaltene Konzentrat in verdünnte Natriumbicarbonatlösung eingegossen wurde. Der entstandene Niederschlag wurde abfiltriert, mit Wasser gewaschen und getrocknet. Dieses Rohprodukt wurde in einer Mischung von 20 ml Trifluoressigsäure, 0,5 ml Wasser und 0,2 ml Phenol gelöst Die Lösung wurde 1 Stunde bei Raumtemperatur stehen gelassen und dann eingeengt. Der verbleibende Rückstand wurde mit Aether digeriert und aus Eisessig lyophilisiert. Nach Reinigung mittels HPLC (C-18 Phase; Gradient: Wasser/Aethanol mit 0,1% Trifluoressigsäure) wurden 75 mg 4,5-Cyclo-[acetyl-L-arginyl-glycyl-L-aspartyl-L-valylaminomethyl]-3,6-dimethoxy-9,9-dimethylxanthen-trifluoracetat erhalten; IR 3364, 2965, 2841, 1664, 1577 und 1497 $cm^{-1}$; MS: 767 $(M+H)^+$.

Beispiel 1.3.1.

Eine Lösung von 0,13 g (0,24 mMol) N-Benzyloxcarbonyl-L-alanyl-O-tert-butyl-L-threonyl-L-valylglycin und 0,11 g (0,3 mMol) O-Benzotriazol-1-yl-N,N,N',N',-tetramethyl-uroniumhexafluorphosphat in 8 ml Dimethylformamid wurde mit 0,08 g (0,2 mMol) 5-Aminomethyl-3,6-dimethoxy-9,9-dimethylxanthen-4-essigsäuremethylester-hydrochlorid versetzt. Das Gemisch wurde auf 0° gekühlt, mit 0,07 g (0,54 mMol) Aethyldiisopropylamin versetzt und 19 Stunden bei 0° nachgerührt. Nach Erwärmen auf Raumtemperatur wurde Wasser zugegeben, worauf der gebildete Niederschlag abfiltriert, mit Wasser gewaschen und in Chloroform/Methanol über Kieselgel gereinigt wurde. Das erhaltene Produkt wurde mit Aether gefällt, wobei 0,16 g 5-[(N-Benzyloxycarbonyl-L-alanyl-O-tert-butyl-L-threonyl-L-valyl-glycyl)aminomethyl]-3,6-dimethoxy-9,9-dimethylxanthen-4-essigsäuremethylester vom Smp. 181-184° erhalten wurden; IR 3277, 2968, 1738, 1671, 1632, 1600, 1524, 1495 $cm^{-1}$.

Beispiel 1.3.2.

Eine Lösung von 70 mg 5-[(N-Benzyloxarbonyl-L-alanyl-O-tert-butyl-L-threonyl-L-valyl-glycyl)-aminomethyl]-3,6-dimethoxy-9,9-dimethylxanthen-4-essigsäuremethylester in 20 ml Dioxan wurde mit 47 mg Palladiumkohle (10%) versetzt und 19 Stunden unter Wasserstoffgerührt. Nach dem Abfiltrieren des Katalysators wurde das Filtrat eingeengt, wobei 59 mg farbloser 5-[(L-Alanyl-O-tert-butyl-L-threonyl-L-valylglycyl)aminomethyl]-3,6-dimethoxy-9,9-dimethylxanthen-4-essigsäuremethylester erhalten wurden; IR 3300, 2970, 1741, 1680, 1658, 1631, 1523, 1494 $cm^{-1}$.

Beispiel 1.3.3.

Eine Lösung von 30 mg 5-[(L-Alanyl-O-tert-butyl-L-threonyl-L-valylglycyl)aminomethyl]-3,6-dimethoxy-9,9-dimethylxanthen-4-essigsäuremethylester in 0,6 ml Pyridin wurde auf 0° gekühlt und in mehreren Portionen mit insgesamt 0,17 ml 2N-Natronlauge versetzt. Das erhaltene Reaktionsgemisch wurde 24 Stunden gerührt und danach zur Trockene gebracht, und der Rückstand wurde in Wasser aufgenommen. Die erhaltene Lösung wurde mit 1N-Salzsäure angesäuert, mit Essigester gewaschen und zur Trockene gebracht. Der farblose Rückstand wurde in 20 ml Dimethylformamid aufgenommen, und die erhaltene Lösung wurde auf 0° gekühlt, bei dieser Temperatur mit 37 mg Natriumbicarbonat und 69 mg Diphenylphosphorylazid versetzt und 14 Stunden unter langsamem Erwärmen auf Raumtemperatur nachgerührt. Der nach Entfernen des Lösungsmittel verbleibende Rückstand wurde mit Pentan gewaschen und danach zwischen Wasser und Essigester verteilt. Die vereinigten organischen Phasen wurden über Magnesiumsulfat getrocknet und eingeengt. Der Rückstand wurde in Chloroform/Methanol über Kieselgel chromatographiert, wobei 7 mg 4,5-Cyclo-[acetyl-L-alanyl-O-tert-butyl-L-threonyl-L-valyl-gycylaminomethyl]-3,6-dimethoxy-9,9-dimethylxanthenerhalten wurden; IR 3415, 3345, 2971, 2931, 1672, 1605, 1515 $cm^{-1}$.

Beispiel 1.3.4.

Eine Lösung von 4 mg 4,5-Cyclo-[acetyl-L-alanyl-O-tert-butyl-L-threonyl-L-valyl-gycylaminomethyl]-3,6-dimethoxy-9,9-dimethylxanthen in 0,4 ml Trifluoressigsäure wurde mit zwei Tropfen Wasser versetzt und 2 Stunden bei Raumtemperatur gerührt. Das Reaktionsgemisch wurde zur Trockene gebracht, und der erhaltene Rückstand wurde über Kieselgel chromatographiert und anschliessend aus Acetonitril umkristallisiert. Es wurden 3 mg 4,5-Cyclo-[acetyl-L-alanyl-L-threonyl-L-valyl-glycylaminomethyl]-3,6-dimethoxy-9,9-dimethylxanthen vom Smp. 239-242° erhalten; IR 3395, 2965, 2933, 1672, 1608, 1531, 1494 $cm^{-1}$.

Beispiel 1.4.1.

Eine Lösung von 0,46 g 5-[(1-tert-Butoxyformamido)methyl]-3,6-dimethoxy-9,9-dimethylxanthen-4-essigsäure und 0,3 g O-Benzyl-L-glutamin-hydrochlorid in 10 ml Dimethylformamid wurde auf 0° gekühlt und zuerst mit 0,45 g O-Benzotriazol-1-yl-N,N,N',N'-tetramethyluroniumhexafluorphosphat und dann mit 0,37 ml Diisopropyläthylamin versetzt. Die erhaltene Lösung wurde auf Raumtemperatur erwärmt, 2 Stunden nachgerührt und dann in verdünnte Natriumbicarbonatlösung gegossen. Der ausgefallene Niederschlag wurde abfiltriert, mit Wasser gewaschen und im Vakuum getrocknet, wobei 0,59 g 5-[N-(tert-Butyloxycarbonyl)aminomethyl]-3,6-dimethoxy-9,9-dimethoxyxanthen-4-essigsäure-(O-benzyl-L-glutaminyl)-amid erhalten wurden; MS: 676 $(M+H)^+$.

Beispiel 1.4.2.

Eine Lösung von 0,54 g 5-[N-(tert-Butyloxycarbonyl)aminomethyl]-3,6-dimethoxy-9,9-dimethoxyxanthen-4-essigsäure-(O-benzyl-L-glutaminyl)-amid in 10 ml Trifluoressigsäure und 0,5 ml Wasser wurde 10 Minuten bei Raumtemperatur stehen gelassen und dann eingeengt. Der Rückstand wurde zweimal mit Toluol zur Trockene gebracht und dann in 10 ml Dimethylformamid aufgenommen. Die erhaltene Lösung wurde nacheinander mit 0,46 g N-(9-Fluorenylmethoxycarbonyl)-O-tert-butyl-L-tyrosin, 0,42 g O-Benzotriazol-1-yl-N,N,N',N'-tetramethyluroniumhexafluorphosphat und 0.35ml Diisopropyläthylamin versetzt. Das Gemisch wurde 1,5 Stunden bei Raumtemperatur gerührt und danach auf verdünnte Natriumbicarbonatlösung gegossen. Der ausgefallene Niederschlag wurde abfiltriert, getrocknet und aus Hexan/Essigsäureäthylester umgefällt, wobei 0,47 g 5-[(N-(9-Fluorenylmethoxycarbonyl)-O-tert-butyl-L-tyrosyl)aminomethyl]-3,6-dimethoxy-9,9-dimethylxanthen-4-essigsäure-(O-benzyl-L-glutaminyl)amid erhalten wurden; MS: 1017.3 $(M+H)^+$.

Beispiel 1.4.3.

Eine Lösung von 0,39 g 5-[(N-(9-Fluorenylmethoxycarbonyl)-O-tert-butyl-L-tyrosyl)aminomethyl]-3,6-dimethoxy-9,9-dimethylxanthen-4-essigsäure-(O-benzyl-L-glutaminyl)amid in 6 ml Dimethylformamid und 4 ml Piperidin wurde 15 Minuten bei Raumtemperatur stehen gelassen und dann zur Trockene gebracht. Der verbleibende Rückstand wurde mit Hexan digeriert und anschliessend in 5 ml Dimethylformamid gelöst. Die erhaltene Lösung wurde auf 0° gekühlt und nacheinander mit 0,36 g $N^\alpha$,$N^G$,$N^G$-Tris(benzyloxycarbonyl)-L-arginylglycyl-4-O-tert-butyl-L-aspartyl-L-valin, 0,06 g N-Hydroxybenzotriazolhydrat, 0,13g O-(1,2-Dihydro-2-oxo-1-pyridyl)-N,N,N',N'-tetramethyluroniumtetrafluorborat und 0,15 ml Diisopropyläthylamin versetzt Das Reaktionsgemisch wurde 30 Minuten bei Raumtemperatur gerührt und dann in verdünnte Natriumbicarbonatlösung eingegossen. Der ausgefallene Niederschlag wurde abfiltriert, mit Wasser und $KHSO_4/K_2SO_4$-Lösung gewaschen und aus Hexan/Essigsäureäthylester umgefällt, wobei 0,53g 5-[$N^\alpha$.NG,NG-Tris-(benzyloxycarbonyl)-L-arginylglycyl-4-O-tert-butyl-L-aspartyl-L-valyl-O-tert-butyl-L-tyrosyl)aminomethyl]-3,6-dimethoxy-9,9-dimethylxanthen-4-essigsäure-(O-benzyl-L-glutaminyl)-amid erhalten wurden; MS: 1681.7 $(M+H)^+$.

Beispiel 1.4.4.

Eine Lösung von 0,47g 5-[($N^\alpha$,$N^G$,$N^G$-Tris(benzyloxycarbonyl)-L-arginylglycyl-4-O-tert-butyl-L-aspartyl-L-valyl-O-tert-butyl-L-tyrosyl)aminomethyl]-3,6-dimethoxy-9,9-dimethylxanthen-4-essigsäure-(O-benzyl-L-glutaminyl)amid in 20 ml Trifluoräthanol wurde unter Zusatz von Palladiumkohle (10%) bei Raumtemperatur und Normaldruck hydriert. Danach wurde der Katalysator abfiltriert, und das Eiltrat wurde eingeengt. Der Rückstand wurde in 20 ml Dimethylformamid aufgenommen, und die erhaltene Lösung wurde mit 0,04 g N-Hydroxybenzotriazol-hydrat versetzt. Diese Lösung wurde innerhalb von 20 Minuten zu einer Lösung von 0,24g O-(1,2-Dihydro-2-oxo-1-pyridyl)-N,N,N',N'-tetramethyluroniumtetrafluorborat und 0,14 ml Diisopropyläthylamin in 80 ml Dimethylformamid getropft. Das Reaktionsgemisch wurde 1 Stunde bei Raumtemperatur gerührt und danach eingeengt. Der Rückstand wurde zwischen n-Butanol/Essigsäureäthylester und gesättigter Natriumbicarbonatlösung verteilt. Die organische Phase wurde abgetrennt, mit gesättigter Kochsalzlösung nachgewaschen und vom Lösungsmitel befreit. Der verbleibende Rückstand wurde in 25 ml Trifluoressigsäure gelöst, und die Lösung wurde 1 Stunde bei Raumtemperatur stehen gelassen und dann eingeengt. Das als Rückstand verbleibende Rohprodukt wurde mittels HPLC (C-18 Phase; Gradient: Wasser/Aethanol mit 0,1% Trifluoressigsäure) gereinigt, wobei 79 mg 4,5-Cyclo-[acetyl-L-glutaminyl-L-arginyl-glycyl-L-aspartyl-L-valyl-L-tyrosylaminomethyl]-3,6-dimethoxy-9,9-dimethylxanthen-trifluoracetat erhalten wurden; IR 3379, 2966, 1663 und 1516 $cm^{-1}$; MS: 1058.4 $(M+H)^+$.

Beispiel 1.5.1.

In einem Peptidsynthesizer [Labortec SPG40] wurden 20g p- Hydroxymethylphenoxy-polystyrol-Harz in 250 ml DMF suspendiert und dann nacheinander mit 9,88g Fmoc-Asp-OAllyl [A. Trzeciak, W. Bannwarth; Tetrahedron Letters, 33, 4557-4560(1992)], 8,34g TBTU, 4,45 ml DIPEA und 317 mg 4-Dimethylaminopyridin versetzt, worauf 18 Stunden bei Raumtemperatur geschüttelt wurde. Das veresterte Harz wurde mit Dimethylformamid, Isopropanol und Diäthylether gewaschen und im Vakuum getrocknet, und es wurden 23,8g beladenes Harz (0.4m Mol / g) erhalten.

4g des beladenen Harzes wurden dem Synthesezyklus gemäss Beispiel 2.2.2. unterworfen und mit den Aminosäurenderivaten Fmoc-Pro-OH, Fmoc-Phe-OH, Fmoc-Asp(OBut)-OH, Fmoc-Gly-OH, Fmoc-Arg(Pmc)-OH, Fmoc-Ala-OH und Fmoc-Ile-OH gekuppelt. Nach beendeter Synthese wurde das Harz getrocknet; Ausbeute 6g.

0.8g des erhaltenen Harzes wurden mit 10 ml einer 20% igen Lösung von Piperdin in DMF behandelt, worauf mit 170 mg 5-[(9-Fluorenylmethoxyformamido)methyl]-3,6-dimethoxy-9,9-dimethylxanthen-4-essigsäure und 103 mg TBTU und 0.055 ml DIPEA gekuppelt wurde. Zur Spaltung des Allylesters wurde das Harz unter einer Argonatmosphäre in einer Mischung von 10,9 ml DMSO, 10,9 ml THF, 1,85 ml Methylanilin und 5,45 ml 0.5N HCl suspendiert worauf 107 mg $Pd^0$ $[P(C_6H_5)_3]_4$ zugegeben wurden und das Gemisch 18 Stunden geschüttelt wurde [vgl. Williams, P.L., et al. Tetrahedron Lett. 1991, 32, 4207.]. Das Harz wurde mit DMF gewaschen, und die Fmoc- Schutzgruppe wurde mittels Piperidin (20% ig in DMF) entfernt. Zur Cyclisierung wurde das Harz in 10 ml DMF suspendiert und mit 160 mg TBTU und 0.085 ml DIPEA versetzt, worauf das Gemisch 3 Stunden geschüttelt und anschliessend mit DMF, Isopropanol und Diäthylether gewaschen wurde. Das Harz wurde hierauf in 40 ml einer Mischung von 82,5% Trifluoressigsäure 5% Phenol, 5% Wasser, 5% Thioanisol und 2,5% Aethandithiol suspendiert, worauf das Gemisch 2 Stunden geschüttelt und dann filtriert wurde. Das Filtrat wurde im Vakuum eingeengt, und der Rückstand wurde mit Diäthylether digeriert. Die Reinigung erfolgte analog wie im Beispiel 2.2.1. beschrieben, und es wurden 44 mg 4,5-Cyclo[-acetyl-L-isoleucyl-L-alanyl-L-arginyl-glycyl-L-aspartyl-L-phenylalanyl-L-prolyl-L-aspartylaminomethyl-]-3,6-dimethoxy-9,9-dimethylxanthen-trifluoracetat erhalten; MS: 1211,7 $(MH^+)$.

Beispiel 1.5.2.

Analog wie im Beispiel 1.5.1. beschrieben wurden die folgenden Verbindungen hergestellt:

a) 4,5-Cyclo[-acetyl-L-alanyl-L-arginyl-L-isoleucyl-L-alanyl-L-arginylglycyl-L-aspartyl-L-phenylalanyl-L-prolyl-L-aspartyl-L-aspartyl-L-arginylaminomethyl-]-3,6-dimethoxy-9,9-dimethylxanthen-trifluoracetat,FAB-MS: 1710;

b) 4,5-Cyclo[-acetyl-L-alanyl-L-arginyl-glycyl-L-aspartyl-L-phenylalanyl. L-prolylaminomethyl]-3,6-dimethoxy-9,9-dimethylxanthen-trifluoracetat, FAB-MS: 984;

c) 4,5-Cyclo[-acetyl-L-arginyl-glycyl-L-aspartyl-L-phenylalanylaminomethyl]-3,6-dimethoxy-9,9-dimethylxanthen-trifluoracetat,FAB-MS:816; und

d) 4,5-Cyclo[-acetyl-L-arginyl-L-isoleucyl-L-alanyl-L-arginyl-glycyl-L-aspartyl-L-phenylalanyl-L-prolyl-L-aspartyl-L-aspartylaminomethyl-]3.6-dimethoxy-9,9-dimethylxanthen-trifluoracetat,FAB-MS: 1482.

Beispiel 1.5.3.

0,6 g (0,26 mMol) Fmoc-Lys(Boc)-Sasrin$^R$ (Bachem AG, D-1365, vgl. EP 0 292 729 A2) wurden dem Syntheseszyklus gemäss Beispiel 2.2.2 unterworfen und nacheinander mit Fmoc-Arg(Pmc)-OH, Fmoc-Val-OH, 5-[(9-Fluorenylmethoxyformamido)methyl]-3,6-dimethoxy-9,9-dimethylxanthen-4-essigsäure und Fmoc-Ile-OH gekuppelt. Nach beendeter Synthese wurde das Peptidharz mehrmals mit Methylenchlorid gewaschen und anschliessend zwei bis vier mal mit 50 ml einer Lösung von 2% TFA in Methylenchlorid während 2 Minuten geschüttelt. Das Harz wurde abfiltriert, und die Filtrate wurden mit Pyridin neutralisiert und im Vakuum eingedampft. Der Rückstand wurde zwischen Essigsäureäthylester und 5% $KHSO_4$/10% $K_2SO_4$-Lösung verteilt, worauf die organische Phase gründlich mit dest. Wasser neutral gewaschen und im Vakuum eingeengt wurde. Der erhaltene Rückstand wurde zwei Mal in Toluol aufgenommen, und das Toluol wurde jeweils im Vakuum abgedampft. Zur Entfernung der Fmoc-Gruppe wurde der Rückstand in 10 ml einer 20%igen Lösung von Piperidin in DMF gelöst, worauf die Lösung 20 Min. bei Raumtemperatur stehen gelassen und anschliessend im Vakuum eingeengt wurde. Der Rückstand wurde mehrmals mit Hexan digeriert und anschliessend wie im Beispiel 2.2.2.c beschrieben cyclisiert, deblockiert und gereinigt. Man erhielt 26 mg 4,5-Cyclo[-acetyl-L-valyl-L-arginyl-L-lysyl-L-isoleucyl-aminomethyl-]3,6-dimethoxy-9,9-dimethylxanthen-trifluoracetat (1:2), MS (ISP) 836,6.

Beispiel 1.5.4.

Analog wie in Beispiel 1.5.3. beschrieben wurden die folgenden Verbindungen hergestellt.

a) 4,5-Cyclo[-acetyl-L-isoleucyl-L-valyl-L-arginyl-L-lysyl-L-lysyl-L-prolyl-aminomethyl-]-3,6-dimethoxy-9,9-dimethylxanthen-trifluoracetat (1:3), MS (ISP) 1061,4;

b) 4,5-Cyclo[-acetyl-glycyl-D-arginyl-L-lysyl-D-isoleucyl-aminomethyl-]-3,6-dimethoxy-9,9-dimethylxanthen-trifluoracetat (1:2), MS (ISP) 794,6;

c) 4,5-Cyclo[-acetyl-L-valyl-L-arginyl-L-lysyl-L-isoleucyl-aminomethyl-]-3,6-dimethoxy-9,9-dimethylxanthen-trifluoracetat (1:2), MS (ISP) 836,6; und

d) 4,5-Cyclo[-acetyl-L-arginyl-L-lysyl-L-isoleucyl-L-glutamyl-L-isoleucyl-L-valyl-L-arginyl-L-lysyl-L-lysyl-L-prolyl-L-isoleucyl-L-phenylalanyl-L-lysyl-L-lysyl-aminomethyl-]-3,6-dimethoxy-9,9-dimethylxanthen-trifluoracetat (1:7), MS (ISP) 2105,0.

Beispiel 2.1.1.a

Zu einer Lösung von 5,0 g (23,4 mMol) 10-Methylphenothiazin in 10 ml absolutem Diäthyläther und 7 ml frisch destilliertem N,N,N',N'-Tetramethyläthylendiamin wurden unter Argon bei -78° 21,7 mi tert-Butyllithium-Lösung (1,4M in Pentan) langsam zugetropft. Das Reaktionsgemisch wurde anschliessend langsam auf Raumtemperatur gebracht, 18 Stunden gerührt, dann auf 0° gekühlt und hierauf mit 3,90 ml (35,1 mMol) N-Formylpiperidin versetzt. Das Reaktionsgemisch wurde 2 Stunden bei Raumtemperatur gerührt und dann auf 50 g Eis, 50 mi 0,1N wässrige Salzsäurelösung und 1.50 ml Diäthyläther gegossen. Die wässrige Phase wurde zweimal mit 100 ml Diäthyläther extrahiert, und die vereinigten organischen Fraktionen wurden über Magnesiumsulfat getrocknet und eingeengt. Der Rückstand wurde an 500 g Kieselgel mit Essigsäureäthylester/Hexan (1:3) chromatographiert, wonach nach Umkristallisation aus Essigsäureäthylester/Hexan, 4,20 g (74,4%) 10-Methylphenothiazin-4-carbaldehyd als gelber Festoff vom Smp. 103° erhalten wurden.

Beispiel 2.1.1.b

Zu einer Lösung von 10,0 g (41,4 mMol) 10-Methyl-phenothiazin-4-carbaldehyd in 135 ml abs. Tetrahydrofuran wurden unter Argonatmosphäre und Eiskühlung 12,0 ml Lithiumborhydrid-Lösung (2M in Tetrahydrofuran) langsam zugetropft. Das Reaktionsgemisch wurde während 30 Minuten bei 0° gerührt und dann auf 100 ml 0,1N wässrige Salzsäurelösung und 200 ml Essigsäureäthylester gegossen. Die wässrige Phase wurde zweimal mit 100 ml Essigsäureäthylester extrahiert, und die vereinigten organischen Fraktionen wurden mit gesättigter Natriumchlorid-Lösung gewaschen, über Magnesiumsulfat getrocknet und eingeengt. Nach Kristallisation aus Essigsäureäthylester/Hexan und Trocknen am Hochvakuum wurden 9,49 g (94,2%) (10-Methyl-phenothiazin-4-yl)methanol als beiger Festkörper vom Smp. 113° erhalten.

Beispiel 2.1.1.ca

Zu einer eisgekühlten Lösung von 17,66 g (72,6 mMol) (10-Methylphenothiazin-4-yl)methanol und 10,87 g (159,7 mMol) Imidazol in 220 ml N,N-Dimethylformamid wurden 22,3 ml (87,1 mMol) tert-Butyldiphenylsilylchlorid langsam zugetropft. Das Reaktionsgemisch wurde während 30 Minuten bei 0° und 1 Stunde bei Raumtemperatur gerührt und dann auf 100 mi 0,5N wässrige Salzsäurelösung, 100g Eis und 200 ml Diäthyläther gegossen. Die wässrige Phase wurde mit Diäthyläther extrahiert, und die vereinigten organischen Fraktionen wurden über Magnesiumsulfat getrocknet und eingeengt. Der Rückstand wurde aus Diäthyläther/Hexan kristallisiert, worauf 27,69 g (79,2%) 4-(tert-Butyl-diphenyl-silanyloxymethyl)-10-methyl-penothiazin als leicht gelblicher Feststoff vom Smp. 127-128° erhalten wurden.

Beispiel 2.1.1.cb

Zu einer eisgekühlten Lösung von 10,0 g (41,1 mMol) (10-Methyl-phenothiazin-4-yl)methanol und 6,16 g (90,4 mMol) Imidazol in 125 ml N,N-Dimethylformamid wurden 8,05 g (53,4 mMol) tert-Butyldimethylsilylchlorid langsam zugetropft. Das Reaktionsgemisch wurde 30 Minuten bei 0° und 1 Stunde bei Raumtemperatur gerührt und dann auf 50g Eis, 50 ml 0,1N wässrige Salzsäurelösung und 250 ml Diäthyläther gegossen. Die organische Phase wurde abgtrennt, über Magnesiumsulfat getrocknet und eingedampft. Der Rückstand wurde an 1 kg Kieselgel mit Diäthyläther/Hexan (1:10) chromatographiert, worauf 13,0 g (88,5%) 4-(tert-Butyl-dimethylsilanyloxymethyl)-10-methyl-phenothiazin als farbloses Oel erhalten wurden. NMR (250 MHz, DMSO-$d_6$): 7,3-7,15 (m,3 arom. H); 7,1-7,0 (m, 1 arom H); 7,0-6,85 (m, 3 arom. H); 4,68 (s, $C\underline{H}_2O$); 3,31 (s, MeN); 0,70 (s, $OSi(CH_3)_2C(C\underline{H}_3)_3$);0,09(s, $OSi(C\underline{H}_3)_2C(CH_3)_3$).

Beispiel 2.1.1.da

Zu einer Lösung von 20,8 g (43,2 mMol) 4-(tert-Butyl-diphenyl-silanyloxymethyl)-10-methyl-penothiazin und 6,8 ml N,N,N',N'-Tetramethyläthylendiamin in 125 ml Diäthyläther wurden unter Argon und bei -78° 41,1 ml tert-Butyllithium-Lösung (1,4M in Pentan) langsam zugetropft. Das Reaktionsgemisch wurde 4 Stunden bei 0° und noch 2 Stunden bei Raumtemperatur gerührt und dann bei 0° mit 7,4 ml (66,5 mMol) N-Formylpiperidin versetzt. Der rohe 6-[(tert-Butyldiphenylsilanyoxy)methyl]-10-methyl-10H-phenothiazin-4-carbaldehyd wurde analog wie in Beispiel 2.1.1.db beschrieben reduziert. Nach Chromatographie an 1 kg Kieselgel mit Diäthyläther/Hexan (1:2) erhielt man nebst 4,0g (19%) 4-(tert-Butyl-diphenylsilanyloxymethyl)-10-methyl-phenothiazin 7,12 g (32,2%) [6-(tert-Butyldiphenyl-silanyloxymethyl)-10-methyl-phenothiazin-4-yl]-methanol als amorphen Festkörper. MS: 511 ($M^+$; 100), 439(52), 316(46), 238(36), 199(48), 139(58), 91(20).

Beispiel 2.1.1.db

Zu einer Lösung von 14,0 g (39,2 mMol) 4-(tert-Butyl-dimethylsilanyloxymethyl)-10-methyl-phenothiazin und 6,39 ml N,N,N',N'-Tetramethyläthylendiamin in 120 ml Diäthyläther wurden unter Argon bei -78° 36,4 ml tert-Butyllithium-Lösung (1,4M in Pentan) langsam zugetropft. Das Reaktionsgemisch wurde 30 Minuten bei -78° gerührt, langsam auf Raumtemperatur gebracht,3 Stunden gerührt, hierauf mit 5,66 ml (51,0 mMol) N-Formylpiperidin versetzt, 40 Minuten bei 0° gerührt und dann auf 50g Eis, 50 ml 0,1N wässrige Salzsäurelösung und 250 ml Diäthyläther gegossen. Die wässrige Phase wurde mehrmals mit Diäthyläther extrahiert. Die vereinigten organischen Phasen wurden über Magnesiumsulfat getrocknet und eingeengt. Der als Rückstand verbleibende rohe 6-(tert-Butyl-dimethylsilanyloxymethyl)-10-methyl-phenothiazin-4-carbaldehyd (ca. 14 g) wurde am Hochvakuum getrocknet, in 120 ml Tetrahydrofuran gelöst und bei 0° mit 50 ml Lithiumborhydrid-Lösung (1M in Tetrahydrofuran) versetzt. Das Reaktionsgemisch wurde 1 Stunde bei Raumtemperatur gerührt und dann auf 100 ml 0,1N wässrige Salzsäurelösung,100g Eis und 150 ml Diäthyläther gegossen. Die wässrige Phase wurde mit Diäthyläther extrahiert, und die vereinigten organischen Fraktionen wurden über Magnesiumsulfat getrocknet und eingeengt. Der Rückstand wurde an 1,3 kg Kieselgel mit Essigsäureäthylester/Hexan (1:4) chromatographiert, worauf nach Trocknen am Hochvakuum 4,57 g (30,0%) [6-(tert-Butyl-dimethyl-silanyloxymethyl)-10-methyl-phenothiazin-4-yl]-methanol als farbloses Oel erhalten wurden. MS: 387 ($M^+$; 100), 238(91), 224(24), 75(97).

Beispiel 2.1.1.ea

Zu einer Lösung von 7,1 g (14,18 mMol) [6-(tert-Butyl-diphenyl-silanyloxymethyl)-10-methyl-phenothiazin-4-yl]-methanol, 3,13 g (21,3 mMol) Phthalimid und 4,20 g (16,0 mMol) Triphenylphosphin in 110 ml Tetrahydrofuran wurden unter Argon und Eiskühlung 2,90 ml (18,43 mMol) Azodicarbonsäurediäthylester langsam zugetropft. Das Reaktionsgemisch wurde während 2,5 Stunden bei 0° gerührt und dann auf 100g Eis, 100 ml Wasser und 200 ml Essigsäureäthylester/Hexan (1:1) gegossen. Die organische Phase wurde abgetrennt, mit Wasser extrahiert, über Magnesiumsulfat getrocknet und eingeengt. Der Rückstand wurde an 1 kg Kieselgel mit Toluol/Essigsäureäthylester (20:1) chromatographiert, worauf nach Kristallisation aus Essigsäureäthylester/Hexan 6,87 g (77,3%) 2-[6-(tert-Butyl-diphenyl-silanyloxy)-methyl-10-methyl-phenothiazin-4-yl-methyl]-2,3-dihydro-1H-isoindol-1,3-dion als leicht gelblicher Feststoff vom Smp. 182-184° erhalten

wurden.

Beispiel 2.1.1.eb

Zu einer Lösung von 3,09 g (7,97 mMol) [6-(tert-Butyl-dimethyl-silanyloxymethyl)-10-methyl-phenothiazin-4-yl]methanol, 1,76 g (11,9 mMol) Phthalimid und 2,30 g (8,75 mMol) Triphenylphosphin in 65 ml Tetrahydrofuran wurden unter Eiskühlung und Argon 1,62 ml (10,34 mMol) Azodicarbonsäurediäthylester langsam zugetropft. Das Reaktionsgemisch wurde während 3 Stunden bei 0° gerührt und dann analog wie in Beispiel 2.1.1.ea beschrieben aufgearbeitet. Der Rückstand wurde an 500 g Kieselgel mit Toluol/Essigsäureäthylester (19:1) chromatographiert, worauf nach Kristallisation aus Aethanol/Essigsäureäthylester, 3,22 g (78,2%) 2-[6-(tert-Butyl-dimethyl-silanyloxymethyl)-10-methyl-phenothiazin-4-ylmethyl]-2,3-dihydro-1H-isoindol-1,3-dion als leicht gelber Festkörper vom Smp. 195-196° erhalten wurden.

Beispiel 2.1.1.fa

Eine Lösung von 6,53 g (10,38 mMol) 2-[6-(tert-Butyl-diphenyl-silanyloxymethyl)-10-methyl-phenothiazin-4-ylmethyl]-2,3-dihydro-1H-isoindol-1,3-dion in 50 ml Methylenchlorid wurde analog wie in Beispiel 2.1.1.fb beschrieben mit 11,5 ml Bortribromid-Lösung (1M in Methylenchlorid) und 5,1 g Natriumcyanid umgesetzt, worauf analog wie in Beispiel 2.1.1.fb beschrieben nach Umkristallisation aus Toluol/Acetonitril, 3,82 g (89,4%) [6-(1,3-Dioxo-2,3-dihydro-1H-isoindol-2-ylmethyl)-10-methyl-phenothiazin-4-yl]-acetonitril vom Smp. 245-247° erhalten wurden.

Beispiel 2.1.1.fb

Zu einer Lösung von 2,1 g (4,06 mMol) 2-[6-(tert-Butyl-dimethylsilanyloxymethyl)-10-methyl-phenothiazin-4-ylmethyl]-2,3-dihydro-1H-isoindol-1,3-dion in 15 ml Methylenchlorid wurden unter Eiskühlung 4,5 ml Bortribromid-Lösung (1M in Methylenchlorid) langsam zugetropft. Das Reaktionsgemisch wurde 10 Minuten bei 0° gerührt, auf Raumtemperatur gebracht, 1 Stunde bei 65° unter Argon gerührt, dann abgekühlt und auf Eis, 1M Natriumdihydrogenphosphat-Lösung und Methylenchlorid gegossen. Die organische Phase wurde abgetrennt, über Magnesiumsulfat getrocknet und eingeengt; der Rückstand wurde während 2 Stunden am Hochvakuum getrocknet und dann in 10 ml N,N-Dimethylformamid gelöst. Die Lösung wurde mit 2,0 g (46,0 mMol) Natriumcyanid versetzt, und das Reaktionsgemisch wurde während 1 Stunde bei 65° gerührt, abgekühlt und auf Eiswasser gegossen. Die erhaltene Suspension wurde 1 Stunde gerührt und filtriert; der Filterrückstand wurde mit Wasser gewaschen und am Hochvakuum über Phosphorpentoxid getrocknet. Nach Umkristallisation aus Toluol/Acetonitril erhielt man 2,32 g (93,9%) [6-(1,3-Dioxo-2,3-dihydro-1H-isoindol-2-ylmethyl)-10-methyl-phenothiazin-4-yl]-acetonitril als farblose Kristalle vom Smp. 245-247°.

Beispiel 2.1.1.ga

Zu einer Lösung von 823 mg (2,0 mMol) [6-(1,3-Dioxo-2,3-dihydro-1H-isoindol-2-ylmethyl)-10-methyl-phenothiazin-4-yl]-acetonitril in 10 ml Dioxan wurden 4,0 ml Hydrazinhydrat-Lösung (1M in Methanol) zugetropft. Das Reaktionsgemisch wurde 3 Stunden bei 80° gerührt, abgekühlt, mit 50 ml 10% wässriger Natriumcarbonat-Lösung und 50 ml Methylenchlorid vermischt und gründlich extrahiert. Die wässrige Phase wurde zweimal mit 30 ml Methylenchlorid extrahiert. Die vereinigten organischen Phasen wurden über Magnesiumsulfat getrocknet und eingedampft, und der Rückstand wurde am Hochvakuum getrocknet. Das erhaltene 6-Aminomethyl-10-methyl-phenothiazin-4-acetonitril wurde in 20 ml Dioxan gelöst. Die Lösung wurde mit 20 ml rauchender Salzsäure versetzt, 2 Stunden auf 100° erhitzt, abgekühlt, und bis zur Trockene eingedampft, und der Rückstand wurde am Hochvakuum getrocknet. Die erhaltene amorphe 6-Aminomethyl-10-methyl-phenothiazin-4-essigsäure wurde in 20 ml Dioxan/Wasser (2:1) aufgenommen. Die Lösung wurde mit 1N wässriger Natriumhydroxid-Lösung tropfenweise basisch gestellt, unter Eiskühlung mit einer Lösung von 546 mg (2,5 mMol) di-tert.Butyl-dicarbonat in 5 ml Dioxan versetzt und dann langsam auf Raumtemperatur gebracht. Das Reaktionsgemisch wurde 1 Stunde bei Raumtemperatur gerührt und dann auf Eiswasser und Methylenchlorid gegossen, worauf mit 1N wässriger Salzsäurelösung sauer gestellt und die wässrige Phase zweimal mit Methylenchlorid extrahiert wurde. Die vereinigten organischen Fraktionen wurden über Magnesiumsulfat getrocknet und eingeengt, und der Rückstand wurde an 100g Kieselgel mit Chloroform/Methanol (9:1) chromatographiert, worauf 660 mg (82,3%) (6-tert-Butoxycarbonylaminomethyl-10-methyl-phenothiazin-4-yl)-essigsäure als amorpher Festkörper erhalten wurden. IR(KBr): 3413w, 3060w, 2975w, 2929w, 1708s, 1678s, 1597s, 1563s, 1503w, 1457s, 1426s, 1394s, 1283m, 1249m, 1167s, 1051w, 757w.

Beispiel 2.1.1.gb 1

Zu einer Lösung von 4,90 g (20,3 mMol) 10-Methylphenothiazin-4-carbaldehyd und 50 mg p-Toluolsulfonsäure x $1H_2O$ in 15 ml Methanol wurden bei Raumtemperatur 15,0 ml Orthoameisensäuretriäthylester zugegeben. Das Reaktionsgemisch wurde während 45 Minuten am Rückfluss gekocht, abgekühlt und auf 30 g Eis, 30 ml gesättigte wässrige Natriumhydrogencarbonat-Lösung und 100 ml Diäthyläther gegossen. Die organische Phase wurde mit 50 ml gesättigter Natriumchlorid-Lösung gewaschen, über Magnesiumsulfat getrocknet und eingeengt. Der Rückstand wurde an 300 g Kieselgel mit Diäthyläther/Hexan (1:4) chromatographiert worauf, nach Kristallisation aus Diäthyläther/Hexan im Kühlschrank, 6,16 g (96.2%) 4-Diethoxymethyl-10-methyl-phenothiazin als weisser Festkörper vom Smp. 44,6-46,2° erhalten wurden.

Beispiel 2.1.1.gb 2

Zu einer Lösung von 2,5 g (7,93 mMol) 4-Diethoxymethyl-10-methylphenothiazin und 2,4 ml N,N,N',N'-Tetramethyläthylendiamin in 25 ml Diäthyläther wurden unter Argon und bei -78° 7,4 ml tert-Butyllithium-Lösung (1,4M in Pentan) langsam zugetropft. Das Reaktionsgemisch wurde langsam auf Raumtemperatur gebracht, 3,5 Stunden gerührt und dann mit 1,32 ml (11,9 mMol) N-Formylpiperidin versetzt. Das Reaktionsgemisch wurde 1 Stunde bei 0° gerührt und dann auf 30g Eis, 30 ml 0,1N wässrige Salzsäurelösung und 70 ml Essigsäureäthylester gegossen. Die wässrige Phase wurde mit Essigsäureäthylester extrahiert, und die vereinigten organischen Phasen wurden über Magnesiumsulfat getrocknet und eingedampft. Der Rückstand wurde an 200 g Kieselgel mit Essigsäureäthylester/Hexan (1:4) chromatographiert, worauf 310 mg (12,4%) Ausgangsprodukt und, nach Kristallisation aus Essigsäureäthylester/Hexan, 520 mg (19%) 6-Diethoxymethyl-10-methylphenothiazin-4-carbaldehyd als gelber Festkörper vom Smp. 154-154,5° erhalten wurden.

Beispiel 2.1.1.gb 3

Eine Lösung von 500 mg (1,46 mMol) 6-Diethoxymethyl-10-methylphenothiazin-4-carbaldehyd in 5 ml Tetrahydrofuran kann analog wie in Beispiel 1.1.1.d beschrieben mit 0,22 g (1,75 mMol) Methyl-(methylthiomethyl)sulfoxid, 0,14 ml Triton B-Lösung (35% in Methanol) und Hydrolyse in 6-Formyl-10-methyl-phenothiazin-4-essigsäuremethylester übergeführt werden. Diese Verbindung kann analog wie in Beispiel 1.1.1.e beschrieben in Methanol, Wasser und Tetrahydrofuran mit Natriumacetat und Hydroxylaminhydrochlorid zu 6-[(Hydroximino)methyl]-10-methylphenothiazin-4-essigsäuremethylester umgesetzt werden. Diese Verbindung kann analog wie in Beispiel 1.1.1.f beschrieben in Methanol und methanolischer Salzsäure (20%) über Palladiumkohle hydriert werden, wobei 6-Aminomethyl-10-methyl-phenothiazin-4-essigsäuremethylesterhydrochlorid erhalten wird, welcher analog wie in Beispiel 1.1.2. beschrieben in Dioxan und 1N Natronlauge mittels di-tert-Butyldicarbonat zu 6-[(1-tert-Butylformamido)methyl]-10-methyl-phenothiazin-4-essigsäuremethylester umgesetzt werden kann. Das erhaltene Produkt kann analog wie in Beispiel 1.1.3 beschrieben in Methanol mit 1N Kaliumhydroxidlösung zu der gemäss Beispiel 2.1.1.g hergestellten (6-tert-Butoxycarbonylaminomethyl-10-methyl-phenothiazin-4-yl)-essigsäure hydrolysiert werden.

Beispiel 2.1.2.

Zu einer Lösung von 80 mg (0,19 mMol) (6-tert-Butoxycarbonylaminomethyl-10-methyl-phenothiazin-4-yl)-essigsäure in 4 ml Methylenchlorid wurde unter Eiskühlung 1 ml Diazomethan-Lösung (~0,3N in Diäthyläther) zugetropft. Das Reaktionsgemisch wurde auf Raumtemperatur gebracht, 1,5 Stunden gerührt und eingedampft. Der Rückstand wurde aus Essigsäureäthylester/Hexan kristallisiert, worauf nach Trocknung am Hochvakuum, 72 mg (91%) (6-tert-Butoxycarbonylaminomethyl-10-methylphenothiazin-4-yl)-essigsäuremethylester als weisser Festkörper erhalten wurde. MS 414 ($M^+$, 64), 358 (100), 343 (15), 314 (15), 238 (15), 57 (16).

Beispiel 2.1.3.

Eine Lösung von 823 mg (2,0 mMol) [6-(1,3-Dioxo-2,3-dihydro-1H-isoindol-2-ylmethyl)-10-methyl-phenothiazin-4-yl]-acetonitril wurde analog wie in Beispiel 2.1.1.g beschrieben zuerst mit 4,0 ml Hydrazinhydrat-Lösung (1M in Methanol) und dann mit 20 ml rauchender Salzsäure behandelt. Der Rückstand wurde am Hochvakuum 3 Stunden getrocknet und in 20 ml Dioxan/Wasser (2:1) gelöst. Die Lösung wurde tropfenweise mit 10% wässriger Natriumcarbonat-Lösung basisch gestellt und unter Eiskühlung mit einer Lösung von 650 mg (2,51 mMol) Fluorenylmethylchlorcarbonat in 5 ml Dioxan versetzt. Das Reaktionsgemisch wurde langsam auf Raumtemperatur gebracht, 1 Stunde gerührt, mit 1N wässriger Salzsäurelösung sauer gestellt und auf Eiswasser gegossen. Das Gemisch wurde mit Methylenchlorid extrahiert. Die organische Phase wurde über Magnesiumsulfat getrocknet und eingeengt. Der Rückstand wurde an 80 g Kieselgel mit Chloroform/Methanol (18:1) chromatographiert, worauf nach Umkristallisation aus Aethanol, 784 mg (75%) [6-(Fluoren-9-ylmethoxycarbonylaminomethyl)-10-methyl-phenothiazin-4-yl]-essigsäure als beiger Festkörper vom Smp. 217-219° erhalten wurde.

Beispiel 2.1.4.

Zu einer Lösung von 801 mg (2,0 mMol) (6-tert-Butoxycarbonylaminomethyl-10-methyl-phenothiazin-4-yl)-essigsäure und 324,5 mg (3,0 mMol) Benzylalkohol in Methylenchlorid wurden unter Eiskühlung 20 mg N,N-Dimethylaminopyridin und eine Lösung von 455 mg (2,2 mMol) N,N-Dicyclohexylcarbodiimid in 5 ml Methylenchlorid zugegeben. Das Reaktionsgemisch wurde 15 Minuten bei 0° gerührt, langsam auf Raumtemperatur gebracht, 2 Stunden gerührt und dann auf Eis und gesättigte Natriumhydrogencarbonat-Lösung gegossen, worauf dreimal mit Methylenchlorid extrahiert wurde. Die vereinigten organischen Phasen wurden über Magnesiumsulfat getrocknet und eingedampft. Der Rückstand wurde an 100 g Kieselgel mit Essigsäureäthylester/Hexan (3:2) chromatographiert, worauf 730 mg (74,4%) (6-tert.-Butoxycarbonylaminomethyl-10-methylphenothiazin-4-yl)-essigsäurebenzylester als amorpher Feststoff erhalten wurden. MS: 490 ($M^+$ 78), 434(100), 416(22), 390(20), 91(60).

Beispiel 2.1.5.

Zu einer Lösung von 730 mg (1,48 mMol) (6-tert.Butoxycarbonylaminomethyl-10-methyl-phenothiazin-4-yl)-essigsäurebenzylester in 3 ml Methylenchlorid wurden unter Eiskühlung 3 ml Trifluoressigsäure langsam zugetropft. Das Reaktionsgemisch wurde 2 Stunden bei 0° gerührt, worauf das Lösungsmittel am Vakuum entfernt wurde und der Rückstand am Hochvakuum getrocknet wurde. Der Rückstand wurde mit 10 ml Diäthyläther versetzt. Die Suspension wurde 1 Stunde gerührt und filtriert. Der weisse Rückstand wurde mit Diäthyläther gewaschen und am Hochvakuum getrocknet, worauf 720 mg (96,4%) [6-Aminomethyl-10-methylphenothiazin-4-yl]-essigsäurebenzylester-Trifluoracetat (1:1) als weisser Feststoff vom Smp. 192° erhalten wurden.

Beispiel 2.1.6.a

Zu einer Lösung von 11,80 g (41,6 mMol) 10-Hexyl-phenothiazin in 125 ml abs. Diäthyläther und 12,5 ml N,N,N,N-Tetramethyläthylendiamin wurden unter Argon und Rühren bei -78° 38,6 ml tert-Butyllithium-Lösung (1,4M in Hexan) langsam zugetropft. Das Reaktionsgemisch wurde während 16 Stunden bei -75° gerührt, langsam auf 0° gebracht, mit 6.92 ml (1.5 Aeq.) N-Formylpiperidin versetzt, 1 Stunde bei 0° gerührt und dann auf Eis, 100 ml 0,1N Salzsäurelösung und 250 ml Essigsäureäthylester gegossen. Die organische Phase wurde mit gesättigter Natriumchlorid-Lösung gewaschen, über Magnesiumsulfat getrocknet und eingeengt. Der Rückstand wurde an 1 kg Kieselgel mit Essigsäureäthylester/Hexan (1:1) chromatographiert, worauf 6,33 g (48,8%) 10-Hexyl-phenothiazin-4-carbaldehyd als leicht gelbliches Oel erhalten wurde. IR(Film): 3064w, 2927m, 2854m, 2731w, 1689s, 1561m, 1455s, 1388w, 1336m, 1286m, 1259s, 1052w, 784m, 750m, 724m.

Beispiel 2.1.6.b

Zu einer Lösung von 6,30 g (20,2 mMol) 10-Hexyl-phenothiazin-4-carbaldehyd in 60 ml Tetrahydrofuran wurden unter Argonatmosphäre und Eiskühlung 6 ml Lithiumborhydrid-Lösung (2M in Tetrahydrofuran) langsam zugetropft. Das Reaktionsgemisch wurde 30 Minuten bei 0° gerührt und auf 100g Eis, 100 ml 0,1N Salzsäurelösung und 200 ml Essigsäureäthylester gegossen. Die organische Phase wurde mit

gesättigter Kochsalzlösung gewaschen und eingeengt; der Rückstand wurde aus Essigsäureäthylester/Hexan im Kühlschrank kristallisiert. Nach Filtration und Trocknen am Hochvakuum wurden 5,75 g (90,8%) (10-Hexylphenothiazin-4-yl)-methanol vom Smp. 73,5-74,5° erhalten.

Beispiel 2.1.6.c

Zu einer eisgekühlten Lösung von 3,20 g (10,21 mMol) (10-Hexyl-phenothiazin-4-yl)-methanol, 1,85 ml (13,27 mMol) Triäthylamin und 62 mg Dimethylaminopyridin in 35 ml Methylenchlorid wurden 3,4 ml (13,27 mMol) tert-Butyldiphenylsilylchlorid zugetropft. Das Reaktionsgemisch wurde 30 Minuten bei 0° und 2 Stunden bei Raumtemperatur gerührt und dann auf 10 ml 2N wässrige Natriumdihydrogenphosphat-Lösung, 20g Eis und 30 ml Diäthyläther gegossen. Die wässrige Phase wurde mit Diäthyläther extrahiert, und die vereinigten organischen Phasen wurden über Magnesiumsulfat getrocknet und eingedampft. Der Rückstand wurde an 400 g Kieselgel mit Diäthyläther/Hexan (1:20) chromatographiert, worauf 5,40 g (95,8%) 4-(tert-Butyl-diphenyl-silanyloxymethyl)-10-hexyl-phenothiazin als farbloses Oel erhalten wurden. MS : 551 ($M^+$, 72), 494(100), 296(65), 211(62), 43(38).

Beispiel 2.1.6.d

Zu einer Lösung von 4,40 g (7,97 mMol) 4-(tert-Butyl-diphenyl-silanyloxymethyl)-10-hexyl-phenothiazin und 1,3 ml N,N,N',N'-Tetramethyläthylendiamin in 25 ml Diäthyläther wurden unter Argon und bei -78° 7,4 ml tert-Butyllithium-Lösung (1,4M in Pentan) langsam zugetropft. Das Reaktionsgemisch wurde auf 0° gebracht, 4 Stunden gerührt, dann mit 1,33 ml (11,96 mMol) N-Formylpiperidin versetzt und anschliessend 40 Minuten bei 0° gerührt. Das Reaktionsgemisch wurde analog wie in Beispiel 2.1.1.db beschrieben aufgearbeitet, und der 6-(tert-Butyldiphenylsilanyloxy)methyl]-10-hexyl-phenothiazin-4-carbaldehyd wurde analog wie in Beispiel 2.1.1 db beschrieben reduziert. Der Rückstand wurde an 400 g Kieselgel mit Diäthyläther/Hexan (1:3) chromatographiert, worauf 1,95 (42%) [6-(tert-Butyl-diphenyl-silanyloxymethyl)-10-hexyl-phenothiazin-4-yl]-methanol als leicht gelblicher amorpher Schaum erhalten wurden. MS: 581 ($M^+$, 100), 439(51), 386(30), 301(35), 199(40), 139(29), 43(36).

Beispiel 2.1.6.e

Zu einer Lösung von 1,95 g (3,35 mMol) [6-(tert-Butyl-diphenyl-silanyloxymethyl)-10-hexyl-phenothiazin-4-yl]-methanol, 876 mg (5,03 mMol) Phthalimid und 968 mg (3,69 mMol) Triphenylphosphin in 25 ml Tetrahydrofuran wurden unter Argon und Eiskühlung 0,68 ml (4,36 mMol) Azodicarbonsäurediäthylester langsam zugetropft. Das Reaktionsgemisch wurde während 2 Stunden bei 0° und 20 Minuten bei Raumtemperatur gerührt und dann analog wie in Beispiel 2.1.1 ea beschrieben aufgearbeitet. Der Rückstand wurde an 250 g Kieselgel mit Diäthyläther/Hexan (1:3) chromatographiert, worauf nach Fällung aus Hexan 1,74 g (73,1%) 2-[6-(tert-Butyl-diphenyl-silanyloxymethyl)-10-hexyl-phenothiazin-4-ylmethyl]-2,3-dihydro-1H-isoindol-1,3-dion als leicht gelblicher amorpher Festkörper erhalten wurden. MS: 710 ($M^+$, 100), 653(61), 506(16), 370(36), 308(41), 268(40), 224(21), 160(34), 130(45), 43(43).

Beispiel 2.1.6.f

Eine Lösung von 1,5 g (1,41 mMol) 2-[6-(tert-Butyl-diphenyl-silanyloxymethyl)-10-hexyl-phenothiazin-4-ylmethyl]-2,3-dihydro-1H-isoindol-1,3-dion in 5 ml Methylenchlorid wurde analog wie in Beispiel 2.1.1 fb beschrieben mit 1,5 ml Bortribromid-Lösung (1M in Methylenchlorid) und mit 691 mg (14,1 mMol) Natriumcyanid umgesetzt. Das Reaktionsgemisch wurde auf Eiswasser und Essigsäureäthylester gegossen. Die organische Phase wurde mit gesättigter Natriumchlorid-Lösung gewaschen und eingeengt. Der Rückstand wurde an 120 g Kieselgel mit Diäthyläther/Hexan (1:3) chromatographiert, worauf 510 mg (75%) [6-(1,3-Dioxo-2,3-dihydro-1H-isoindol-2-ylmethyl)-10-hexyl-phenothiazin-4-yl]-acetonitril als leicht gelblicher amorpher Schaum erhalten wurden. MS: 481 ($M^+$, 100), 410(50), 396(70), 263(15), 140(15).

Beispiel 2.1.6.g

Analog wie in Beispiel 2.1.1.ga beschrieben wurde eine Lösung von 500 mg (1,04 mMol) [6-(1,3-Dioxo-2,3-dihydro-1H-isoindol-2-ylmethyl)-10-hexyl-phenothiazin-4-yl]-acetonitril in 5 ml Dioxan mit 2 ml Hydrazinhydrat-Lösung (1M in Aethanol) behandelt und das erhaltene 6-Aminomethyl-10-hexyl-phenothiazin-4-yl)-acetonitril mit einer Lösung von rauchender Salzsäure in Dioxan in 6-Aminomethyl-10-hexyl-phenothiazin-4-

essigsäure übergeführt, welche dann analog wie in Beispiel 2.1.1.ga beschrieben mit 273 mg (1,25 mMol) di-tert-Butyldicarbonat in Dioxan umgesetzt wurde. Nach Chromatographie an Kieselgel mit Chlroform/Methanol (9:1) wurden 395 mg (85%) 6-[(1-tert-Butoxyformamido)methyl]-10-hexyl-phenothiazin-4-essigsäure als amorpher Festkörper erhalten. MS (FAB): 447 ($M^+ + 1$), 446 ($M^+$).

Beispiel 2.2.1.

Eine Lösung von 75 mg [6-Aminomethyl-10-methyl-phenotbiazin-4-yl]-essigsäurebenzylester-Trifluoracetat (1:1) in 3 ml N,N-Dimethylformamid (DMF) wurde mit 135 mg $N^\alpha$,$N^G$,$N^E$ Tris-(benzyloxycarbonyl)-L-arginin-N-hydroxysucinimidester versetzt. Der pH-Wert wurde mit N-Methylmorpholin auf 8,5 eingestellt, worauf das Reaktionsgemisch 1 Stunde bei 20° gerührt und dann in eine verdünnte $NaHCO_3$-Lösung gegossen wurde. Das ausgefallene Produkt wurde abfiltriert, mit 5% $KHSO_4$/10% $K_2SO_4$-Lösung und dest. Wasser nachgewaschen und dann mit Aethanol digeriert. Der kristalline Festkörper wurde in Trifluoräthanol gelöst und in Gegenwart von 10% Pd-C hydriert. Der Katalysator wurde abfiltriert, und das Filtrat wurde eingedampft. Der Rückstand wurde in 3 ml DMF gelöst und mit 20,3 mg 1-Hydroxybenzotriazol $H_2O$ und 0,05 ml Diisopropylaethylamin versetzt. Diese Lösung wurde unter Rühren innerhalb von 10 Minuten zu einer Lösung von 89 mg O-(1,2-Dihydro-2-oxo-1-pyridyl)-N,N,N',N',-tetramethyl- uronium-tetrafluoroborat in 20 ml DMF zugetropft. Das Reaktionsgemisch wurde weitere 30 Minuten gerührt und im Vakuum eingeengt. Das als Rückstand verbleibende Rohprodukt wurde mittels HPLC an einer Zorbase ODS $C_{18}$(8 $\mu$m) Säule im Gradientensystem 0,1% Trifluoressigsäure-Aethanol gereinigt. Es wurden 18,2 mg lyophilisiertes (S)-8-(3-Guanidinopropyl)-17-methyl-1,15-imino-6,7,8,9,10,11-hexahydro-5H-dibenzo[b,k][1,5,8]-thiadiazacyclodecin-7,10-dion-Trifluoracetat (1:1) erhalten. MS: 439 ($M^+$, 10), 327(25), 237(60).

Beispiel 2.2.2.

a) In einem Peptidsynthesizer [Labortec SP G40] wurden 4 g 4-(2',4'-Dimethoxyphenyl-hydroxymethyl)-phenoxy-polystyrol-Harz (Novabiochem, 01-64-0012) eingefüllt. Das Harz wurde in 40 ml DMF suspendiert und nacheinander mit 3,3 g $(Fmoc\text{-}Val)_2O$, 61 mg 4-Dimethylaminopyridin und 0,85 ml Diisopropylaethylamin versetzt und 3 Stunden bei 20° geschüttelt. Das veresterte Harz wurde dann folgendem Synthesezyklus unterworfen.

| Stufe | Reagenz | Zeit |
|---|---|---|
| 1 | DMF | 2 x 1 Min. |
| 2 | 20% Piperidin/DMF | 1 x 7 Min. |
| 3 | DMF | 5 x 1 Min. |
| 4 | 2,5 Aeq. Fmoc- bzw. Z-Aminosäure /DMF + 2,5 Aeq. 1-Benzotriazol-1-yl-tetramethyluronium-hexafluorphosphat + 2,5 Aeq. Diisopropylaethylamin | 1 x 90 Min. |
| 5 | DMF | 3 x 1 Min. |
| 6 | Isopropylalkohol | 2 x 1 Min. |

In jeder Stufe wurden 30 ml Lösungsmittel benutzt. Fmoc-Asp(OBut)-OH, Fmoc-Gly-OH und Z-Arg-(Pmc)-OH wurden nach obigem Protokoll gekuppelt. Nach beendeter Synthese wurde das Peptidharz in 60 ml Essigsäure/Methylenchlorid (1:2) suspendiert und 20 Minuten geschüttelt, worauf das Harz abfiltriert und das Filtrat eingeengt wurde. Der Rückstand wurde zwischen Essigsaureäthylester und Wasser verteilt. Die organische Phase wurde mit gesättigter Natriumchlorid-Lösung gewaschen und über Natriumsulfat getrocknet. Das erhaltene Z-Arg(Pmc)-Gly-Asp(OBut)-Val-OHwurde aus Essigsäureäthylester/Hexan gefällt; Ausbeute: 1,05 g, MS: 902 $MH^+$.

b) Zu einer Lösung von 128 mg 6-Aminomethyl-10-methyl-phenothiazin-4-yl]-essigsäurebenzylester-Trifluoracetat (1:1) und 270 mg Z-Arg(Pmc)-Gly-Asp(OBut)-Val-OH in 5 ml DMF wurden 133 mg 1-Benzotriazol-1-yl-N,N,N',N'-tetramethyluroniumhexafluorophosphat und 0,17 ml Diisopropyläthylamin zugegeben, worauf das Reaktionsgemisch 1 Stunde bei 20° gerührt und dann im Vakuum eingedampft wurde. Der Rückstand wurde zwischen Essigsäureäthylester und gesättigter $NaHCO_3$-Lösung verteilt. Die organische Phase wurde mit gesättigter NaCl-Lösung gewaschen, über $Na_2SO_4$ getrocknet und eingedampft. Der Rückstand wurde mit Diäthyläther digeriert, und man erhielt 260 mg 6-[((($N^\alpha$-Benzyloxycarbonyl-$N^G$-(2,5,5,7,8)-pentamethyl-chroman-6-sulfonyl)-L-arginyl-glycyl-4-O-tert.-butyl-L-aspartyl-L-valyl)aminomethyl)-3,7-dimethoxy-10-methylphenothiazin-4-yl]-essigsäurebenzylester, MS: 1274,5 $MH^+$

c) 255 mg des erhaltenen Produkts wurden in 25 ml Trifluoräthanol gelöst und in Gegenwart von 10% Pd-C hydriert. Der Katalysator wurde abfiltriert, und das Filtrat wurde eingedampft. Der Rückstand wurde in 20 ml DMF gelöst und mit 0,2 ml Diisopropylaethylamin versetzt. Die erhaltene Lösung wurde unter Rühren während 20 Minuten zu einer Lösung von 379,2 mg 1-Benzotriazol-1-yl-N,N,N',N'-tetramethyluroniumhexafluorophosphatin 50 ml DMF zugetropft. Das Gemisch wurde 1 Stunde bei 20° gerührt, anschliessend im Vakuum eingeengt und dann in verd. Natriumhydrogencarbonatlösung gegossen, wobei ein Festkörper ausfiel, welcher abfiltriert, mit dest. Wasser auf dem Filter nachgewaschen und getrocknet wurde. Das erhaltene amorphe Pulver wurde in 20 ml Trifluoressigsäure/0,2 ml Phenol/1 ml Wasser gelöst. Die Mischung wurde 2 Stunden bei 20° stehen gelassen und dann im Vakuum eingeengt. Der Rückstand wurde mit Diäthyläther digeriert und aus Essigsäure lyophilisiert. Das Lyophilisat wurde wie in Beispiel 2.2.1. beschrieben gereinigt, und es wurden 15,8 mg 10-Methyl-4,6-cyclo[-acetyl-L-arginyl-glycyl-L-aspartyl-L-valyl-aminomethyl-]phenothiazin-Trifluoracetat (1:1) erhalten; MS 710 $MH^+$.

<u>Beispiel 3.1.1.a</u>

Zu einer Suspension von 20,0 g (85,0 mMol) Resorufin, 17,6 g frisch pulverisiertem Kaliumcarbonat und 10 Tropfen tris-[2-(2-Methoxyäthoxy)äthyl]amin in 150 ml Dioxan wurden bei Raumtemperatur unter gutem Rühren 12,1 ml Dimethylsulfat zugegeben. Das Reaktionsgemisch wurde anschliessend während 2 Stunden bei 100° gerührt, abgekühlt und dann mit 150 ml Wasser versetzt. Der Festkörper wurde abfiltriert, mit Wasser gewaschen und über Phosphorpentoxid getrocknet. Man erhielt 11,5 g (59,5%) Resorufin-methyläther vom Smp. 240-244° (Zers.).

Zu einer Suspension von 10,0 g (44,0 mMol) Resorufin-methyläther in einem Gemisch von 60 ml Wasser und 250 ml Aceton wurden unter Argon bei Raumtemperatur portionenweise 21,2 g Natriumdithionit zugegeben. Das Reaktionsgemisch wurde danach während 3 Stunden unter Argon am Rückfluss gekocht, abgekühlt und mit 100 ml 5% Natriumdithionit-Lösung und 300 ml Essigsäureäthylester versetzt. Die organische Phase wurde abgetrennt, über Magnesiumsulfat getrocknet und eingedampft. Der braungrüne Feststoff wurde am Vakuum getrocknet, in 150 ml Dioxan gelöst und unter Argon mit 18,4 g Kaliumcarbonat (pulverisiert), 15 Tropfen tris-[2-(2-Methoxyäthoxy)äthyl]amin und 12,6 ml Dimethylsulfat versetzt. Das Reaktionsgemisch wurde unter Argon während 2 Stunden auf 100° erhitzt, dann mit 6,1 g Kaliumcarbonat und 4,2 ml Dimethylsulfat versetzt, während 18 Stunden unter Argon bei 100° gerührt, abgekühlt und auf 100 g Eis, 150 ml gesättigte Natriumbicarbonat-Lösung und 300 ml Essigsäureäthylester gegossen. Die Wasser-Phase wurde mit 2 x 150 ml Essigsäureäthylester extrahiert. Die vereinigten organischen Phasen wurden über Magnesiumsulfat getrocknet und eingedampft, und der Rückstand wurde an 500 g Kieselgel mit Hexan/Diäthyläther (4:1) chromatographiert. Nach Umkristallisation aus Essigsäureäthylester/Hexan (1:2) erhielt man 6,25 g (55,2%) 3,7-Dimethoxy-10-methyl-10H-phenoxazin als weisse Nadeln. Smp. 125-127°.

<u>Beispiel 3.1.1.b</u>

Zu einer Suspension von 4,0 g (15,55 mMol) 3,7-Dimethoxy-10-methyl-10H-phenoxazin in 15 ml Tetrahydrofuran und 60 ml Diäthyläther wurden bei -78° 12,63 ml n-Butyllithium-Lösung (1,6M in Hexan) langsam zugetropft. Das Reaktionsgemisch wurde langsam auf 0° gebracht, wobei sich bei -20° für kurze Zeit eine klare Lösung bildete. Bei -10° bildete sich ein Niederschlag, und die Suspension wurde während 2 Stunden bei 0° gerührt, gefolgt durch Zugabe von 2,6 ml (1,5 Aeq.) N-Formylpiperidin und 1-stündigem Rühren bei 0°. Das Reaktionsgemisch wurde auf Eis/0,5N Salzsäurelösung und Diäthyläther gegossen, die wässrige Phase wurde zweimal mit Essigsäureäthylester extrahiert, und die vereinigten organischen Phasen wurden mit gesättigter Natriumchlorid-Lösung gewaschen, über Magnesiumsulfat getrocknet und eingedampft. Der feste orange Rückstand wurde in 60 ml Tetrahydrofuran gelöst, worauf bei 0° langsam 10 ml einer 2M Lithiumborhydrid-Lösung (in Tetrahydrofuran) zugegeben wurde. Das Gemisch wurde 1 Stunde bei 0° gerührt und dann mit 50 ml gesättigter AmmoniumchloridLösung, Eis und Diäthyläther versetzt. Die wässrige Phase wurde zweimal mit Diäthyläther extrahiert. Die vereinigten organischen Phasen wurden über Magnesiumsulfat getrocknet und eingeengt. Der Rückstand wurde an 180 g $SiO_2$ mit einem Gemisch von Hexan/Essigsäureäthylester (2:3) chromatographiert. Nach Trocknung am Hochvakuum und nach Umkristallisation aus Hexan/Essigsäureäthylester erhielt man 3,71 g (83,3%) 3,7-Dimethoxy-10-methyl-10H-dibenzo[b,e][1,4]oxazin-4-methanol vom Smp. 65-66°.

Beispiel 3.1.1.c

Zu einer Lösung von 3,3 g (11,49 mMol) 3,7-Dimethoxy-10-methyl-10H-dibenzo[b,e][1,4]oxazin-4-methanol in 33 ml absolutem Tetrahydrofuran wurden bei -78° 8,6 ml n-Butyllithium-Lösung (1,6M in Hexan) zugetropft, wobei sich ein weisser Niederschlag bildete. Das Reaktionsgemisch wurde auf 0° gebracht, 30 Minuten gerührt, bei 0° mit 1,96 ml (1,5 Aeq.) 2-(Methoxyäthoxy)methylchlorid versetzt, 1 Stunde bei Raumtemperatur gerührt und dann auf Eis, gesättigte Ammoniumchlorid-Lösung und Diäthyläther gegossen. Die organische Phase wurde über Magnesiumsulfat getrocknet und eingedampft. Der Rückstand wurde an Kieselgel mit einem Gemisch von Essigsäureäthylester/Hexan (1:2) chromatographiert, worauf nach Trocknung am Hochvakuum, 4,13 g (95,7%) 3,7-Dimethoxy-4-[[(2-methoxyäthoxy)methoxy]-methyl]-10-methyl-10H-dibenzo[b,e][1,4]oxazin als farbloses Oel erhalten wurden. MS: 375 ($M^+$, 100), 360-(11), 270(14), 256(21), 226(14).

Beispiel 3.1.1.d

Zu einer Lösung von 4,10 g (10,92 mMol) 3,7-Dimethoxy-4-[[(2-methoxyäthoxy)methoxy]methyl]-10-methyl-10H-dibenzo[b,e][1,4]oxazin in 10 ml Tetrahydrofuran und 40 ml Diäthyläther wurden unter Argon bei -78° 8,9 ml n-Butyllithium-Lösung zugegeben. Das Reaktionsgemisch wurde langsam auf 0° gebracht und 1,5 Stunden gerührt, worauf die Suspension mit 1,82 ml (1,5 Aeq.) N-Formylpiperidin versetzt, 30 Minuten bei 0° nachgerührt und auf 100g Eis, 100 ml 0,1N Salzsäurelösung und 150 ml Diäthyläther gegossen wurde. Die Wasserphase wurde dreimal mit Chloroform extrahiert, und die vereinigten organischen Phasen wurden über $MgSO_4$ getrocknet und eingedampft. Der rötliche Rückstand wurde in 50 ml Tetrahydrofuran gelöst und bei 0° unter Argon mit 5 ml Lithiumborhydrid-Lösung (2M in Tetrahydrofuran) versetzt. Das Reaktionsgemisch wurde 30 Minuten bei 0° gerührt, und dann auf 50g Eis, 50 ml gesättigte Ammoniumchlorid-Lösung und Diäthyläther gegossen. Die Wasserphase wurde zweimal mit Essigsäureäthylester extrahiert, und die vereinigten organischen Phasen wurden über $MgSO_4$ getrocknet und eingedampft. Der Rückstand wurde an 200 g Kieselgel mit Essigsäureäthylester/Hexan (2:1) chromatographiert, worauf nach Trocknung am Hochvakuum, 3,90 g (88,1%) 3,7-Dimethoxy-6-[[(2-methoxyäthoxy)methoxy]-methyl]-10-methyl-10H-dibenzo[b,e][1,4]oxazin-4-methanol als farbloser Festkörper vom Smp. 68-70° erhalten wurden.

Beispiel 3.1.1.e

Zu einer Lösung von 3,0 g (7,40 mMol) 3,7-Dimethoxy-6-[[(2-methoxyäthoxy)methoxy]methyl]-10-methyl-10H-dibenzo[b,e][1,4]oxazin-4-methanol in 40 ml Tetrahydrofuran wurden unter Eiskühlung portionenweise 1,63 g (1,5 Aeq.) Phthalimid und 2,91 g (1,5 Aeq.) Triphenylphosphin zugegeben. Dann wurde bei 0° eine Lösung von 2,06 g (1,6 Aeq.) Diäthyldiazodicarboxylat in 10 ml Tetrahydrofuran während 2 Stunden langsam zugetropft. Das Reaktionsgemisch wurde 10 Stunden bei 0° gerührt und dann auf 50 ml Wasser, 100 ml Hexan, 50 ml Methanol und 50 ml Essigsäureäthylester gegossen. Die organische Phase wurde abgetrennt, mit gesättigter Natriumchlorid-Lösung gewaschen, über Magnesiumsulfat getrocknet und eingeengt. Der Rückstand wurde an 200 g Kieselgel mit Essigsäureäthylester/Hexan (1:1) chromatographiert, worauf 3,61 g (93,7%) N-[[3,7-Dimethoxy-6-[[(2-methoxyäthoxy)methoxy]methyl]-10-methyl-10H-dibenzo[b,e][1,4]oxazin-1-yl]methyl]-2,3-dihydro-1H-isoindol-1,3-dion als amorpher Schaum erhalten wurden. MS: 534 ($M^+$, 100), 160(8).

Beispiel 3.1.1.f

Zu einer eisgekühlten Lösung von 5,73 g (10,72 mMol) N-[[3,7-Dimethoxy-6-[[(2-methoxyäthoxy]methoxy]-methyl]-10-methyl-10H-dibenzo[b,e][1,4]oxazin-1-yl]methyl]-2,3-dihydro-1H-isoindol-1,3-dion in 60 ml Methylenchlorid wurden unter Argon langsam 9,0 ml 33-%ige Bromwasserstoffsäure in Essigsäure zugetropft. Nach 45-minütigem Rühren bei Raumtemperatur wurden nochmals 2,5 ml 33-%ige Bromwasserstoffsäure in Eisessig zugetropft, worauf das Reaktionsgemisch 2 Stunden bei Raumtemperatur gerührt und dann auf 100 g Eis, 100 ml gesättigte Natriumhydrogencarbonat-Lösungund 50 ml Methylenchlorid gegossen wurde. Die wässrige Phase wurde zweimal mit Methylenchlorid extrahiert. Die vereinigten organischen Phasen wurden über Magnesiumsulfat getrocknet und eingeengt. Der Rückstand wurde am Hochvakuum getrocknet und anschliessend in 60 ml N,N-Dimethylformamid gelöst und mit 5,26 g pulverisiertem Natriumcyanid versetzt. Das Reaktionsgemisch wurde während 45 Minuten bei 60° gerührt, dann abgekühlt und auf Eiswasser gegossen. Die Suspension wurde nach 2 Stunden filtriert, und der Filterrückstand wurde mit

Wasser gewaschen und über Phosphorpentoxid am Hochvakuum getrocknet, worauf 4,66 g (95,3%)N-[3,7-Dimethoxy-6-(1,3-dioxo-2,3-dihydro-1H-isoindol-2-ylmethyl)-10-methyl-10H-dibenz[b,e][1,4]-oxazin-4-yl]-acetonitril als leicht gelblicher Festkörper vom Smp. 215-217° erhalten wurden.

Beispiel 3.1.1.ga

Eine Suspension von 270 mg (0,57 mMol) [3,7-Dimethoxy-6-(1,3-dioxo-2,3-dihydro-1H-isoindol-2-ylmethyl)-10-methyl-10H-dibenz[b,e][1,4]-oxazin-4-yl]-acetonitril in 2 ml Dioxan und 2 ml konzentrierter Salzsäure wurde im Bombenrohr während 1,5 Stunden auf 100° erhitzt, dann abgekühlt und auf Eiswasser gegossen. Der ausgefallene Niederschlag wurde abfiltriert, mit Wasser mehrmals gewaschen und über Phosphorpentoxid am Hochvakuum getrocknet. Nach Umkristallisation aus Aceton/Methanol (1:1) erhielt man 240 mg (88,7%) [3,7-Dimethoxy-6-(1,3-dioxo-2,3-dihydro-1H-isoindol-2-ylmethyl)-10-methyl-10H-dibenz[b,e][1,4]-oxazin-4-yl]-essigsäure als beigen Festkörper vom Smp. >220° (Zers.).

Beispiel 3.1.1.gb

Zu einer Suspension von 280 mg [3,7-Dimethoxy-6-(1,3-dioxo-2,3-dihydro-1H-isoindol-2-ylmethyl)-10-methyl-10H-dibenz[b,e][1,4]oxazin-4-yl]acetonitril wurden bei Raumtemperatur 2,0 ml einer äthanolischen Hydrazinhydrat-Lösung (1M) zugegeben. Das Reaktionsgemisch wurde während 4 Stunden auf 85° erwärmt, dann abgekühlt und auf Methylenchlorid und 10%ige Natriumcarbonat-Lösung gegossen. Die wässrige Phase wurde mit Methylenchlorid extrahiert, und die vereinigten organischen Fraktionen wurden über Magnesiumchlorid getrocknet und eingeengt. Der Rückstand wurde am Hochvakuum getrocknet, und nach Umkristallisation aus tert-Butylmethyläther/Methanol wurden 180 mg (90,1%) 6-Aminomethyl-3,7-dimethoxy-10-methyl-10H-dibenzo[b,e][1,4]-oxazin-4-acetonitril als beiger Festkörper vom Smp. 167-169° (Zers.) erhalten.

Beispiel 3.1.1.ha

Eine Lösung von 200 mg (0,42 mMol) [3,7-Dimethoxy-6-(1,3-dioxo-2,3-dihydro-1H-isoindol-2-ylmethyl)-10-methyl-10H-dibenz[b,e][1,4]oxazin-4-yl]-essigsäure in 1 ml Methanol wurde bei Raumtemperatur mit 1 ml Hydrazinhydratlösung (1M in Aethanol) versetzt, und das Reaktionsgemisch wurde 4 Stunden bei 80° gerührt, dann abgekühlt und zur Trockene eingedampft. Der Rückstand wurde in 2 ml Dioxan suspendiert, und die Suspension wurde mit 1N Natronlauge neutralisiert, mit 140 mg (1,5 Aeq.) di-tert-Butyldicarbonat versetzt und bei Raumtemperatur 3 Stunden gerührt. Das Reaktionsgemisch wurde auf Eis, 1N HCl-Lösung und Essigsäureäthylester gegossen, worauf die organische Phase abgetrennt, über $MgSO_4$ getrocknet und eingedampft wurde. Der Rückstand wurde an Kieselgel mit Chloroform/Methanol (9:1) chromatographiert, worauf 121 mg (65%) 6-[(1-tert-Butoxformamido)methyl]-3,7-dimethoxy-10-methyl-10H-phenoxazin-4-essigsäure als gelblicher amorpher Festkörper erhalten wurden. MS: 445 ($M^+$).

Beispiel 3.1.1.hb

Eine Lösung von 150 mg (0,46 mMol) 6-Aminomethyl-3,7-dimethoxy-10-methyl-10H-dibenzo[b,e][1,4]-oxazin-4-acetonitril in 2 ml Methanol wurde mit 2 ml 6N Natronlauge versetzt und 2 Stunden bei 110° im Bombenrohr erhitzt. Das Reaktionsgemisch wurde zur Trockene eingedampft, und der Rückstand wurde mit 2N Salzsäure neutralisiert. Nach erneutem Eindampfen wurde der Rückstand in 2 ml Dioxan gelöst, worauf der pH mit 1N Natronlauge auf 8 eingestellt wurde und 151 mg (0,69 mMol) di-tert-Butyldicarbonat zugegeben wurden. Das Reaktionsgemisch wurde 2 Stunden bei Raumtemperatur gerührt und dann auf Eis/2N HCl-Lösung und Essigsäureäthylester gegossen. Die organische Phase wurde abgetrennt, über $MgSO_4$ getrocknet und eingedampft. Der Rückstand wurde an Kieselgel mit Chloroform/Methanol (9:1) chromatographiert, worauf 160 mg (78,3%) 6-[(1-tert-Butoxyformamido)methyl]-3,7-dimethoxy-10-methyl-10H-phenoxazin-4-essigsäure als gelblicher amorpher Feststoff erhalten wurden; MS: 445 ($M^+$).

Beispiel 3.1.2.a

Eine Suspension von 5,0 g (21,3 mMol) Resorufin, 10 Tropfen tris-[2-(Methoxyäthoxy)äthyl]amin und 3,5 ml (1,5 Aeq.) Diäthylsulfat in 70 ml Dioxan wurde bei Raumtemperatur vermischt, während 18 Stunden bei 100° gerührt und dann auf ein Gemisch von 50g Eis und 50 ml 2N Salzsäurelösung gegossen. Der Niederschlag wurde abfiltriert, und der braun-orange Filterrückstand wurde mit Wasser gewaschen. Nach

Trocknen des Rückstandes im Exikkator über Phosphorpentoxid am Vakuum erhielt man 4,15 g (80,8%) Resorufinäthyläther.

Eine Lösung von 3,0 g (12,44 mMol) dieses Produkts in 130 ml Aceton und 16 ml Wasser wurde mittels eines Stickstoff-Stroms während 1 Stunde entgast, dann mit 6,0 g Natriumdithionit versetzt, während 2 Stunden unter Stickstoff auf 70° erhitzt, abgekühlt und in ein Gemisch von 2%iger Natriumdithionit-Lösung und Essigsäureäthylester gegossen. Die organische Phase wurde abgetrennt, über Magnesiumsulfat getrocknet, filtriert und eingeengt. Der Rückstand wurde am Vakuum getrocknet und unter Argon bei Raumtemperatur mit 7,95 g (4.4Aeq.) Kaliumcarbonat, 6,30 ml Diäthylsulfat, 20 Tropfen tris-[2-(2-Methoxyäthoxy)äthyl]amin und 45 ml Dioxan vermischt. Das Gemisch wurde während 48 Stunden bei 100° gerührt, abgekühlt und auf Wasser/Diäthyläther gegossen. Die Wasserphase wurde mit 2 x 100 ml Diäthyläther extrahiert. Die vereinigten organischen Phasen wurden mit gesättigter Natriumchlorid-Lösung gewaschen, über Magnesiumsulfat getrocknet und eingeengt. Der Rückstand wurde an 300 g Kieselgel mit einem Gemisch von Diäthyläther/Hexan (1:12) chromatographiert, worauf nach Umkristallisation 2,82 g (82,3%) 3,7-Diäthoxy-10-äthyl-10H-phenoxazin vom Smp. 69,0-69,5° erhalten wurden.

Beispiel 3.1.2.b

Zu einer Suspension von 1,35 g (4,51 mMol) 3,7-Diäthoxy-10-äthyl-10H-phenoxazin in 4 ml wasserfreiem Tetrahydrofuran und 16 ml Diäthyläther wurden bei -78° unter Argon langsam 4,2 ml n-Butyllithium-Lösung (1,6M in Hexan) zugetropft. Das Reaktionsgemisch wurde während 15 Minuten bei -78° gerührt, langsam auf 0° gebracht und noch während 4 Stunden bei 0° gerührt. Danach wurden 1,0 ml N-Formylpiperidin bei 0° zugetropft, worauf das Reaktionsgemisch auf Eis/0,5N Salzsäurelösung und Diäthyläther gegossen wurde. Die wässrige Phase wurde zweimal mit Diäthyläther extrahiert. Die vereinigten organischen Phasen wurden über Magnesiumsulfat getrocknet und eingeengt. Der Rückstand wurde am Hochvakuum getrocknet und in 20 ml absolutem Tetrahydrofuran gelöst, worauf bei 0° 2,3 ml Lithiumborhydrid-Lösung (2M in Tetrahydrofuran) zugegeben wurden. Das Reaktionsgemisch wurde 30 Minuten bei 0° gerührt und dann auf Eis, gesättigte Ammoniumchlorid-Lösung und Diäthyläther gegossen. Die wässrige Phase wurde zweimal mit Diäthyläther extrahiert. Die vereinigten organischen Phasen wurden über Magnesiumsulfat getrocknet und eingeengt. Der Rückstand wurde an 100 g $SiO_2$ mit Hexan/Diäthyläther (3:2) chromatographiert. Man erhielt, nach Trocknung am Hochvakuum, 970 mg (65,3%) 3,7-Diäthoxy-10-äthyl-10H-dibenzo[b,e][1,4]oxazin-4-methanol als weissen Feststoff vom Smp. 71,5-72,5.

Beispiel 3.1.2.ca

Zu einer Lösung von 1,0 g (3,04 mMol) 3,7-Diäthoxy-10-äthyl-10H-phenoxazin in 10 ml absolutem Tetrahydrofuran wurden bei -78° 2,1 ml n-Butyllithium-Lösung (1,6M in Hexan) zugetropft. Das Reaktionsgemisch wurde 30 Minuten bei -78° gerührt, langsam auf 0° gebracht, mit 0,45 ml 2-(Methoxyäthoxy)-methylchlorid versetzt, 2 Stunden bei Raumtemperatur gerührt und dann auf Eis, gesättigte Natriumbicarbonat-Lösung und Diäthyläther gegossen. Die wässrige Phase wurde zweimal mit Diäthyläther extrahiert, und die vereinigten organischen Phasen wurden über Magnesiumsulfat getrocknet und eingedampft. Der Rückstand wurde an 100 g Kieselgel mit einem Gemisch von Diäthyläther/Hexan (1:1) chromatographiert, worauf nach Trocknung am Hochvakuum 1,13 g (89%) 10-Aethyl-3,7-diäthoxy-4-[[(2-methoxyäthoxy)-methoxy]methyl]-10H-dibenzo[b,e][1,4]oxazin als farbloses Oel erhalten wurden. MS: 417 ($M^+$; 100), 388-(58), 313(20), 284(44), 254(60), 226(30).

Beispiel 3.1.2.cb

Zu einem Gemisch von 3,23 g (9,80 mMol) 3,7-Diäthoxy-10-äthyl-10H-dibenzo[b,e][1,4]oxazin-4-methanol und 1,47 g (2,2 Aeq.) Imidazol in 30 ml N,N-Dimethylformamid wurde unter Eiskühlung eine Lösung von 1,92 g (1,3 Aeq.) tert-Butyldimethylsilylchlorid in 10 ml N,N-Dimethylformamid zugetropft. Das Reaktionsgemisch wurde 30 Minuten bei 0° gerührt, langsam auf Raumtemperatur gebracht, 1 Stunde gerührt und dann auf 100 ml Wasser, 50 ml Diäthyläther und 50 ml Hexan gegossen. Die organische Phase wurde abgetrennt, zweimal mit Wasser extrahiert, über Magnesiumsulfat getrocknet und eingeengt. Der Rückstand wurde an 250 g Kieselgel mit Diäthyläther/Hexan (1:10) chromatographiert, worauf nach Trocknung am Hochvakuum 4,30 g (98,9%) 10-Methyl-3,7-diäthoxyl-4-[(tert-butyldimethylsilyl)oxy]methyl]-10H-dibenzo-[b,e][1,4]oxazin als farbloser Feststoff vom Smp. 77,5-78,5° erhalten wurden.

Beispiel 3.1.2.da

Eine Lösung von 1,13 g (2,70 mMol) 10-Aethyl-3,7-diäthoxy-4-[[(2-methoxyäthoxy)methoxy]methyl]-10H-dibenzo[b,e][1,4]oxazin in 2 ml Tetrahydrofuran und 8 ml Diäthyläther wurde in Analogie zu Beispiel 3.1.1.d mit 2,2 ml n-Butyllithium-Lösung (1,6M in Hexan) ud 0,54 ml N-Formylpiperidin umgesetzt. Die Reduktion erfolgte ebenfalls in Analogie zu Beispiel 3.1.1.d mit 1,5 ml Lithiumborhydrid-Lösung (2M in Tetrahydrofuran). Nach Chromatographie des Rohprodukts an 80 g Kieselgel mit Diäthyläther/Hexan (3:1), und Trocknung am Hochvakuum wurden 880 mg (72,8%) 10-Aethyl-3,7-diäthoxy-6-[[(2-methoxyäthoxy)-methoxy]methyl]-10H-dibenzo-[b,e][1,4]oxazin-4-methanol als farbloser Festkörper vom Smp. 60-62° erhalten.

Beispiel 3.1.2.db

Zu einer Lösung von 4,0 g (9,02 mMol) 10-Aethyl-3,7-diäthoxy-4-[(tert-butyldimethylsilyl)oxy]methyl]-10H-dibenzo[b,e][1,4]oxazin in 40 ml Diäthyläther und 10 ml Tetrahydrofuran wurden unter Argon bei -78° 7,33 ml n-Butyllithium-Lösung (1,6M in Hexan) zugetropft. Das Reaktionsgemisch wurde auf 0° gebracht, 3 Stunden gerührt; mit 1,85 ml N-Formylpiperidin versetzt, 30 Minuten bei 0° gerührt und dann auf 50 g Eis, 50 ml gesättigte Natriumdihydrogenphosphat-Lösung und 100 ml Diäthyläther gegossen. Die Wasserphase wurde zweimal mit Diäthyläther extrahiert, und die vereinigten organischen Phasen wurden über Magnesiumsulfat getrocknet und eingedampft. Der Rückstand wurde am Hochvakuum getrocknet, in 50 ml Tetrahydrofuran gelöst und bei 0° mit 5 ml Lithiumborhydrid-Lösung (2M in Tetrahydrofuran) versetzt. Das Gemisch wurde 30 Minuten bei 0° gerührt und dann auf 50 g Eis, 50 ml gesättigte Natriumdihydrogenphosphatlösung und 100 ml Essigsäureäthylester gegossen. Die organische Phase wurde über Magnesiumsulfat getrocknet und eingedampft. Der Rückstand wurde an 250 g Kieselgel mit Diäthyläther/Hexan (1:2) chromatographiert, worauf 3,90 g (91%) 10-Aethyl-3,7-diäthoxy-6-[[(tert-butyldimethylsilyl)oxy]methyl]-10H-dibenzo[b,e][1,4]oxazin-4-methanol als farbloses Oel erhalten wurden. MS: 473 ($M^+$; 100), 444(32), 387-(24), 238(24).

Beispiel 3.1.2.ea

Eine Lösung von 700 mg (1,56 mMol) 10-Aethyl-3,7-diäthoxy-6-[[(2-methoxyäthoxy)methoxy]methyl]-10H-dibenzo[b,e][1,4]oxazin-4-methanol in Tetrahydrofuran kann analog wie in Beispiel 3.1.1.e mit Triphenylphosphin, Diäthyldiazodicarboxylat und Phthalimid in N-[[3,7-Diäthoxy-6-[[(2-methoxyäthoxy)methoxy]-methyl]-10-hexyl-10H-dibenzo[b,e][1,4]oxazin-1-yl]methyl]-2,3-dihydro-1H-isoindol-1,3-dion übergeführt werden. Durch Behandlung mit Bromwasserstoffsäure in Essigsäure und dann mit Natriumcyanid in Dimethylformamid kann hieraus analog wie in Beispiel 3.1.1.f beschrieben 3,7-Diäthoxy-10-äthyl-6-(1,3-dioxo-2,3-dihydro-1H-isoindol-2-yl)methyl)-dibenzo[b,e][1,4]oxazin-4-acetonitril erhalten werden; Smp. >205° (Zers.).

Beispiel 3.1.2.eb1

Zu einer Lösung von 3,73 g (7,87 mMol) 10-Aethyl-3,7-diäthoxy-6[[(tert-butyldimethylsilyl)oxy]methyl]-10H-dibenzo[b,e][1,4]oxazin-4-methanol, 2,27 g (1,5 Aeq.) Triphenylphosphin und 1,74 g (1,5 Aeq.) Phthalimid in 50 ml absolutem Tetrahydrofuran wurde bei 0° während 2 Stunden langsam eine Lösung von 1,64 g (1,2 Aeq.) Azodicarbonsäurediäthylester in 10 ml Tetrahydrofuran zugetropft. Das Reaktionsgemisch wurde 4 Stunden bei Raumtemperatur gerührt und dann auf 100 g Eis, 100 ml Hexan, 50 ml Diäthyläther und 50 ml Methanol gegossen. Die organische Phase wurde zweimal mit Wasser extrahiert, über Magnesiumsulfat getrocknet und eingedampft. Der Rückstand wurde an 300 g Kieselgel mit Diäthyläther/Hexan (2:3) chromatographiert, worauf nach Kristallisation aus Essigsäureäthylester/Hexan, 3,59 g (75,7%) N-[[10-Aethyl-3,7-diäthoxy-6-[[(tert-butyldimethylsilyl)oxy]methyl]-10H-dibenzo[b,e][1,4]oxazin-1-yl]methyl]-2,3-dihydro-1H-isoindol-1,3-dion vom Smp. 158-159° erhalten wurden.

Beispiel 3.1.2.eb2

Zu einer Lösung von 2,45 g (4,06 mMol) N-[[10-Aethyl-3,7-diäthoxy-6-[[(tert-butyldimethylsilyl)oxy]-methyl]-10H-dibenzo[b,e][1,4]oxazin-1-yl]-methyl]-2,3-dihydro-1H-isoindol-1,3-dion in 20 ml Methylenchlorid wurden unter Eiskühlung langsam 7,0 ml 33%ige Bromwasserstoff-Lösung in Eisessig zugetropft. Das Reaktionsgemisch wurde langsam auf Raumtemperatur gebracht, 1,5 Stunden gerührt und dann auf 50 g Eis, 40 ml gesättigte Natriumhydrogencarbonat-Lösung und 20 ml Methylenchlorid gegossen. Die wässrige

Phase wurde zweimal mit Methylenchlorid extrahiert. Die vereinigten organischen Phasen wurden über Magnesiumsulfat getrocknet und eingedampft. Der Rückstand wurde am Hochvakuum getrocknet, in 15 ml N,N-Dimethylformamid gelöst und mit 2,64 g pulverisiertem Natriumcyanid versetzt. Das Reaktionsgemisch wurde während 1 Stunde bei 60° gerührt, dann abgekühlt und auf Eiswasser gegossen. Die entstandene Suspension wurde 1 Stunde bei Raumtemperatur gerührt und dann filtriert. Der Filterrückstand wurde mit Wasser gewaschen und am Hochvakuum über Phosphorpentoxid getrocknet. Nach Umkristallisation aus Aceton/Aethanol (1:1) wurden 1,91 g (94,9%) [3,7-Diaethoxy-10-aethyl-6-(1,3-dioxo-2,3-dihydro-1H-isoindol-2-ylmethyl)dibenz[b,e][1,4]oxazin-4-yl]-essigsäure vom Smp. >205° (Zers.) erhalten.

Beispiel 3.1.2.f

Eine Lösung von 1,0 g (2,72 mMol) [3,7-Diaethoxy-10-aethyl-6-(1,3-dioxo-2,3-dihydro-1H-isoindol-2-ylmethyl)-dibenz[b,e][1,4]oxazin-4-yl]-essigsäure in 40 ml Dioxan und 40 ml konzentrierter Salzsäure wurde 2 Stunden im Bombenrohr auf 100° erhitzt, dann abgekühlt und zur Trockene eingedampft. Der Rückstand wurde am Hochvakuum über Nacht getrocknet und dann in 30 ml Dioxan/Wasser (2:1) gelöst. Die Lösung wurde auf 0° gekühlt und tropfenweise mit 1N Natriumhydroxid-Lösung versetzt, bis der pH ~8 erreicht hat. Das Gemisch wurde portionenweise mit 742 mg (3,4 mMol) di-tert-Butyldicarbonat versetzt und 1 Stunde bei Raumtemperatur gerührt. Das Reaktionsgemisch wurde auf Eiswasser und Methylenchlorid gegossen und mit 1M Salzsäure-Lösung leicht sauer gestellt. Die organische Phase wurde abgetrennt, über Magnesiumsulfat getrocknet und eingedampft. Der Rückstand wurde an 150 g Kieselgel mit Chloroform/Methanol (9:1) chromatographiert, worauf nach Umkristallisation aus Chloroform/Acetonitril, 1,16 g (87,6%) 3,7-Diäthoxy-10-aethyl-6-[((1-tert-butoxyformamido)methyl)-10H-dibenz[b,e][1,4]oxazin-4-yl]-essigsäure als beiger Festkörper vom Smp. >230° (Zers.) erhalten wurden.

Beispiel 3.1.3.

Zu einer eisgekühlten Lösung von 370 mg (0,76 mMol) 3,7-Diäthoxy-10-aethyl-6-[((1-tert-butoxyformamido)methyl)-10H-dibenz[b,e][1,4]-oxazin-4-yl]-essigsäure, 100 mg Benzylalkohol und 20 mg N,N-Dimethylaminopyridin in 2 ml Methylenchlorid wurde eine Lösung von 173 mg (1,1 Aeq.) N,N-Dicyclohexylcarbodiimid in 1 ml Methylenchlorid langsam zugetropft. Das Reaktionsgemisch wurde während 1 Stunde bei 0° gerührt, dann langsam auf Raumtemperatur gebracht und noch 3 Stunden gerührt. Der ausgefallene Festkörper wurde abfiltriert und mit Methylenchlorid gewaschen. Die organischen Filtrate wurden mit 5 ml gesättigter Natriumhydrogencarbonat-Lösung und 5 ml 1N wässriger Salzsäurelösung gewaschen, über Magnesiumsulfat getrocknet und eingeengt. Der Rückstand wurde an Kieselgel mit Essigsäureäthylester/Hexan (1:1) chromatographiert, worauf nach Umkristallisation aus Essigsäureäthylester/Hexan, 230 mg (52,5%) [3,7-Diaethoxy-10-aethyl-6-[((1-tert-butoxyformamido)methyl)-10H-dibenz-[b,e][1,4]oxazin-4-yl]-essigsäurebenzylester als leicht gelblichen Festkörper vom Smp. 124-126° erhalten wurden.

Beispiel 3.2.1.

a) Zu einer Lösung von 13,65 g Z-Asp(OBut)-OH•$H_2O$ und 15,9 g Val-OBzl•TosOH in 250 ml DMF wurden 15,83 g 1-Benztriazol-1-yl-N,N,N',N',-tetramethyl-uronium-hexafluorphophat und 9 ml N-Methylmorpholin zugegeben. Nach 2-stündigem Rühren wurde das Reaktionsgemisch im Vakuum eingedampft, und der Rückstand wurde zwischen Essigsäureäthylester und Wasser verteilt. Die organische Phase wurde mit 5% $KHSO_4$/10% $K_2SO_4$-Lösung, Wasser, gesättigter $NaHCO_3$-Lösung, Wasser und gesättigter NaCl-Lösung gewaschen, über $Na_2SO_4$ getrocknet, filtriert und eingedampft. Der Rückstand wurde aus Diäthyläther/Hexan kristallisiert, und man erhielt 17,5 g Z-Asp(OBut)-Val-OBzl vom Smp. 99°; $[\alpha]_{D}^{;20}$-27,7° (c = 1, MeOH).

b) Eine Lösung von 10,25 g Z-Asp(OBut)-Val-OBzl in 150 ml Methanol wurde in Gegenwart von 10% Pd-C hydriert. Der Katalysator wurde abfiltriert, und das Filtrat wurde eingedampft. Der Rückstand wurde in 30 ml DMF aufgenommen, worauf 6,12 g Z-Gly-OSu zugegeben wurden und das pH mit N-Methylmorpholin auf 8,5 eingestellt wurde. Das Reaktionsgemisch wurde 18 Stunden bei 20° gerührt und analog wie in Absatz a) beschrieben aufgearbeitet. Das erhaltene Oel wurde in Methanol/Wasser gelöst und über 10% Pd-C hydriert, worauf der Katalysator abfiltriert wurde. Das Filtrat wurde eingedampft, und der Rückstand wurde aus Methanol/Dimethyläther kristallisiert, wobei 5,52 g Gly-Asp(OBut)-Val erhalten wurden; MS 346 $MH^+$.

c) Das pH einer Suspension von 3 g Gly-Asp(OBut)-Val und 5,86 g $Z_3$-Arg-OSu in 50 ml DMF wurde mit N-Methylmorpholin auf 8,5 eingestellt. Das Gemisch wurde 20 Stunden bei 20° gerührt und dann in

verd. $KHSO_4/K_2SO_4$-Lösung gegossen. Das ausgefallene Produkt wurde abfiltriert und aus Essigsäureäthylester kristallisiert, wobei 5,3 g $Z_3$-Arg-Gly-Asp(OBut)-Val-OH erhalten wurden; MS: 904 $MH^+$.

d) Eine Lösung von 130 mg [3,7-Diaethoxy-10-aethyl-6-[((1-tertbutoxyformamido)methyl)-10H-dibenz-[b,e][1,4]oxazin-4-yl]essigsäurebenzylester in 3 ml Trifluoressigsäure wurde 15 Minuten bei 20° stehen gelassen und dann im Vakuum eingeengt. Der Rückstand wurde in 3 ml DMF gelöst, worauf das pH mit Diisopropylaethylamin auf 8,5 eingestellt und bei 0° 207,9 mg $Z_3$-Arg-Gly-Asp(OBut)-Val-OH, 34 mg 1-Hydroxybenzotriazol •$H_2O$ und 74,3 mg O-(1,2-Dihydro-2-oxo-1-pyridyl)-N,N,N',N',-tetramethyl-uronium-tetrafluoroborat zugegeben wurden. Das pH wurde mit Diisopropylaethylamin auf 8,5 eingestellt, worauf das Reaktionsgemisch 2 Stunden bei 20° gerührt und anschliessend in verd. $NaHCO_3$-Lösung eingetropft wurde. Der ausgefallene Festkörper wurde abgenutscht und aus Aethanol kristallisiert, wobei 207 mg [6-(($N^{\alpha}N^{G}N^{E}$-Tribenzyloxycarbonyl)-L-arginyl-glycyl-4-O-tert-butyl-L-aspartyl-L-valyl)aminomethyl)-3,7-diaethoxy-10-aethyl-10H-dibenz[b,e][1,4]oxazin-4-yl]essigsäurebenzylester erhalten wurden; MS: 1362,5 $MH^+$.

e) 190,5 mg des erhaltenen Produkts wurden in 25 ml Trifluoräthanol gelöst und in Gegenwart von 10% Pd-C hydriert. Der Katalysator wurde abfiltriert, und das Filtrat wurde im Vakuum eingeengt. Der Rückstand wurde in 20 ml DMF gelöst und mit 18,9 mg 1-Hydroxybenzotriazol •$H_2O$ versetzt. Die erhaltene Lösung wurde unter Rühren während 20 Minuten zu einer Lösung Von 208 mg O-(1,2-Dihydro-2-oxo-1-pyridyl)-N,N,N',N',-tetramethyl- uronium-tetrafluoroborat und 0,12 ml Diisopropylaethylamin in 50 ml DMF zugetropft, worauf das Reaktionsgemisch 1 Stunde bei 20° gerührt und dann in verd. $NaHCO_3$-Lösung eingetropft wurde. Der ausgefallene Festkörper wurde abfiltriert, mit Wasser gewaschen und in 10 ml Trifluoressigsäure gelöst.Nach 1 Stunde wurde die Lösung im Vakuum eingedampft, und der Rückstand wurde aus Essigsäure lyophilisiert Das Lyophilisat wurde mittels HPLC wie in Beispiel 2.2.1. beschrieben gereinigt und als Lyophilisat isoliert, wobei 99 mg 4,6-Cyclo[-acetyl-L-arginyl-glycyl-L-aspartyl-L-valylaminomethyl-]-3,7-diaethoxy-10-aethyl-10H-dibenz[b,e][1,4]oxazin-trifluoracetat erhalten wurden; MS: 796 $MH^+$.

Beispiel 4.1.1.a

Ein Gemisch von 40.0g (0.174 Mol) 4,4'-Dimethoxydiphenylamin und 11.0g (0.341 Mol) Schwefel wurde fein pulverisiert und in einem Kolben mit mechanischem Rührer mit 0.20g (0.78 mMol) Jod versetzt. Das feste Gemisch wurde unter Rühren auf 185° erhitzt, und die enstandene Schmelze wurde 1 Stunde gerührt, nach beendeter Gasentwicklung 5 Minuten auf 195° erhitzt und dann abgekühlt. Die erstarrte Schmelze wurde auf dem Dampfbad in 600 ml Acetonitril aufgenommen. Dann wurde heiss filtriert, worauf das Filtrat bis zur beendeten Kristallisation bei 0° stehen gelassen wurde. Der Niederschlag wurde abfiltriert und getrocknet, wobei 35.2g (78%) 3,7-Dimethoxy-phenothiazin als leicht bräunliche Kristalle vom Smp. 193-194° erhalten wurden.

Beispiel 4.1.1.b

Eine Suspension von 21.75 g (83.9 mMol) 3,7-Dimethoxy-phenothiazin, 43.36 g (0.34 Mol) getrocknetem, pulverisiertem Kaliumcarbonat, 25.22 g (0.20 Mol) Dimethylsulfat und 1.35 g (4.19 mMol) Tris-[2-(2-Methoxyäthoxy)äthyl]amin in 300 ml Toluol wurde 1 Stunde bei 110° gerührt, dann abgekühlt und auf 1 l Eiswasser gegossen. Das Gemisch wurde mit 2N Salzsäurelösung leicht angesäuert und mit Essigsäureäthylester extrahiert. Die vereinigten organischen Phasen wurden mit gesättigter Natriumchloridlösung extrahiert, über Magnesiumsulfat getrocknet und eingedampft. Der Rückstand wurde aus Aethanol/Aceton kristallisiert, worauf nach Trocknung 18.4 g (80.2%) 3,7-Dimethoxy-10-methylphenothiazin als weisser Festkörper vom Smp. 166-167° erhalten wurden.

Beispiel 4.1.1.c

Zu einer Lösung von 5.46 g (10.0 mMol) 3,7-Dimethoxy-10-methylphenothiazin in 100 ml Diäthyläther/Tetrahydrofuran (4:1) wurde unter Argon bei -78° 16.25 ml n-Butyllithium-Lösung (1.6M in Hexan) zugetropft. Das Reaktionsgemisch wurde 15 Minuten bei -70° gerührt, langsam auf 0° gebracht, 2 Stunden gerührt und dann bei 0° mit 3.39 g (30.0 mMol) N-Formylpiperidin versetzt. Nach 2-stündigem Rühren bei 0° wurde das Gemisch auf 500 ml Eiswasser gegossen, worauf mit 0.5N Salzsäurelösung leicht angesäuert und mit Essigsäureäthylester extrahiert wurde. Die vereinigten organischen Phasen wurden mit gesättigter Natriumchlorid-Lösung extrahiert, über Magnesiumsulfat getrocknet und eingedampft. Der Rückstand wurde aus Aceton kristallisiert, worauf nach Trocknung, 5.42 g (90%) 3,7-Dimethoxy-10-methyl-phenothiazin-4-carbaldehyd als roter Feststoff vom Smp. 150° erhalten wurden.

Beispiel 4.1.1.da

Zu einer Lösung von 5.42 g (18.0 mMol) 3,7-Dimethoxy-10-methylphenothiazin-4-carbaldehyd in 90 ml Tetrahydrofuran wurden unter Argon und Eiskühlung langsam 20.0 ml Lithiumborhydrid-Lösung (1M in Tetrahydrofuran) zugetropft. Das Reaktionsgemisch wurde 15 Minuten bei 0° gerührt, langsam auf Raumtemperatur gebracht, 1 Stunde gerührt und dann auf Eiswasser gegossen. Das Gemisch wurde mit 0.5N HCl auf pH 4 angesäuert und dreimal mit Essigsäureäthylester extrahiert. Die vereinigten organischen Phasen wurden mit gesättigter Kochsalzlösung gewaschen, über Magnesiumsulfat getrocknet und eingedampft. Der Rückstand wurde am Hochvakuum getrocknet.

Das erhaltene rohe 3,7-Dimethoxy-10-methylphenothiazin-4-methanol wurde in 100 ml N,N-Dimethylformamid gelöst, und die Lösung wurde unter Eiskühlung mit 2.45 g (36.0 mMol) Imidazol und einer Lösung von 3.26 g (21.6 mMol) tert.-Butyldimethylchlorsilan in 10 ml N,N-Dimethylformamid versetzt. Das Reaktionsgemisch wurde auf Raumtemperatur gebracht, 4 Stunden gerührt und auf Eiswasser gegossen, worauf dreimal mit Essigsäureäthylester extrahiert wurde. Die vereinigten organischen Phasen wurden mit Wasser und gesättigter Kochsalzlösung extrahiert, über Magnesiumsulfat getrocknet und eingedampft. Der Rückstand wurde am Hochvakuum getrocknet. Nach Umkristallisation aus Hexan erhielt man 5.0 g (66.2%) 4-(tert.-Butyl-dimethyl-silanyloxymethyl)-3,7-dimethoxy-10-methyl-phenothiazin als rötliche Kristalle vom Smp. 110-111°.

Beispiel 4.1.1.db

Eine Lösung von 1.90 g (6.32 mMol) 3,7-Dimethoxy-10-methyl-phenothiazin-4-carbaldehyd in 30 ml Tetrahydrofuran wurde analog wie in Beispiel 4.1.1.da beschrieben mit 7.6 ml Lithiumborhydrid-Lösung (1M in Tetrahydrofuran) reduziert. Das erhaltene 3,7-Dimethoxy- 10-methylphenothiazin-4-methanol wurde in 20 ml Tetrahydrofuran gelöst, worauf bei -78° mit 4.36 ml n-Butyllithium-Lösung (1.6M in Hexan) zugegeben wurden. Das Reaktionsgemisch wurde langsam auf 0° gebracht, dann mit 1.02 g (8.19 mMol) 2-Methoxyäthoxymethylchlorid versetzt, 2 Stunden bei Raumtemperatur gerührt und schliesslich auf Eiswasser/gesättigte Natriumhydrogencarbonat-Lösunggegossen. Nach dreimaliger Extraktion mit Diäthyläther wurden die vereinigten organischen Phasen mit Kochsalzlösung gewaschen, über Magnesiumsulfat getrocknet und eingedampft. Der Rückstand wurde an Kieselgel mit Diäthyläther/Hexan (3:2) chromatographiert, worauf 2.09 g (84.6%) 3,7-Dimethoxy-4-(2-methoxy-äthoxymethoxymethyl)-10-methyl-phenothiazin als farbloses Oel erhalten wurden.
MS: 391 ($M^+$, 100), 376 (40), 288 (12), 272 (17), 256 (13), 242 (13), 128 (10).

Beispiel 4.1.1.ea

Eine Lösung von 4.56 g (10.9 mMol) 4-(tert.-Butyl-dimethyl-silanyloxymethyl)-3,7-dimethoxy-10-methylphenothiazin in 50 ml Diäthyläther/Tetrahydrofuran (4:1) wurde analog wie in Beispiel 4.1.1.c beschrieben mit 7.5 ml n-Butyllithium-Lösung (1.6M in Hexan) und 1.89 g (16.84 mMol) N-Formylpiperidin umgesetzt. Der erhaltene 3,7-Dimethoxy-4-(tert.butyldimethylsilanyloxymethyl)-10-methylphenothiazin-4-carbaldehyd wurde in 50 ml Tetrahydrofuran gelöst und analog wie in Beispiel 4.1.8.b beschrieben mit 12.0 ml Lithiumborhydrid-Lösung (1M in Tetrahydrofuran) reduziert. Das Reaktionsgemisch wurde auf Eis/1M Natriumdihydrogenphosphat-Lösung gegossen, worauf dreimal mit Essigsäureäthylester extrahiert wurde. Die vereinigten organischen Phasen wurden mit gesättigter Kochsalzlösung gewaschen, über Magnesiumsulfat getrocknet und eingeengt. Der Rückstand wurde an Kieselgel mit Essigsäureäthylester/Hexan (1:1) chromatographiert, worauf nach Umkristallisation aus Essigsäureäthylester/Hexan und Trocknung, 3.30 g (67.6%) [6-(tert.-Butyldimethyl-silanyloxymethyl)-3,7-dimethoxy-10-methyl-phenothiazin-4-yl]methanol als weisser Feststoff vom Smp. 46-47° erhalten wurden.

Beispiel 4.1.1.eb

Eine Lösung von 8.31 g (21.73 mMol) 3,7-Dimethoxy-4-(2-methoxyäthoxymethoxymethyl)-10-methylphenothiazin in 100 ml Diäthyläther/Tetrahydrofuran (4:1) wurde analog wie in Beispiel 4.1.1.c beschrieben mit 17.6 ml n-Butyllithium-Lösung (1.6M in Hexan) und 3.68 g (32.6 mMol) N-Formylpiperidin umgesetzt. Der erhaltene 3,7-Dimethoxy-6-(2-methoxyäthoxymethoxymethyl)-10-methylphenothiazin-4-carbaldehyd wurde in 100 ml Tetrahydrofuran gelöst und analog wie in Beispiel 4.1.8.b beschrieben mit 22 ml

Lithiumborhydrid-Lösung (1M in Tetrahydrofuran) reduziert. Das Rohprodukt wurde an Kieselgel mit Essigsäureäthylester/Hexan (1:1) chromatographiert, worauf nach Trocknung am Hochvakuum, 6.44 g (70.3%) [3,7-Dimethoxy-6-(2-methoxy-äthoxymethoxymethyl)-10-methylphenothiazin-4-yl]-methanol als leicht gelbliches Oel erhalten wurden.
MS: 421 ($M^+$, 100), 406 (24), 316 (10).

Beispiel 4.1.1.fa

Eine Lösung von 1.44 g (3.21 mMol) [6-(tert.-Butyl-dimethyl-silanyloxymethyl)-3,7-dimethoxy-10-methyl-phenothiazin-4-yl]-methanol, 0.70 g (4.75 mMol) Phthalimid und 0.92 g (3.5 mMol) Triphenylphosphin in 15 ml Tetrahydrofuran wurde analog wie in Beispiel 4.1.1.fb beschrieben mit einer Lösung von 0.67 g (3.84 mMol) Azodicarbonsäuredimethylester in 3 ml Tetrahydrofuran umgesetzt. Aufarbeitung und Chromatographie erfolgten ebenfalls analog wie in Beispiel 4.1.1.fb beschrieben. Nach Umkristallisation aus Aethanol/Toluol wurden 0.60 g (32.3%) 2-[6-(tert.-Butyl-dimethyl-silanyloxymethyl)-3,7-dimethoxy-10-methyl-phenothiazin-4-ylmethyl]-2,3-dihydro-1H-isoindol-1,3-dion als weisser Feststoff erhalten.
MS: 576 ($M^+$, 100), 561 (12), 505 (19), 504 (57), 489 (19), 444 (9), 298 (14), 160 (9), 75(13).

Beispiel 4.1.1.fb

Zu einer Lösung von 6.44 g (15.24 mMol) [3,7-Dimethoxy-6-(2-methoxyäthoxymethoxymethyl)-10-methyl-phenothiazin-4-yl]-methanol, 2.68 g (18.11 mMol) Phthalimid und 4.40 g (16.77 mMol) Triphenylphosphin in 75 ml Tetrahydrofuran wurde unter Argon und unter Eiskühlung innerhalb 4 Stunden eine Lösung von 3.19 g (18.32 mMol) Azodicarbonsäuredimethylester in 10 ml Tetrahydrofuran zugegeben. Das Reaktionsgemisch wurde noch 2 Stunden bei 0° gerührt, langsam auf Raumtemperatur gebracht und dann auf Eiswasser und Essigsäureäthylester gegossen. Das Gemisch wurde mit Hexan und Methanol versetzt, worauf kräftig geschüttelt wurde. Die organische Phase wurde abgetrennt, mit gesättigter Natriumhydrogencarbonat-Lösung und gesättigter Kochsalzlösung gewaschen, über Magnesiumsulfat getrocknet und eingedampft, und der Rückstand wurde an Kieselgel mit Essigsäureäthylester/Hexan (1:1) chromatographiert. Nach Umkristallisation aus Methanol wurden 6.83 g (81.2%) 2-[3,7-Dimethoxy-6-(2-methoxy-äthoxymethoxymethyl)-10-methyl-phenothiazin-4-ylmethyl]-2,3-dihydro-1H-isoindol-1,3-dion als weisser Feststoff vom Smp. 135-137° erhalten.

Beispiel 4.1.1.ga

Analog wie in Beispiel 4.1.8.f beschrieben wurde eine Lösung von 500 mg (0.77 mMol) 2-[6-(tert.-Butyl-dimethyl-silanyloxymethyl)-3,7-dimethoxy-10-methyl-phenothiazin-4-ylmethyl]-2,3-dihydro-1H-isoindol-1,3-dion in Methylenchlorid mit Bortribromid-Lösung (1M in Methylenchlorid) und das erhaltene Produkt mit Natriumcyanid in N,N-Dimethylformamid umgesetzt, worauf 317 mg (92%) [6-(1,3-Dioxo-2,3-dihydro-1H-isoindol-2-ylmethyl)-3,7-dimethoxy-10-methyl-phenothiazin-4-yl]-acetonitril als gelber Festkörper erhalten wurden; Smp. 159-160°.

Beispiel 4.1.1.gb

Zu einer Lösung von 6.81 g (12.36 mMol) 2-[3,7-Dimethoxy-6-(2-methoxyäthoxymethoxymethyl)-10-methyl-phenothiazin-4-ylmethyl]-2,3-dihydro-1H-isoindol-1,3-dion in 80 ml Methylenchlorid wurden unter Argon und unter Eiskühlung langsam 12.5 ml Bromwasserstoffsäure (33-prozentig in Eisessig) zugetropft. Das Reaktionsgemisch wurde langsam auf Raumtemperatur gebracht, 1 Stunde gerührt, mit 6 ml Bromwasserstoffsäure (33-prozentig in Eisessig) versetzt, 1 Stunde weitergerührt und in 300 ml eiskalte gesättigte Natriumbicarbonat-Lösung gegossen. Das Gemisch wurde dreimal mit Methylenchlorid extrahiert. Die vereinigten organischen Phasen wurden mit gesättigter Kochsalzlösung gewaschen, über Magnesiumsulfat getrocknet und eingeengt. Der Rückstand wurde 1 Stunde am Hochvakuum getrocknet und dann in 80 ml N,N-Dimethylformamid gelöst, worauf 5.8 g (120 mMol) Natriumcyanid zugegeben wurden. Das Reaktionsgemisch wurde 30 Minuten bei 60° gerührt, abgekühlt und auf 350 ml Eiswasser gegossen. Das ausgefallene Produkt wurde abfiltriert, mit Wasser gewaschen, über Phosphorpentoxid getrocknet und aus Methanol/Aceton umkristallisiert. Nach Trocknung wurden 4.92 g (84.5%) [6-(1,3-Dioxo-2,3-dihydro-1H-isoindol-2-ylmethyl)-3,7-dimethoxy-10-methyl-phenothiazin-4-yl]-acetonitril als gelber Feststoff vom Smp. 159-160° erhalten.

Beispiel 4.1.1.h

Zu einer Lösung von 1.0 g (2.12 mMol) [6-(1,3-Dioxo-2,3-dihydro-1H-isoindol-2-ylmethyl)-3,7-dimethoxy-10-methyl-phenothiazin-4-yl]-acetonitril in 10 ml Dioxan wurden bei Raumtemperatur 8.0 ml Hydrazinhydrat-Lösung (1M in Aethanol) zugegeben. Das Reaktionsgemisch wurde 3 Stunden bei 80° gerührt, abgekühlt, mit 200 ml 10-prozentiger Natriumcarbonat-Lösung versetzt und gut gerührt. Das Gemisch wurde dreimal mit Methylenchlorid extrahiert, und die vereinigten organischen Phasen wurden über Magnesiumsulfat getrocknet und eingeengt. Der Rückstand wurde am Hochvakuum getrocknet und in 30 ml Dioxan gelöst, worauf 30 ml konzentrierte Salzsäure zugegeben wurden. Das Reaktionsgemisch wurde im Bombenrohr 2 Stunden auf 100° erhitzt, abgekühlt und zur Trockene eingedampft, worauf der Rückstand am Hochvakuum getrocknet und in 10 ml Dioxan/Wasser (2:1) gelöst wurde. Die Lösung wurde durch Zutropfen von 1N wässriger Natronlauge auf pH 8 gebracht, unter Eiskühlung mit einer Lösung von 600 mg (2.74 mMol) di-tert.-Butyldicarbonat in 5 ml Dioxan versetzt, 1 Stunde bei Raumtemperatur gerührt und dann auf Eiswasser gegossen. Das Gemisch wurde mit 0.5N Salzsäure leicht angesäuert und dreimal mit Methylenchlorid extrahiert. Die vereinigten organischen Phasen wurden mit Kochsalzlösung gewaschen, über Magnesiumsulfat getrocknet und eingeengt. Der Rückstand wurde an Kieselgel mit Methanol/Chloroform (9:1) chromatographiert, worauf nach Trocknung 580 mg (59.5%) (6-tert.-Butoxycarbonylaminomethyl-3,7-dimethoxy-10-methyl-phenothiazin-4-yl)-essigsäure als amorpher Festkörper erhalten wurden.
MS: 460 ($M^+$, 65), 404 (36), 386 (100), 341 (68), 360 (17), 284 (32), 269 (12), 256 (20), 149 (12), 59 (42), 57 (30), 41 (50).

Beispiel 4.1.2.

Eine Lösung von 510 mg (1.081 mMol) [6-(1,3-Dioxo-2,3-dihydro-1H-isoindol-2-ylmethyl)-3,7-dimethoxy-10-methyl-phenothiazin-4-yl]-acetonitril in 20 ml Dioxan und 20 ml rauchender Salzsäure wurde im Bombenrohr 1 Stunde auf 100° erhitzt. Das Reaktionsgemisch wurde abgekühlt und auf 250 ml Eiswasser gegossen. Der Festkörper wurde abfiltriert, mit Wasser gewaschen und über Phosphorpentoxid getrocknet, wobei 400 mg (75.4%) [6-(1,3-Dioxo-2,3-dihydro-1H-isoindol-2-ylmethyl)-3,7-dimethoxy-10-methylphenothiazin-4-yl]-essigsäure als beiger Feststoff erhalten wurden.
IR (KBr): 3357w (br.), 3092w, 2938w, 2834w, 1772w, 1715s, 1584w, 1463s, 1436m, 1391s, 1346m, 1259s, 1181w, 1048m, 801w, 719m.

Beispiel 4.1.3.

Zu einer eisgekühlten Lösung von 320 mg (0.695 mMol) (6-tert.-Butoxycarbonylaminomethyl-3,7-dimethoxy-10-methyl-phenothiazin-4-yl)-essigsäure, 90.2 mg (0.84 mMol) Benzylalkohol und 20 mg N,N-Dimethylaminopyridin in 2.5 ml Methylenchlorid wurde eine Lösung von 158 mg (0.77 mMol) N,N-Dicyclohexylcarbodiimid in Methylenchlorid zugetropft. Das Reaktionsgemisch wurde über Nacht bei Raumtemperatur gerührt und filtriert. Der Filterrückstand wurde mit Methylenchlorid gewaschen. Die vereinigten Filtrate wurden mit gesättigter Natriumbicarbonat-Lösung gewaschen, über Magnesiumsulfat getrocknet und eingeengt. Der Rückstand wurde am Kieselgel mit Essigsäureäthylester/Hexan (1:4) chromatographiert, worauf nach Umkristallisation aus Aethanol und Trocknung 280 mg (73.3%) (6-tert.-Butoxycarbonylaminomethyl-3,7-dimethoxy-10-methyl-phenothiazin-4-yl)-essigsäurebenzylester als weisse Kristalle vom Smp. 131-132° erhalten wurden.

Beispiel 4.1.4.

Zu einer Lösung von 275 mg (0.5 mMol) (6-tert.-Butoxycarbonylaminomethyl-3,7-dimethoxy-10-methyl-phenothiazin-4-yl)-essigsäurebenzylester in 1.5 ml Methylenchlorid wurden unter Eiskühlung 4 ml Trifluoressigsäure zugegeben. Das Reaktionsgemisch wurde 30 Minuten bei 0° gerührt und dann zur Trockene eingedampft, der Rückstand wurde in Diäthyläther/Hexan suspendiert, die Suspension wurde filtriert, und der Filterrückstand wurde am Hochvakuum getrocknet, worauf 260 mg (92%) (6-Aminomethyl-3,7-dimethoxy-10-methyl-phenothiazin-4-yl)-essigsäurebenzylester-trifluoracetat (1:1) als weisser Feststoff vom Smp. 196° erhalten wurden.
MS: 450 ($M^+$-$CF_3COOH$, 100) 435 (50), 91 (24).

Beispiel 4.1.5.a

Eine Suspension von 15.0 g (57.8 mMol) 3,7-Dimethoxy-phenothiazin, 31.9 g (0.231 mMol) pulverisiertem Kaliumcarbonat, 61.29 g (0.289 Mol) 1-Jodhexan und 1.6 g (5.0 mMol) tris-[2-(2-Methoxymethoxy)-äthyl]amin wurde während 64 Stunden auf 110° erhitzt. Die Aufarbeitung erfolgte analog wie in Beispiel 4.1.1.b beschrieben. Nach Chromatographie an Kieselgel mit Essigsäureäthylester/Hexan (1:4) wurden 18.33 g (91.8%) 10-Hexyl-3,7-dimethoxy-phenothiazin als bräunliches Oel erhalten.
MS: 343 (M+, 45), 273 (15), 259 (18), 258 (100).

Beispiel 4.1.5.b

Eine Lösung von 3.43 g (10.0 mMol) 10-Hexyl-3,7-dimethoxy-phenothiazin in 50 ml Diäthyläther/Tetrahydrofuran (4:1) wurde analog wie in Beispiel 4.1.1.c beschrieben mit 8.12 mi n-Butyllithium-Lösung (1.6M in Hexan) und 3.35 g (29.6 mMol) N-Formylpiperidin umgesetzt. Das Produkt wurde an Kieselgel mit Essigsäureäthylester/Hexan (1:4) chromatographiert, worauf 2.39 g (64.4%) 10-Hexyl-3,7-dimethoxy-phenothiazin-4-carbaldehyd als rotes Oel erhalten wurden.
MS: 371 ($M^+$, 39), 300 (11), 286 (100), 243 (7).

Beispiel 4.1.5.c

Eine Lösung von 2.39g (6.43 mMol) 10-Hexyl-3,7-dimethoxyphenothiazin-4-carbaldehyd in 30 ml Tetrahydrofuran wurde analog wie in Beispiel 4.1.1.da beschrieben mit 7.0 ml Lithiumborhydrid-Lösung (1M in Tetrahydrofuran) reduziert. Das erhaltene 10-Hexyl-3,7-dimethoxyphenothiazin-4-methanol wurde analog wie in Beispiel 4.1.1.db beschrieben mit 4.0 ml n-Butyllithium-Lösung (1.6M in Hexan) und 1.0 g (8.0 mMol) 2-Methoxyäthoxy-methylchlorid umgesetzt. Nach Chromatographie an Kieselgel mit Diäthyläther/Hexan (3:2) wurden 2.33 g (78.5%) 10-Hexyl-3,7-dimethoxy-4-(2-methoxy-äthoxymethoxymethyl)-phenothiazin als leicht gelbliches Oel erhalten.
MS: 461 ($M^+$,83) 376 (100), 356 (16), 256 (23), 242 (27), 241 (13), 228 (9), 59 (15), 45 (17), 43 (26).

Beispiel 4.1.5.d

Eine Lösung von 8.0 g (17.33 mMol) 10-Hexyl-3,7-dimethoxy-4-(2-methoxy-äthoxymethoxymethyl)-phenothiazin in 100 ml Diäthyläther/Tetrahydrofuran (4:1) wurde analog wie in Beispiel 4.1.1.c beschrieben mit 12.0 ml n-Butyllithium-Lösung (1.6M in Hexan) und 2.94 g (26.0 mMol) N-Formylpiperidin umgesetzt. Der erhaltene 3,7-Dimethoxy-10-hexyl-6-(2-methoxyäthoxymethoxymethyl)phenothiazin-4-carbaldehyd wurde in 100 ml Tetrahydrofuran gelöst und analog wie in Beispiel 4.1.8.b beschrieben mit 22 ml Lithiumborhydrid-Lösung (1M in Tetrahydrofuran) reduziert. Das Produkt wurde an Kieselgel mit Essigsäureäthylester/Hexan (1:1) chromatographiert, worauf nach Trocknung am Hochvakuum, 4.41 g (51.7%) [10-Hexyl-3,7-dimethoxy-6-(2-methoxy-äthoxymethoxymethyl)-phenothiazin-4-yl]-methanol als leicht gelbliches Oel erhalten wurden.
MS: 491 ($M^+$, 100), 407 (24), 406 (90), 386 (9).

Beispiel 4.1.5.e

Eine Lösung von 11.38 g (24.65 mMol) [10-Hexyl-3,7-dimethoxy-6-(2-methoxy-äthoxymethoxymethyl)-phenothiazin-4-yl]-methanol, 5.44 g (37.0 mMol) Phthalimid und 7.11 g (27.1 mMol) Triphenylphosphin in 100 ml Tetrahydrofuran wurde analog wie in Beispiel 4.1.1.fb beschrieben mit einer Lösung von 5.15 g (29.6 mMol) Azodicarbonsäuredimethylester, in 15 ml Tetrahydrofuran umgesetzt. Aufarbeitung und Chromatographie erfolgten ebenfalls analog wie in Beispiel 4.1.1.fb beschrieben. Nach Umkristallisation aus Aethanol wurden 14.13 g (92.3%) 2-[10-Hexyl-3,7-dimethoxy-6-(2-methoxyäthoxymethoxymethyl)-phenothiazin-4-ylmethyl]-2,3-dihydro-1H-isoindol-1,3-dion als weisser Feststoff vom Smp. 86-87° erhalten.

Beispiel 4.1.5.f

Analog wie in Beispiel 4.1.1.gb beschrieben wurde eine Lösung von 13.32 g (21.34 mMol) 2-[10-Hexyl-3,7-dimethoxy-6-(2-methoxyäthoxymethoxymethyl)-phenothiazin-4-ylmethyl]-2,3-dihydro-1H-isoindol-1,3-dionin 70 ml Methylenchlorid mit insgesamt 30 ml Bromwasserstoffsäure (33-prozentig in Eisessig) behandelt und das Produkt mit 10.4 g (0.212 Mol) Natriumcyanid in 70 ml N,N-Dimethylformamid

umgesetzt. Nach Umkristallisation aus Aethanol/tert.-Butylmethyläther wurden 9.45 g (81.3%) [6-(1,3-Dioxo-2,3-dihydro-1H-isoindol-2-ylmethyl)-10-hexyl-3,7-dimethoxy-phenothiazin-4-yl]-acetonitril als gelber Festkörper vom Smp. 186-187° erhalten.

Beispiel 4.1.5.g

Analog wie in Beispiel 4.1.1.h beschrieben wurden ausgehend von einer Lösung von 2.16 g (4.0 mMol) [6-(1,3-Dioxo-2,3-dihydro-1H-isoindol-2-ylmethyl)-10-hexyl-3,7-dimethoxy-phenothiazin-4yl]-acetonitril in 20 ml Dioxan mittels 8.0 ml Hydrazinhydrat-Lösung (1M in Aethanol), 40 ml konzentrierter Salzsäure und einer Lösung von 1.09 g (5.0 mMol) di-tert.-Butyldicarbonat in Dioxan, 1.25 g (58.9%) (6-tert.-Butoxycarbonylaminomethyl-10-hexyl-3,7-dimethoxy-phenothiazin-4-yl)-essigsäure als amorpher Festkörper erhalten.
MS: 531 ($M^+$+1, 38), 530 ($M^+$, 100), 475 (10), 474 (10), 429 (15), 414 (55), 389 (75), 345 (12).

Beispiel 4.1.6

Eine Lösung von 610 mg (1.15 mMol) (6-tert.-Butoxycarbonylaminomethyl-10-hexyl-3,7-dimethoxy-phenothiazin-4-yl)-essigsäure, 150 mg (1.38 mMol) Benzylalkohol und 30 mg N,N-Dimethylaminopyridin in 5 ml Methylenchlorid wurde analog wie in Beispiel 4.1.3. beschrieben mit einer Lösung von 261 mg (1.26 mMol) N,N-Dicyclohexylcarbodiimid in Methylenchlorid umgesetzt. Aufarbeitung und Chromatographie erfolgten analog wie in Beispiel 4.1.3. beschrieben. Nach Umkristallisation aus Aethanol und Trocknung wurden 370 mg (51.8%) (6-tert.-Butoxycarbonylaminomethyl-10-hexyl-3,7-dimethoxy-phenothiazin-4-yl)-essigsäurebenzylester als weisser Feststoff erhalten.
MS: (FAB): 620 ($M^+$, 100), 564 (16), 521 (12), 479 (22).

Beispiel 4.1.7.

(6-tert.-Butoxycarbonylaminomethyl-10-hexyl-3,7-dimethoxy-phenothiazin-4-yl)-essigsäurebenzylester wurde analog wie in Beispiel 4.1.4. beschrieben in (6-Aminomethyl-3,7-dimethoxy-10-hexyl-phenothiazin-4-yl)essigsäurebenzylester-trifluoracetat (1:1) übergeführt.

Beispiel 4.1.8.a

Eine Suspension von 10.0 g (38.56 mMol) 3,7-Dimethoxy-phenothiazin, 21,3 g (0.154 Mol) pulverisiertem Kaliumcarbonat, 33.0 g (0.193 Mol) Benzylbromid und 1.3 g (4.0 mMol) tris-[2-(2-Methoxyäthoxy)äthyl]-amin wurde während 2 Stunden auf 110° erhitzt. Die Aufarbeitung erfolgte analog wie in Beispiel 4.1.1.b beschrieben. Nach Chromatographie an Kieselgel mit Essigsäureäthylester/Hexan (1:4) und Umkristallisation aus Aethanol/Wasser (9:1) erhielt man 12.06 g (89.5%) 10-Benzyl-3,7-dimethoxyphenothiazin als weisse Kristalle vom Smp. 110°.

Beispiel 4.1.8.b

Eine Lösung von 10.0 g (28.6 mMol) 10-Benzyl-3,7-dimethoxy-phenothiazin in 150 ml Diäthyläther/Tetrahydrofuran (4:1) wurde analog wie in Beispiel 4.1.1.c beschrieben mit 21.5 ml n-Butyllithium-Lösung (1.6M in Hexan) und 4.9 g (42.9 mMol) N-Formylpiperidin umgesetzt.

Der erhaltene rohe 10-Benzyl-3,7-dimethoxyphenothlazin-4-carbaldehyd wurde in 150 ml absolutem Tetrahydofuran gelöst, worauf die Lösung bei 0° mit 31.5 ml Lithiumborhydrid-Lösung (1M in Tetrahydrofuran) versetzt wurde. Das Reaktionsgemisch wurde langsam auf Raumtemperatur gebracht, 1 Stunde gerührt und dann auf Eiswasser gegossen, und der pH wurde mit 0.5N Salzsäurelösung auf 3-4 gestellt. Das Gemisch wurde mit Essigsäureäthylester extrahiert. Die vereinigten organischen Phasen wurden mit gesättigter Kochsalzlösung gewaschen, über Magnesiumsulfat getrocknet und eingedampft. Nach Chromatographie an Kieselgel mit Essigsäureäthylester/Hexan (1:2) erhielt man 8.0 g (73.7%) (10-Benzyl-3,7-dimethoxy-phenothiazin-4-yl)-methanol als weissen amorphen Feststoff.
MS: 379 ($M^+$, 8), 289 (19), 288 (100), 91 (27), 65 (11).

Beispiel 4.1.8.c

Eine Lösung von 21.9 g (57.7 mMol) (10-Benzyl-3,7-dimethoxy-phenothiazin-4-yl)-methanol und 8.57 g (0.126 Mol) Imidazol in 300 ml N,N-Dimethylformamid wurde analog wie in Beispiel 4.1.1.da beschrieben

mit einer Lösung von 10.47 g (69.4 mMol) tert.-Butyldimethylchlorsilan in 25 ml N,N-Dimethylformamid umgesetzt. Nach Umkristallisation aus Hexan/tert.-Butylmethyläther erhielt man 22.2 g (77.9%) 10-Benzyl-4-(tert.-butyl-dimethyl-silanyloxymethyl)-3,7-dimethoxy-phenothiazin als weissen Feststoff vom Smp. 55°.

Beispiel 4.1.8.d

Eine Lösung von 4.93 g (10.0 mMol) 10-Benzyl-4-(tert.-butyl-dimethylsilanyloxymethyl)-3,7-dimethoxy-phenothiazin in 50 ml Diäthyläther/Tetrahydrofuran (4:1) wurde analog wie in Beispiel 4.1.1.c beschrieben mit 7.5 ml n-Butyllithium-Lösung (1.6M in Hexan) und 1.47 g (13.0 mMol) N-Formylpiperidin umgesetzt. Der erhaltene 10-Benzyl-3,7-dimethoxy-6-(tert.-butyldimethylsilanyloxymethyl)phenothiazin-4-carbaldehyd wurde in 50 ml Tetrahydrofuran gelöst und analog wie in Beispiel 4.1.8.b beschrieben mit 10.0 ml Lithiumborhydrid-Lösung (1M in Tetrahydrofuran) reduziert. Das Reaktionsgemisch wurde analog wie in Beispiel 4.1.1.ea beschrieben aufgearbeitet und chromatographiert. Nach Trocknung am Hochvakuum wurden 3.72 g (71.9%) 10-Benzyl-6-(tert.-butyl-dimethylsilanyloxymethyl)-3,7-dimethoxy-phenothiazin-4-yl-methanol als amorpher weisser Festkörper erhalten.
MS: 523 ($M^+$, 8), 434 (14), 433 (33), 432 (100), 360 (11), 91 (22), 75 (7).

Beispiel 4.1.8.e

Eine Lösung von 3.19 g (6.09 mMol) 10-Benzyl-6-(tert.-butyldimethylsilanyloxymethyl)-3,7-dimethoxy-phenothiazin-4-yl-methanol, 1.34 g (9.10 mMol) Phthalimid und 1.75 g (6.67 mMol) Triphenylphosphin in 30 ml Tetrahydrofuran wurde analog wie in Beispiel 4.1.1fb beschrieben mit einer Lösung von 1.27 g (7.29 mMol) Azodicarbonsäuredimethylester umgesetzt. Aufarbeitung und Chromatographie erfolgten ebenfalls analog wie in Beispiel 4.1.1.fb beschrieben. Nach Umkristallisation aus Aethanol wurden 3.27 g (82.2%) 2-[10-Benzyl-6-(tert.-butyl-dimethyl-silanyloxymethyl)-3,7-dimethoxy-phenothiazin-4-ylmethyl]-2,3-dihydro-1H-isoindol-1,3-dion als leicht gelblicher Feststoff erhalten.
MS: 652 ($M^+$,8), 563 (26), 562 (56), 561 (100), 490 (18), 489 (10), 91 (16).

Beispiel 4.1.8.f

Zu einer Lösung von 3.25 g (4.97 mMol) 2-[10-Benzyl-6-(tert.-butyldimethyl-silanyloxymethyl)-3,7-dimethoxy-phenothiazin-4-ylmethyl]-2,3-dihydro-1H-isoindol-1,3-dion in 25 ml Methylenchlorid wurden unter Eiskühlung und unter Argon langsam 5.0 ml Bortribromid-Lösung (1M in Methylenchlorid) zugetropft. Das Reaktionsgemisch wurde 15 Minuten bei 0° und 2 Stunden bei Raumtemperatur gerührt. Aufarbeitung und Umsetzung des Produkts mit 3.25 g (50.0 mMol) Natriumcyanid erfolgten analog wie in Beispiel 4.1.1.gb beschrieben. Nach Kristallisation aus Essigsäureäthylester/Hexan wurden 2.71 g (99%) [10-Benzyl-6-(1,3-dioxo-2,3-dihydro-1H-isoindol-2-ylmethyl)-3,7-dimethoxy-phenothiazin-4-yl]acetonitril als gelber Festkörper vom Smp. 230° erhalten.

Beispiel 4.1.8.g

Eine Lösung von 1.09 g (2.0 mMol) [10-Benzyl-6-(1,3-dioxo-2,3-dihydro-1H-isoindol-2-ylmethyl)-3,7-dimethoxy-phenothiazin-4-yl]acetonitril in 10 ml Dioxan wurde analog wie in Beispiel 4.1.1.h beschrieben mit 10.0 ml Hydrazinhydrat-Lösung (1M in Aethanol) umgesetzt. Das erhaltene Produkt wurde in 20 ml Aethanol gelöst und mit 20 ml 6N wässriger Natronlauge versetzt, worauf das Gemisch 2 Stunden bei 110° gerührt und dann zur Trockene eingedampft wurde. Der Rückstand wurde mit 2N wässriger Salzsäure neutralisiert, und die Lösung wurde eingeengt. Der Rückstand wurde am Hochvakuum getrocknet und anschliessend analog wie in Beispiel 4.1.1.h beschrieben mit 654 mg (3.0 mMol) di-tert.-Butyldicarbonat in Dioxan umgesetzt. Aufarbeitung und Chromatographie erfolgten analog wie in Beispiel 4.1.1.h beschrieben. Nach Trocknung wurden 650 mg (60.5%) (10-Benzyl-6-tert.-butoxycarbonylaminomethyl-3,7-dimethoxy-phenothiazin-4-yl)-essigsäure als amorpher Feststoff erhalten.
MS: 536 ($M^+$, 5), 445 (27), 389 (42), 371 (33), 345 (71), 97 (29), 83 (34), 71 (45), 69 (65), 57 (82), 41 (100).

Beispiel 4.1.9.

Eine Suspension von 776 mg (1.44 mMol) (10-Benzyl-6-tert.-butoxycarbonylaminomethyl-3,7-dimethoxy-phenothiazin-4-yl)-essigsäure und 100 mg Palladium auf Kohle in 10 ml Methanol wurde mit 0.5 ml Essigsäure versetzt und über das Wochenende unter 1.5 bar Wasserstoff hydriert. Das Gemisch wurde

filtriert, das Filtrat wurde eingeengt, und der Rückstand wurde am Hochvakuum getrocknet. Nach Chromatographie an Kieselgel mit Chloroform/Methanol (9:1) und Trocknung wurden 585 mg (91%) [6-(1-tert.-Butoxycarbonylaminomethyl)-3,7-dimethoxylphenothiazin-4-y-]-essigsäure als leicht bräunlicher amorpher Schaum erhalten. FAB (MS): 446 ($M^+$, 20).

Zu einer Lösung von 585 mg (1.31 mMol) der erhaltenen Verbindung in 10 ml N,N-Dimethylformamid wurden unter Eiskühlung und unter Argon 0.34 g (1.97 mMol) Benzylbromid und innerhalb von 1 Stunde 0.50 ml (1.97 mMol) Diazabicycloundecan (DMU) zugetropft. Das Reaktionsgemisch wurde 30 Minuten bei 0° gerührt, langsam auf Raumtemperatur gebracht, noch 2 Stunden gerührt und auf Wasser gegossen, worauf mit Essigsäureäthylester extrahiert wurde. Die organische Phase wurde zweimal mit Wasser und mit 0.5N wässriger Salzsäure extrahiert, über Magnesiumsulfat getrocknet und eingedampft. Der Rückstand wurde an Kieselgel mit Essigsäureäthylester/Hexan (1:1) chromatographiert, worauf 523 mg (75%) (6-tert.-Butoxycarbonylaminomethyl-3,7-dimethoxy-phenothiazin-4-yl)-essigsäurebenzylester als beiger Festkörper erhalten wurden. FAB (MS): 536 ($M^+$, 100), 480 (70), 390 (65).

Beispiel 4.1.10.

Ein Gemisch von 360 mg (0.67 mMol) (6-tert.-Butoxycarbonylaminomethyl-3,7-dimethoxy-phenothiazin-4-yl)-essigsäurebenzylester, 91.7 mg (0.80 mMol) Glutarsäureanhydrid und 63.7 mg (0.80 mMol) Pyridin in 5 ml Toluol wurde mit 10 mg N,N-Dimethylaminopyridin versetzt. Das Gemisch wurde unter Argon während 48 Stunden auf 110° erhitzt und dann auf Eiswasser gegossen, worauf mit 1N Salzsäure angesäuert und mit Methylenchlorid erschöpfend extrahiert wurde. Die organische Phase wurde über Magnesiumsulfat getrocknet und eingeengt. Der Rückstand wurde an Kieselgel mit Chloroform/Methanol (9:1) chromatographiert, worauf 310 mg (71%) 5-(4-Benzyloxycarbonylmethyl-6-tert.-butoxycarbonylaminomethyl-3,7-dimethoxy-phenothiazin-10yl)-5-oxo-pentansäure als weisser Feststoff vom Smp. 65-68° erhalten wurden. MS (FAB): 651 ($M^+ + H$, 20), 595 (20), 536 (20), 389 (25), 217 (80), 91 (100).

Beispiel 4.2.1.

Eine Lösung von 364 mg (0.74 mMol) [6-(1,3-Dioxo-2,3-dihydro-1H-isoindol-2-ylmethyl)-3,7-dimethoxy-10-methyl-phenothiazin-4-yl]-essigsäure, 309 mg (0.815 mMol) 1-Benzotriazo-1-yl-N,N,N',N'-tetramethyluroniumhexafluorophosphat (HBTU) und 253.2 mg (0.815 mMol) Gly-Gly-Ala-Gly-methylester-hydrochlorid in 3 ml N,N-Dimethylformamid wurde unter Eiskühlung mit 187.3 mg (1.85 mMol) N-Methylmorpholin versetzt. Das Reaktionsgemisch wurde 30 Minuten bei Raumtemperatur gerührt, dann mit Wasser versetzt und schliesslich filtriert. Der Rückstand wurde mit Waser gewaschen, über Phosphorpentoxid getrocknet und in Methanol kurz aufgekocht. Der Festkörper wurde abfiltriert und getrocknet, worauf man 450 mg (81.5%) [3,7-Dimethoxy-10-methyl-6-(1,3-dioxo-2,3-dihydro-1H-isoindol-2-ylmethyl)-phenothiazin-4-yl]-acetyl-glycyl-glycyl-D-alanyl-glycinmethylester als leicht grünlicher Feststoff erhalten wurde. Smp. >210°. MS (FAB): 747 ($M^+$, 10), 367 (19), 318 (10), 223 (20), 185 (45), 156 (60), 119 (100).

Beispiel 4.2.2.

Zu einer Lösung von 390 mg (0.522 mMol) des Produktes von Beispiel 4.2.1. in 10 ml Methanol wurde 1 ml Hydrazinhydrat zugegeben. Das Reaktionsgemisch wurde während 6 Stunden bei 50° gerührt, dann abgekühlt und filtriert. Der Filterrückstand wurde mit Methanol gewaschen, getrocknet und in 5 ml N,N-Dimethylformamid gelöst. Die Lösung wurde auf 0° gekühlt, worauf 1 ml rauchende Salzsäure zugetropft und dann 0.5 ml 14-prozentige Natriumnitrit-Lösung zugegeben wurden. Das Gemisch wurde 45 Minuten bei -10° gerührt und dann tropfenweise mit 1 ml N-Methylmorpholin versetzt. Das Reaktionsgemisch wurde 1 Stunde gerührt und dann zur Trockene eingedampft. Der Rückstand wurde an Kieselgel mit Chloroform/Methanol (4:1) chromatographiert, worauf 75 mg (24.6%) 3,7-Dimethoxy-10-methyl-4,6-cyclo-[acetyl-glycyl-glycyl-L-alanylglycylaminomethyl]-phenothiazin als weisser Feststoff erhalten wurden. Smp. >260°. MS (FAB): 585 ($M^+ + H$, 60), 318 (10), 253 (10), 200 (10), 136 (25), 110 (25), 87 (100).

Beispiel 4.3.1.

Zu einer Lösung von 150 mg (0.265 mMol) (6-Aminomethyl-3,7-dimethoxy-10-methyl-phenothiazin-4-yl)-essigsäure-benzylester-trifluoracetat (1:1) in 30 ml Acetonitril wurden 25 mg getrocknetes Natriumhydrogencarbonat und dann bei 0° 238.2 mg (0.35 mMol) tri-Carbobenzoxy-argininhydroxysuccinimidester gegeben. Das Reaktionsgemisch wurde 30 Minuten bei 0° und über Nacht bei Raumtemperatur gerührt

und dann auf Eiswasser und Methylenchlorid gegossen, worauf die organische Phase mit Kochsalzlösung gewaschen, über Magnesiumsulfat getrocknet und eingeengt wurde. Der Rückstand wurde am Hochvakuum getrocknet, in 20 ml 2,2,2-Trifluoräthanol aufgenommen und mit 150 mg Palladium auf Kohle (10%) während 2 Stunden unter Normaldruck hydriert. Der Katalysator wurde abfiltriert, und das Filtrat wurde eingengt. Der Rückstand wurde aus Diäthyläther gefällt, abfiltriert, am Hochvakuum getrocknet und in 5 ml N,N-Dimethylformamid aufgenommen. Das Gemisch wurde mit 136.4 mg (0.36 mMol) 1-Benzotriazol-1-yl-N,N,N',N'-tetramethyl-uroniumhexafluorphosphat (HBTU) und dann bei 0° mit 91.0 mg (0.9 mMol) N-Methylmorpholin versetzt. Das Reaktionsgemisch wurde über Nacht bei Raumtemperatur gerührt, worauf das Lösungsmittel abdestilliert wurde. Der Rückstand wurde in Wasser und Chloroform suspendiert, abfiltriert und am Hochvakuum getrocknet, worauf 80 mg (50%) (S)-4,12-Dimethoxy-8-(3-guanidino-propyl)-17-methyl-1,15-imino-6,7,8,9,10,11-hexahydro-5H-dibenzo[b,k][1,5,8]thiadiazacyclododecin-7,10-dion-trifluoracetat (1:1) als grauer Festkörper erhalten wurde.
MS (FAB): 499 ($M^+$(freie Base) + H, 35), 431 (10), 239 (20), 217( 100), 131 (65), 126 (50), 109 (95).

Beispiel 4.3.2.

Aus 112 mg (6-Aminomethyl-3,7-dimethoxy-10-methyl-phenothiazin-4-yl)-essigsäure-benzylester-trifluoracetat und 270 mg Z-Arg(Pmc)-Gly-Asp(OBut)-Val-OH wurden analog wie in Beispiel 2.2.2. beschrieben 24 mg 3,7-Dimethoxy-10-methyl-4,6-cyclo-[acetyl-L-arginyl-glycyl-L-aspartyl-L-valyl-aminomethyl]-phenothiazin-trifluoracetat (1:1) erhalten. MS: 770 $MH^+$.

Beispiel 4.4.1.

Zu einer Lösung von 150 mg (0.33 mMol) (6-tert.-Butoxycarbonylaminomethyl-3,7-dimethoxy-10-methyl-phenothiazin-4-yl)-essigsäure, 65.02 mg (0.36 mMol) L-Alanin-tert.-butylester-hydrochlorid und 135.8 mg (0.36 mMol) 1-Benzotriazol-1-yl-N,N,N',N'-tetramethyl-uronium-hexafluorphosphat (HBTU) in 5 ml N,N-Dimethylformamid wurden bei 0° 82.5 mg (0.82 mMol) 4-Methylmorpholin zugegeben. Das Reaktionsgemisch wurde langsam auf Raumtemperatur gebracht, 1 Stunde gerührt und auf Eiswasser gegossen. Der ausgefallene Niederschlag wurde abfiltriert, mit Wasser gewaschen, über Phosphorpentoxid getrocknet und in 5 ml kalter Trifluoressigsäure 1 Stunde bei 0° gerührt, worauf am Hochvakuum zur Trockene eingedampft wurde. Der amorphe Rückstand wurde am Hochvakuum getrocknet und in 10 ml N,N-Dimethylformamid gelöst. Die Lösung wurde unter Eiskühlung zu einer Suspension von 152 mg Natriumhydrogencarbonat (pulverisiert und getrocknet) und 142.8 mg (0.537 mMol) Diphenyl-phosphorylazid (DPPA) gegeben. Das Reaktionsgemisch wurde 1 Stunde bei 0° gerührt und dann zur Trockene eingedampft. Der Rückstand wurde in Wasser und Chloroform aufgenommen, die organische Phase wurde abgetrennt, und die wässrige Phase wurde mit Chloroform extrahiert. Die vereinigten organischen Phasen wurden über Magnesiumsulfat getrocknet und eingedampft. Der Rückstand wurde aus Methanol/Hexafluorisopropanol kristallisiert und getrocknet, worauf 100 mg (74.6%) (S)-4,12-Dimethoxy-8,17-dimethyl-1,15-imino-6,7,8,9,10,11-hexahydro-5H-dibenzo[b,k][1,5,8]thiadiazacyclododecin-7,10-dion als weisser Feststoff erhalten wurden.
MS (FAB): 414 ($M^+$ + H, 33), 413 ($M^+$, 20), 325 (33), 217 (100), 126 (75), 109 (90).

Beispiel 4.4.2.

a) 184 mg (6-tert.-Butoxycarbonylaminomethyl-3,7-dimethoxy-10-methylphenothiazin-4-yl)-essigsäure in 3 ml DMF wurde nacheinander mit 236.7 mg Gly-Gly-OBzl• ToSOH, 379 mg HBTU und 0.34 ml DIPEA versetzt. Das Reaktionsgemisch wurde in verdünnte $NaHCO_3$-Lösung gegossen, und der ausgefallene Niederschlag wurde abfiltriert, auf dem Filter mit $KHSO_4/K_2SO_4$-Lösung und Wasser gewaschen und in 10 ml 1N HCl/Essigsäure gelöst. Nach 10 Minuten wurde Aether zugegeben, wobei ein Produkt ausfiel, welches abgetrennt und in 5 ml DMF gelöst wurde. Die Lösung wurde mit 175 mg Boc-Gly-OH, 379 mg HBTU und 0.34 ml DIPEA versetzt, worauf das Reaktionsgemisch in verdünnte $NaHCO_3$-Lösung gegossen wurde. Der ausgefallene Niederschlag wurde abfiltriert und aus Aethanol kristallisiert, wobei 195 mg [6-((tert.-Butoxycarbonylglycyl)aminomethyl)-3,7-dimethoxy-10-methyl-phenothiazin-4-yl]-acetyl-glycyl-glycin-benzylester erhalten wurden; MS: 722 $MH^+$.
b) Das erhaltene Produkt wurde in 10 ml 1N HCl/Essigsäure gelöst, worauf nach 10 Minuten Aether zugegeben wurde. Der ausgefallene Niederschlag wurde abfiltriert und in 10 ml DMF gelöst, worauf die Lösung mit 210 mg Z-Asp(OBut)-OSu und 0.17 ml DIPEA versetzt wurde. Nach 18-stündigem Rühren wurde die Reaktionslösung in verdünnte $NaHCO_3$-Lösung gegossen. Der ausgefallene Niederschlag wurde abfiltriert, mit $KHSO_4/K_2SO_4$-Lösung und Wasser gewaschen und im Vakuum getrocknet, wobei

202 mg [6-((N-Benzyloxycarbonyl-O-tert.-butyl-L-aspartyl)-glycylaminomethyl)-3,7-dimethoxy-10-methyl-phenothiazin-4-yl]-acetyl-glycylglycin-benzylester erhalten wurden; MS: 927 $MH^+$.

c) 190 mg dieser Verbindung wurden analog erhalten, wie in Beispiel 2.2.1. beschrieben hydriert und cyclisiert. Aus dem Cyclisationsprodukt wurde die Schutzgruppe mit 1N HCl/Essigsäure abgespalten, und das erhaltene Produkt wurde aus Essigsäure lyophilisiert, wobei 78 mg 3,7-Dimethoxy-10-methyl-4,6-cyclo[-acetyl-glycyl-L-aspartyl-glycyl-glycylaminomethyl-]pheno-thiazin erhalten wurden; MS: 629 $MH^+$.

Beispiel 4.5.1.

Eine Lösung von 150 mg (6-tert.-Butoxycarbonylaminomethyl-10-hexyl-3,7-dimethoxy-phenothiazin-4-yl)-essigsäure in 5 ml Trifluoressigsäure wurde 1 Stunde bei 20° stehen gelassen und dann im Vakuum eingedampft. Der Rückstand wurde in 5 ml DMF gelöst, worauf der pH mit DIPEA auf 8.5 eingestellt wurde und bei 0° 269 mg $Z_3$-Arg-OSu zugegeben wurden. Das Reaktionsgemisch wurde 1 Stunde bei 20° gerührt und dann in 5% $KHSO_4$/10% $K_2SO_4$-Lösung gegossen. Der ausgefallene Festkörper wurde abfiltriert und aus Aethanol kristallisiert. Die Kristalle wurden in 20 ml Trifluoräthanol gelöst und in Gegenwart von 10% Pd-C hydriert. Die weitere Verarbeitung erfolgte analog wie in Beispiel 2.2.1. beschrieben, und es wurden 91 mg (S)-8-(3-Guanidino-propyl)-17-hexyl-4,12-dimethoxy-1,15-imino-6,7,8,9,10,11-hexahydro-5H-dibenzo[b,k][1,5,8]thiadiazacyclododecin-7,10-dion-trifluoracetat (1:1) erhalten. MS: 569 $MH^+$.

Beispiel 4.5.2.

a) Eine Lösung von 700 mg (1.31 mMol) (6-tert.-Butoxycarbonylaminomethyl-10-hexyl-3,7-dimethoxy-phenothiazin-4-yl) essigsäure, 547 mg (1.44 mMol) 1-Benzotriazol-1-yl-N,N,N',N'-tetramethylnoniumhexa-fluorphosphat (HBTU) und 488.1 mg (1.44 mMol) Gly-Gly-Ala-Gly-methyl-ester-hydrochloridin 5 ml DMF wurde analog wie in Beispiel 4.8.3. beschrieben bearbeitet, worauf nach Chromatographie an Kieselgel mit Chloroform/Methanol (9:1) 800 mg (77.6%) (6-tert.-Butoxycarbonylaminomethyl-10-hexyl-3,7-dimethoxy-phenothiazin-4-yl)-acetyl-glycyl-glycyl-L-alanyl-glycin-methylester als amorpher Schaum erhalten wurden.

MS (FAB): 786 ($M^+$, 85), 687 (100).

IR (KBr): 3298m, 2932w, 1740w, 1700s, 1656s, 1634s, 1518s, 1462s, 1365m, 1256s, 1166m, 1049m.

b) Eine Lösung von 58.5 mg (0.074 mMol) der obigen Verbindung wurde in 2 ml Methanol analog wie in Beispiel 4.8.3. beschrieben mit 2 ml wässriger 2N Natronlauge und anschliessend mit 27.5 mg (0.1 mMol) Diphenylphosphorylazid und 31 mg (0.37 mMol) Natriumhydrogencarbonat in 5 ml N,N-Dimethylformamid behandelt. Der Rückstand wurde mittels präparativer Hochdruckflüssigkeits-Chromatographie (RP 18, Acetonitril/Wasser/0.04% Trifluoressigsäure) gereinigt, worauf nach Lyophilisierung 20 mg (41.2%) 10-Hexyl-3,7-dimethoxy-4,6-cyclo-[-acetyl-glycyl-glycyl-L-alanylaminomethyl-]-phenothiazin als amorpher Schaum erhalten wurden.

MS (FAB): 655 ($M^+$ + H, 100), 654 ($M^+$, 69).

Beispiel 4.6.1.

a) Analog wie in Beispiel 2.2.2. beschrieben, wurde Z-Gly-Arg(Pmc)-Gly-OH durch Festphasensynthese hergestellt; MS: 689 $MH^+$.

b) Eine Lösung von 217 mg (6-tert.-Butoxycarbonylaminomethyl-10-hexyl-3,7-dimethoxy-phenothiazin-4-yl)-essigsäure-benzylester in 10 ml Trifluoressigsäure wurde 1 Stunde bei 20° stehen gelassen und dann im Vakuum eingeengt. Der Rückstand wurde analog wie in Beispiel 2.2.1. beschrieben unter Verwendung von 276 mg Z-Gly-Arg(Pmc)-Gly-OH weiterverarbeitet. Es wurden 40 mg 10-Hexyl-3,7-dimethoxy-4,6-cyclo[-acetylglycyl-L-arginyl-glycyl-aminomethyl-]-phenothiazin-trifluoracetat (1:1) erhalten; MS: 683 $MH^+$.

Beispiel 4.7.1.

a) 285.7 mg (6-Aminomethyl-3,7-dimethoxy-10-hexyl-phenothiazin-4-yl)essigsäurebenzylester-trifluoracetat un 298 mg Fmoc-Arg(Pmc)-OH in 5 ml DMF wurden bei 0° mit 160.6 mg TBTU und 0.17 ml DIPEA versetzt. Das Reaktionsgemisch wurde 30 Minuten bei 20° gerührt und dann in verdünnte $NaHCO_2$-Lösung eingegossen. Der ausgefallene Festkörper wurde abfiltriert, mit $KHSO_4$/$K_2SO_4$-Lösung und

Wasser gewaschen und im Vakuum getrocknet. Es wurden 460 mg [6-(((N$^{\alpha}$(9H-Fluoren-9-ylmethoxycarbonyl)-N$^{6}$-(2,2,5,7,8-pentamethylchroman-6-sulfonyl)-L-arginyl)-aminomethyl)-10-hexyl-3,7-dimethoxy-phenothiazin-4-yl]-essigsäurebenzylester erhalten; MS: 1165 MH$^{\oplus}$.

b) Eine Lösung von 232 mg der erhaltenen Verbindung in 2 ml Piperidin und 8 ml DMF wurde 1 Stunde bei 20° stehen gelassen und anschliessend im Vakuum eingedampft. Der Rückstand wurde mit Hexan digeriert, in 5 ml DMF gelöst und mit 95.5 mg Z-Glu(OBut)-OSu versetzt. Das Reaktionsgemisch wurde 2 Stunden gerührt und dann im Vakuum eingedampft, worauf der Rückstand aus Essigsäureäthylester/Hexan kristallisiert wurde. Es wurden 165 mg [6-(((N$^{\alpha}$-Benzyloxycarbonyl-O-tert.-butyl-L-glutamyl)-(N$^{6}$-(2,2,5,7,8-pentamethylchroman-6-sulfonyl)-L-arginyl)-aminomethyl)-10-hexyl-3,7-dimethoxy-phenothiazin-4-yl]-essigsäurebenzylester erhalten; MS: 1262.5 MH$^{+}$.

c) 139 mg der erhaltenen Verbindung wurden wie in Beispiel 2.2.1. beschrieben weiterverarbeitet, wobei aber die chromatographische Reinigung mit Trifluoressigsäure/Wasser erfolgte. Es wurden 2.5 mg 3,7-Dimethoxy-10-hexyl-4,6-cyclo-[-acetyl-L-α-glutamyl-L-arginylaminomethyl-]phenothiazin-trifluoracetat (1:1) erhalten; MS: 698.3 MH$^{+}$.

Beispiel 4.7.2.

a) 280 mg [6-((N$^{\alpha}$-(9H-Fluoren-9-ylmethoxycarbonyl)-N$^{6}$-(2,2,5,7,8-pentamethylchroman-6-sulfonyl)-L-arginyl)-aminomethyl)-10-hexyl-3,7-dimethoxy-phenothiazin-4-yl)-essigsäurebenzylester wurden in 2 ml Piperidin und 8 ml DMF gelöst. Die Lösung wurde 1 Stunde bei 20° stehen gelassen und dann im Vakuum eingeengt. Der Rückstand wurde mit Hexan digeriert und in 5 ml DMF gelöst, worauf unter Verwendung von 157 mg Z-Gln(Trt)-OH, 97 mg TBTU und 0.1 ml DIPEA analog wie in Beispiel 4.7.1. Absatz b) beschrieben verfahren wurde. Es wurden 280 mg [6-(((N$^{\alpha}$-Benzyloxycarbonyl-N$^{6}$-triphenylmethyl)-L-glutamyl)-(N$^{6}$-(2,2,5,7,8-pentamethylchroman-6-sulfonyl)-L-arginyl)-aminomethyl)-10-hexyl-3,7-dimethoxy-phenothiazin-4-yl]-essigsäurebenzylester erhalten; MS: 1447.6 MH.

b) 275 mg der erhaltenen Verbindung wurden wie in Beispiel 2.2.1. weiterverarbeitet, wobei aber die chromatographische Reinigung mit Trifluoressigsäure/Wasser erfolgte. Es wurden 46 mg 10-Hexyl-3,7-dimethoxy-4,6-cyclo-[-acetyl-L-glutaminyl-L-arginylaminomethyl-]phenothiazin-trifluoracetat (1:1) erhalten; MS: 697.2 MH.

Beispiel 4.7.3.

a) Analog wie in Beispiel 2.2.2. beschrieben wurde das geschützte Tetrapeptid Z-Val-Arg(Pmc)-Lys(Boc)-Lys(Boc)-OH durch Festphasensynthese hergestellt. MS: 1130.6 MH$^{+}$.

b) 260 mg (6-Aminomethyl-3,7-dimethoxy-10-hexyl-phenothiazin-4-yl)-essigsäurebenzylester und 572 mg Z-Val-Arg(Pmc)-Lys(Boc)-Lys(Boc)-OH wurden analog wie in Beispiel 2.2.2. beschrieben mit TBTU behandelt. Es wurden 735 mg [6-(((((N-Benzyloxycarbonyl-L-valyl-(N$^{6}$-(2,2,5,7,8-pentamethylchroman-6-sulfonyl)-L-arginyl-(N$^{6}$-(tert.-butoxycarbonyl)-L-lysyl-(N$^{6}$(tert.-butoxycarbonyl)-L-lysyl)-aminomethyl)-10-hexyl-3,7-dimethoxyphenothiazin-4-yl]-essigsäurebenzylester erhalten; MS: 1334 MH$^{+}$.

c) 650 mg der erhaltenenVerbindung wurden analog wie in Beispiel 2.2.1. beschrieben weiterverarbeitet. Das Produkt wurde in einer Dowex 44 Säule in der Acetatform umgesalzen. Es wurden 20 mg 10-Hexyl-3,7-dimethoxy-4,6-cyclo[-acetyl-L-valyl-L-arginyl-L-lysyl-L-lysyl-aminomethyl-]phenothiazin-acetat (1:3) erhalten; MS: 924.8 MH$^{+}$.

Beispiel 4.7.4.

Aus 222 mg (6-Aminomethyl-3,7-dimethoxy-10-hexyl-phenothiazin-4-yl)-essigsäurebenzylester und 362 mg $Z_3$-Arg-Gly-Asp(OBut)-Val-OH wurden analog wie in Beispiel 2.2.2. beschrieben, 28 mg 10-Hexyl-3,7-dimethoxy-4,6-cyclo[-acetyl-L-arginyl-glycyl-L-aspartyl-L-valyl-aminomethyl-]-phenothiazin-trifluoracetat (1:1) erhalten; MS: 840 MH$^{+}$.

Beispiel 4.7.5.

a) Analog wie in Beispiel 4.7.1. Absatz b) beschrieben wurden 280 mg 6-(Fmoc-Arg(Pmc)-aminomethyl)-3,7-dimethoxy-10-hexylphenothiazin-4-yl]essigsäurebenzylester mit Piperidin behandelt, worauf unter Verwendung von 89 mg Z-Ser(But)-OH, 97 mg TBTU und 0.1 ml DIPEA analog wie in Beispiel 4.7.1. Absatz b) beschrieben verfahren wurde. Es wurden 240 mg [6-(((N-tert.-Butoxycarbonyl-O-tert.-butyl)-L-seryl-(N$^{6}$-(2,2,5,7,8-pentamethylchroman-6-sulfonyl)-L-arginyl)-aminomethyl)-10-hexyl-3,7-dimethoxy-phenothiazin-4-yl]-essigsäurebenzylester erhalten; MS: 1220.6, $MH^+$.
b) 220 mg der erhaltenen Verbindung wurden analog wie in Beispiel 2.2.1. beschrieben weiterverarbeitet, wobei 57 mg 10-Hexyl-3,7-dimethoxy-4,6-cyclo-[-acetyl-L-seryl-L-arginyl-aminomethyl-]phenothiazin-trifluoracetat (1:1) erhalten wurden; MS: 656.6, $MH^+$.

Beispiel 4.7.6.

a) 250 mg (6-Aminomethyl-3,7-dimethoxy-10-hexyl-phenothiazin-4-yl)essigsäurebenzylester-trifluoracetat (1:1) wurden analog wie in Beispiel 4.7.1. beschrieben mit 153 mg Fmoc-Ser(But)-OH, 141 mg TBTU und 0.15 ml DIPEA umgesetzt. Es wurden 200 mg [6-((N-(9H-Fluoren-9-ylmethoxycarbonyl)-O-tert.-butyl-L-seryl)-aminomethyl)-10-hexyl-3,7-dimethoxyphenothiazin-4-yl]-essigsäurebenzylester erhalten; MS: 886.6 $MH^{\oplus}$.
b) 195 mg der erhaltenen Verbindung wurden analog wie in Beispiel 4.7.1. beschrieben mit Piperidin behandelt und mit 126 mg Z-Arg(Pmc)-OH, 80 mg TBTU und 0.087 ml DIPEA umgesetzt. Es wurden 167 mg 10-Hexyl-3,7-dimethoxy-4,6-cyclo[-acetyl-L-arginyl-L-seryl-aminomethyl-]phenothiazintrifluoracetat (1:1) erhalten; MS: 1220.5 $MH^+$.

Beispiel 4.7.7.

250 mg (6-Aminomethyl-3,7-dimethoxy-10-hexylphenothiazin-4-yl)essigsäurebenzylester-trifluoracetat wurden analog wie in Beispiel 4.7.1. beschrieben mit 244 mg Fmoc-Gln(Trt)-OH, 141 mg TBTU und 0.152 ml DIPEA umgesetzt. Der erhaltene [6-(($N^{\alpha}$(9H-Fluoren-9-ylmethoxycarbonyl)-$N^5$-triphenylmethyl-L-glutaminyl)-aminomethyl)-10-hexyl-3,7-dimethoxyphenothiazin-4-yl]-essigsäurebenzylester wurde analog wie in Beispiel 4.7.1. beschrieben mit Piperidin behandelt und mit 184 mg Z-Arg(Pmc)-OH, 113 mg TBTU und 0.121 ml DIPEA umgesetzt, wobei 22 mg 10-Hexyl-3,7-dimethoxy-4,6-cyclo[-acetyl-L-arginyl-L-glutaminyl-aminomethyl-]pheno thiazin-trifluoracetat (1:1) erhalten wurden; MS: 697.2 $MH^{\oplus}$.

Beispiel 4.7.8.

72.8 mg (6-Aminomethyl-3,7-dimethoxy-10-hexylphenothiazin-4-yl)essigsäurebenzylester-trifluoracetat wurden analog wie in Beispiel 4.7.1. beschrieben mit 39.7 mg Boc-Phe-OH umgesetzt. Der erhaltene kristalline [6-((N-tert.-Butoxycarbonyl-L-phenylalanyl)-aminomethyl)-10-hexyl-3,7-dimethoxy-phenothiazin-4-yl]-essigsäurebenzylester wurde in Trifluoressigsäure gelöst, worauf die Lösung 10 Minuten bei 20° stehen gelassen und im Vakuum eingeengt wurde. Der Rückstand wurde in 3 ml DMF gelöst, worauf 84.3 mg Z-Tyr(Bzl)-ONp zugegeben wurden und der pH-Wert mit DIPEA auf 8.5 eingestellt wurde. Nach 3 Stunden wurde das Reaktionsgemisch wie in Beispiel 4.7.1. beschrieben aufgearbeitet. Der erhaltene kristalline [6-((N,O-Bis-benzyloxycarbonyl-L-tyrosinyl)-L-phenylalanyl)aminomethyl)-10-hexyl-3,7-dimethoxy-phenothiazin-4-yl]-essigsäurebenzylester wurde analog wie in Beispiel 2.2.1. beschrieben weiter verarbeitet, wobei das Rohprodukt aus Aethanol kristallisiert wurde. Es wurden 21.3 mg 10-Hexyl-3,7-dimethoxy-4,6-cyclo[-acetyl-L-tyrosyl-L-phenylalanyl-aminomethyl-]phenothiazin erhalten; MS: 723.5 $MH^+$.

Beispiel 4.8.1.

Zu einer Lösung von 220 mg (0.409 mMol) (10-Benzyl-6-tert.-butoxycarbonylaminomethyl-3,7-dimethoxy-phenothiazin-4-yl)-essigsäure und 94.5 mg (0.45 mMol) Alanyl-alanin-methylester-hydrochlorid in 10 ml N,N-Dimethylformamid wurden unter Eiskühlung 170.6 mg (0.45 mMol) 1-Benzotriazol-1-yl-N,N,N',N'-tetramethyluronium-hexafluorphosphat (HBTU) und 103.4 mg (1.02 mMol) N-Methylmorpholin zugegeben. Das Reaktionsgemisch wurde 1 Stunde bei Raumtemperatur gerührt und dann auf Eiswasser und Methylenchlorid gegossen. Die organische Phase wurde abgetrennt, mit gesättigter Natriumchlorid-Lösung extrahiert, über Magnesiumsulfat getrocknet und eingeengt. Der Rückstand wurde am Hochvakuum getrocknet und in 10 ml Tetrahydrofuran/Methanol/Wasser (3:1:1) gelöst, worauf die Lösung bei 0° mit 50 mg Lithiumhydroxid versetzt und 1 Stunde bei Raumtemperatur gerührt wurde. Das Gemisch wurde auf Eiswasser gegossen, worauf mit wässriger Salzsäure angesäuert und mit Methylenchlorid extrahiert wurde. Die organische Phase wurde mit gesättigter Kochsalzlösung gewaschen, über Magnesiumsulfat getrocknet und eingeengt. Der Rückstand wurde am Hochvakuum getrocknet und in 2 ml Methylenchlorid gelöst, worauf die Lösung bei 0° mit 2 ml kalter Trifluoressigsäure versetzt, 30 Minuten gerührt und zur Trockene eingedampft wurde. Der Rückstand wurde getrocknet und in 20 ml N,N-Dimethylformamid gelöst, worauf die Lösung bei 0° mit 132.5 mg (0.48 mMol) Diphenylphosphorylazid (DPPA) und 134.4 mg (1.60 mMol)

Natriumhydrogencarbonat versetzt wurde. Das Reaktionsgemisch wurde 1 Stunde bei 0° gerührt und nach Abdestillieren des Lösungsmittels auf Wasser und Chloroform gegossen. Die organische Phase wurde abgetrennt, über Magnesiumsulfat getrocknet und eingeengt. Der Rückstand wurde an Kieselgel mit Chloroform/Methanol (9:1) chromatographiert, worauf 180 mg (78%) 10-Benzyl-3,7-dimethoxy-4,6-cyclo-[acetyl-L-alanyl-L-alanylaminomethyl]phenothiazin als farbloser Festkörper erhalten wurden.
MS (FAB): 561 ($M^+$ + H, 20), 560 ($M^+$, 8), 469 (30), 327 (30), 276 (30), 365 (30), 250 (100), 221 (20), 197 (20), 181 (20), 149 (20).

Beispiel 4.8.2.

Analog wie in Beispiel 2.2.2. beschrieben wurde an einem 4-(4-Hydroxymethyl-3-methoxyphenoxy)-butyryl-aminobenzyl-polystyrolharz das Peptidharz-Derivat von H-Leu-DTrp-DAsp(OBzl) hergestellt. 250 mg dieses Peptidharzes wurden mit 80 mg (10-Benzyl-6-tert.-butoxycarbonylaminomethyl-3,7-dimethoxy-phenothiazin-4-yl)-essigsäure in Gegenwart von 96 mg TBTU und 0.1 ml DIPEA gekuppelt. Das erhaltene modifizierte Harz wurde in 8 ml Essigsäure/2 ml $H_2O$ suspendiert, worauf die Suspension 3 Stunden bei 60° stehen gelassen und dann filtriert wurde. Das Filtrat wurde lyophilisiert und das erhaltene Produkt (49 mg) wurde in 5 ml Trifluoressigsäure/0.5 ml $H_2O$ gelöst, worauf die Lösung 15 Minuten bei 20° stehen gelassen und dann eingedampft wurde. Der Rückstand wurde analog wie in Beispiel 2.2.1. beschrieben mit HBTU und DIPEA in N,N-Dimethylformamid cyclisiert. Das Produkt wurde in 4 ml Tetrahydrofuran/Methanol (1:1) gelöst; die Lösung wurde zwecks Verseifung mit 0.4 ml 1N LiOH versetzt, dann mit verd. $H_2SO_4$ angesäuert und schliesslich mit Essigsäureäthylester extrahiert. Die organischen Phasen wurden eingedampft, und der Rückstand wurde durch HPLC gereinigt. Es wurden 8 mg 10-Benzyl-3,7-dimethoxy-4,6-cyclo[-acetyl-L-leucyl-D-tryptophyl-D-aspartyl-aminomethyl-]phenothiazin erhalten; MS: 833.2 $MH^+$.

Beispiel 4.8.3.

a) Zu einer Lösung von 650 mg (1.21 mMol) (10-Benzyl-6-tert.-butoxycarbonylaminomethyl-3,7-dimethoxy-phenothiazin-4-yl)-essigsäure, 413.5 mg (1.37 mMol) Gly-Gly-Ala-Gly-methylester-hydrochlorid und 504.5 mg (1.33 mMol) 1-Benzotriazo-1-yl-N,N,N',N'-tetramethyluronium-hexyfluorphosphat (HBTU) in 12 ml N,N-Dimethylformamid wurden bei 0° 306 mg (3.02 mMol) 4-Methylmorpholin zugegeben. Das Reaktionsgemisch wurde 1 Stunde bei Raumtemperatur gerührt, worauf das Lösungsmittel am Vakuum abdestilliert wurde und der Rückstand in Methylenchlorid und gesättigter Natriumhydrogencarbonat-Lösung aufgenommen wurde. Die wässrige Phase wurde 2 mal mit Methylenchlorid extrahiert; die vereinigten organischen Phasen wurden über Magnesiumsulfat getrocknet und eingedampft. Der Rückstand wurde an Kieselgel mit Chloroform/Methanol (9:1) chromatographiert, worauf nach Trocknung 790 mg (82.9%) (10-Benzyl-6-tert.-butoxycarbonylaminomethyl-3,7-dimethoxy-phenothiazin-4-yl)-acetyl-glycyl-glycyl-L-alanyl-glycin-methylester als amorpher Schaum erhalten wurden.
MS (FAB): 792 ($M^+$ + H, 20), 791 ($M^+$, 20), 701 (25), 692 (100). IR (KBr): 3294m, 3086w, 3084w, 2975w, 1742w, 1698m, 1664s, 1632s, 1519s, 1463s, 1366m, 1259s, 1166m, 1049m, 801w.
b) Zu einer Lösung von 710 mg (0.895 mMol) der obigen Verbindung in 20 ml Methanol wurden unter Eiskühlung 20 ml 2N wässrige Natronlauge zugetropft. Das Reaktionsgemisch wurde 1 Stunde bei Raumtemperatur gerührt und dann auf Eiswasser gegossen, worauf mit wässriger Salzsäure auf pH 3 angesäuert und mit Methylenchlorid erschöpfend extrahiert wurde. Die vereinigten organischen Phasen wurden über Magnesiumsulfat getrocknet und eingedampft. Der Rückstand wurde am Hochvakuum getrocknet und in 8 ml kalter Trifluoressigsäure gelöst, worauf die Lösung während 30 Minuten bei 0° gerührt und dann am Hochvakuum eingeengt wurde. Der Rückstand wurde in Diäthyläther suspendiert, die Suspension wurde filtriert, und der Rückstand wurde getrocknet und in 100 ml N,N-Dimethylformamid gelöst. Die Lösung wurde mit 295 mg (3.5 mMol) getrocknetem, pulverisiertem Natriumhydrogencarbonat und dann bei 0° mit 212 mg (0.77 mMol) Diphenylphosphorylazid (DPPA) versetzt. Das Reaktionsgemisch wurde über Nacht bei 0° gerührt, das N,N-Dimethylformamid wurde abdestilliert, der Rückstand wurde in 25 ml Methanol aufgenommen und das Produkt wurde durch Zugabe von 150 ml Wasser ausgefällt. Der Niederschlag wurde abfiltriert und getrocknet, wobei 380 mg (81.4%) 10-Benzyl-3,7-dimethoxy-4,6-cyclo-[-acetyl-glycyl-glycyl-L-alanylglycyl-aminomethyl-]-phenothiazin als beiger Feststoff erhalten wurden.
MS (FAB): 661 ($M^+$ + H, 12), 569 (20), 251 (45), 197 (90), 181 (70), 165 (55), 147 (70), 105 (100).

Beispiel 4.9.1.

a) Zu einer Lösung von 430 mg (0.66 mMol) 5-(4-Benzyloxycarbonylmethyl-6-tert.-butoxycarbonylaminomethyl-3,7-dimethoxy-phenothiazin-10-yl)-5-oxopentansäure in 15 ml N,N-Dimethylformamid wurden unter Eiskühlung 103 mg (0.726 mMol) Methyljodid und 110 mg (0.726 mMol) 1,8-Diazabicyclo[5.4.0]-undec-7-en zugegeben. Das Reaktionsgemisch wurde zuerst bei Raumtemperatur und dann bei 40° gerührt, hierauf abgekühlt und schliesslich auf Wasser und Essigsäureäthylester gegossen. Die organische Phase wurde 2 mal mit Wasser extrahiert, über Magnesiumsulfat getrocknet und eingeengt. Der Rückstand wurde an Kieselgel mit Essigsäureäthylester/Hexan (1:1) chromatographiert, worauf nach Trocknung 390 mg (88%) 5-(4-Benzyloxycarbonylmethyl-6-tert.-butoxycarbonylaminomethyl-3,7-dimethoxy-phenothiazin-10-yl)-5-oxo-pentansäure-methylester als weisser amorpher Schaum erhalten wurden. MS (FAB): 664 ($M^+$).

b) 310 mg (0.466 mMol) der erhaltenen Verbindung wurden in 5 ml Methylenchlorid gelöst. Die Lösung wurde bei 0° mit 5 ml Trifluoressigsäure versetzt und 30 Minuten gerührt, worauf das Lösungsmittel abdestilliert wurde. Der Rückstand wurde am Hochvakuum getrocknet und in 10 ml N,N-Dimethylformamid gelöst, worauf die Lösung mit 256.1 mg (0.699 mMol) N-α-Benzyloxycarbonyl-N-δ-tert.-butoxycarbonyl-L-ornithin, 125.9 mg (0.932 mMol) N-Hydroxybenzotriazol und 185 mg (0.652 mMol) N-Dimethylaminopropyl-N'-äthylcarbodiimid-hydrochlorid versetzt wurde. Das Reaktionsgemisch wurde unter Eiskühlung mit 0.36g (1.78 mMol) N-Aethyldiisopropylamin versetzt und während 18 Stunden bei 0° gerührt, worauf das N,N-Dimethylformamid am Hochvakuum abdestilliert wurde und der Rückstand in Methylenchlorid und Wasser aufgenommen wurde. Die organische Phase wurde abgetrennt, über Magnesiumsulfat getrocknet und eingedampft. Der Rückstand wurde am Hochvakuum getrocknet und in 15 ml Trifluoräthanol gelöst, worauf 200 mg Palladium auf Kohle (10%) zugegeben und während 3 Stunden bei 1.8 bar Wasserstoff hydriert wurde. Die Suspension wurde über Celite filtriert, und das Filtrat wurde eingedampft. Der Rückstand wurde getrocknet und in 180 ml N,N-Dimethylformamid gelöst. Die Lösung wurde bei 0° unter Argon mit 353 mg (0.932 mMol) 1-Benzotriazol-1-yl-N,N,N',N'-tetramethyl-uronium-hexafluorphosphat und 196 ml (2.33 mMol) Natriumhydrogencarbonat (getrocknet und pulverisiert) versetzt. Das Reaktionsgemisch wurde während 2.5 Stunden bei Raumtemperatur gerührt, worauf das N,N-Dimethylformamid am Vakuum abdestilliert wurde und der Rückstand in Wasser/Methylenchlorid aufgenommen wurde. Die organische Phase wurde abgetrennt, über Magnesiumsulfat getrocknet und eingedampft. Der Rückstand wurde an Kieselgel mit Chloroform/Methanol (18:1) chromatographiert, worauf 234 mg (75%) 5-[(S)-8-(3-tert.-Butoxycarbonylamino-propyl)-4,12-dimethoxy-7,10-dioxo-1,15-imino-6,7,8,9,10,11-hexahydro-5H-dibenzo[b,e][1,5,8]thiadiazacyclodecin-17-yl]-5-oxo-pentansäuremethylester als amorpher Schaum erhalten wurden.
MS (FAB): 671 ($M^+$ + H, 70), 571 (100).

## Patentansprüche

1. Verbindungen der allgemeinen Formel

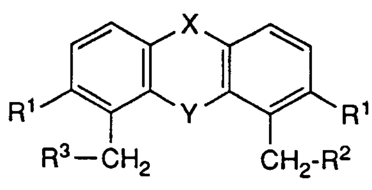

I

worin X eine Gruppe der Formel

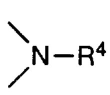

(a)

oder

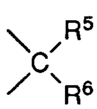

(b)

;

Y Sauerstoff oder Schwefel;

$R^1$ Wasserstoff oder niederes Alkoxy;
$R^2$ geschütztes Amino, Amino oder einen Rest der Formel -NH-$R^7$ (c);
$R^3$ Carboxyl, funktionell abgewandeltes Carboxyl oder einen Rest der Formel -CO-$R^8$ (d);
$R^4$ niederes Alkyl, Aryl, Aryl-niederes Alkyl, Wasserstoff oder Acyl;
$R^5$ und $R^6$ je niederes Alkyl, Aryl oder Aryl-niederes Alkyl; und
$R^7$ und $R^8$ je einzeln oder zusammen einen Rest einer Aminosäure oder eine Kette von bis zu 20 Aminosäureresten bedeuten, wobei der Aminosäurerest bzw. die Aminosäurereste geschützt sein kann bzw. sein können und wobei das Molekül insgesamt höchstens 20 Aminosäurereste enthält,
sowie Salze davon.

**2.** Verbindungen nach Anspruch 1, worin X eine Gruppe der Formel (a) und $R^4$ Methyl, Aethyl, Hexyl, Benzyl, Wasserstoff, 4-Methoxycarbonylbutyryl oder 4-Carboxybutyryl bedeuten.

**3.** Verbindungen nach Anspruch 2, worin $R^4$ 4-Carboxybutyryl bedeutet und $R^3$ nicht Carboxyl bedeutet.

**4.** Verbindungen nach Anspruch 1, worin X eine Gruppe der Formel (b) und $R^5$ und $R^6$ beide Methyl bedeuten.

**5.** Verbindungen nach einem der Ansprüche 1 bis 4, worin die beiden Symbole $R^1$ beide Methoxy oder beide Aethoxy bedeuten.

**6.** Verbindungen nach einem der Ansprüche 1 bis 5, worin $R^2$ einen Rest der Formel (c), $R^3$ einen Rest der Formel (d) und $R^7$ und $R^8$ zusammen einen Rest einer Aminosäure oder eine Kette von bis zu 20 Aminosäureresten bedeuten.

**7.** Verbindungen nach Anspruch 6, worin $R^7$ und $R^8$ zusammen einen Rest einer Aminosäure oder eine Kette von bis zu 14 Aminosäureresten bedeuten.

**8.** Verbindungen nach Anspruch 6 oder 7, worin der Aminosäurerest bzw. die Kette von Aminosäureresten keine Schutzgruppe(n) enthält.

**9.** Verbindungen nach Anspruch 8, enthaltend als Aminosäurerest bzw. als Kette von Aminosäureresten

-Arg-

-Ala-

-Ala-Ala-

-Arg-Gln-

-Arg-Ser-

-Gln-Arg-

-Glu-Arg-

-Lys-Glu-

-Ser-Arg-

-Thr-Gly-

-Tyr-Phe-

-Leu-D-Try-D-Asp-

-Gly-Arg-Gly-

-Ile-Tyr-Ala-

-Leu-Tyr-Asp-

-Ala-Thr-Val-Gly-

-Arg-Gly-Asp-Val-

-Gly-Asp-Gly-Gly-

-Gly-Gly-Ala-Gly-

-Val-Arg-Lys-Lys-

-Ala-Arg-Gly-Asp-Phe-Pro-

-Glu-Arg-Gly-Asp-Val-Tyr-

-Ile-Ala-Arg-Gly-Asp-Phe-Pro-Asp-

-Val-Ala-Ala-Phe-Leu-Ala-Leu-Ala-

-Arg-Ile-Ala-Arg-Gly-Asp-Phe-Pro-Asp-Asp-

-Ala-Arg-Ile-Ala-Arg-Gly-Asp-Phe-Pro-Asp-Asp-Arg-

**10.** 4,5-Cyclo[-acetyl-L-alanyl-L-arginyl-L-isoleucyl-L-alanyl-L-arginylglycyl-L-aspartyl-L-phenylalanyl-L-proyly-L-aspartyl-L-aspartyl-L-arginylaminomethyl-]-3,6-dimethoxy-9,9-dimethylxanthen.

**11.** 4,5-Cyclo[-acetyl-L-arginyl-L-isoleucyl-L-alanyl-L-arginyl-glycy-L-aspartyl-L-phenylalanyl-L-prolyl-L-aspartyl-L-aspartylaminomethyl-]-3,6-dimethoxy-9,9-dimethylxanthen.

**12.** 4,5-Cyclo[-acetyl-L-isoleucyl-L-alanyl-L-arginyl-glycyl-L-aspartyl-L-phenylalanyl-L-prolyl-L-aspartylaminomethyl-]-3,6-dimethoxy-9,9-dimethylxanthen.

**13.** 4,5-Cyclo[-acetyl-L-alanyl-L-arginyl-glycyl-L-aspartyl-L-phenylalanyl-L-prolylaminomethyl-]-3,6-dimethoxy-9,9-dimethylxanthen.

**14.** 4,5-Cyclo[-acetyl-L-arginyl-glycyl-L-aspartyl-L-phenylalanylaminomethyl-]-3,6-dimethoxy-9,9-dimethylxanthen.

**15.** 4,5-Cyclo[-acetyl-L-arginyl-glycyl-L-aspartyl-L-valylaminomethyl-]-3,6-dimethoxy-9,9-dimethylxanthen.

**16.** 10-Methyl-4,6-cyclo[-acetyl-L-arginyl-glycyl-L-aspartyl-L-valylaminomethyl-]phenothiazin.

**17.** 4,6-Cyclo[-acetyl-L-arginyl-glycyl-L-aspartyl-L-valylaminomethyl-]-3,7-diäthoxy-10-aethyl-10H-dibenz-[b,e][1,4] oxazin.

**18.** 10-Hexyl-3,7-dimethoxy-4,6-cyclo[-acetyl-L-arginyl-glycyl-L-aspartyl-L-valylaminomethyl-]phenothiazin.

**19.** 3,7-Dimethoxy-10-methyl-4,6-cyclo[-acetyl-L-arginyl-glycyl-L-aspartyl-L-valylaminomethyl-]phenothiazin.

**20.** (S)-4,12-Dimethoxy-8-(3-guanidinopropyl)-17-methyl-1,15-imino-6,7,8,9,10,11-hexahydro-5H-dibenzol-[b,k][1,5,8]thiadiazacyclodecin-7,10-dion;

(S)-8-(3-Guanidinopropyl)-17-hexyl-1,15-imino-6,7,8,9,10,11-hexahydro-5H-dibenzo[b,k][1,5,8]-thiadiazacyclodecin-7,10-dion;

(S)-8-(3-Guandinopropyl)-17-methyl-1,15-imino-6,7,8,9,10,11-hexahydro-5H-dibenzo[b,k][1,5,8]-thiadiazacyclodecin-7,10-dion;

10-Hexyl-3,7-dimethoxy-4,6-cyclo[-acetyl-L-glutaminyl-L-arginylaminomethyl-]phenothiazin;

10-Hexyl-3,7-dimethoxy-4,6-cyclo[acetyl-L-arginyl-L-glutaminylaminomethyl-]phenothiazin;

10-Hexyl-3,7-dimethoxy-4,6-cyclo[-acetyl-L-seryl-L-arginylaminomethyl]-phenothiazin;

10-Hexyl-3,7-dimethoxy-4,6-cyclo[-acetyl-L-arginyl-L-serylaminomethyl]-phenothiazin; und

10-Hexyl-3,7-dimethoxy-4,6-cyclo[-acetyl-glycyl-L-arginyl-glycylaminomethyl-]phenothiazin.

**21.** Verbindungen der Formeln

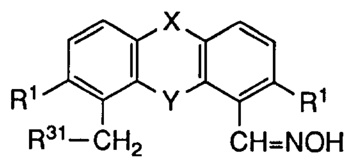
II

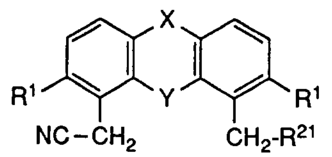
III

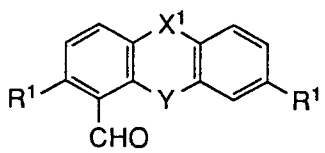
V

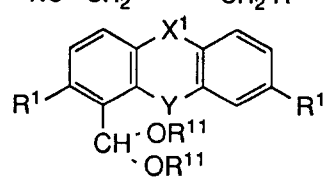
VI

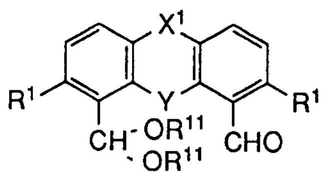
VII

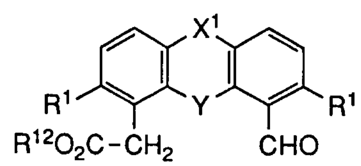
VIII

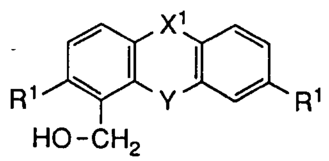
IX

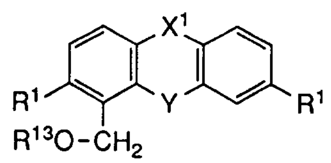
X

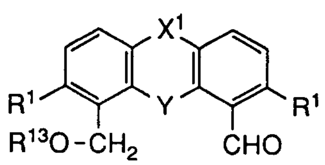
XI

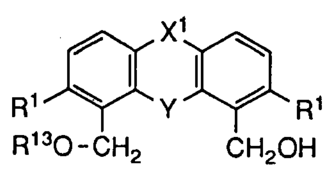
XII

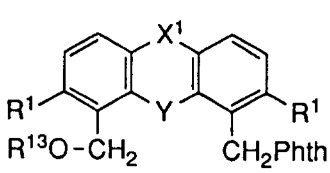
XIII

worin X, Y und $R^1$ die in Anspruch 1 angegebene Bedeutung besitzen; $X^1$ einen Rest der in Anspruch 1 definierten Formel (a), worin $R^4$ niederes Alkyl, Aryl oder Aryl-niederes Alkyl bedeutet, oder einen Rest der in Anspruch 1 definierten Formel (b) bedeutet; die beiden Symbole $R^{11}$ je niederes Alkyl oder zusammen niederes Alkylen bedeuten; $R^{12}$ niederes Alkyl, Aryl oder Aryl-niederes Alkyl bedeutet; $R^{13}$ eine Schutzgruppe bedeutet; Phth die Phthalimidgruppe bedeutet; $R^{21}$ geschütztes Amino oder Amino bedeutet; und $R^{31}$ Carboxyl oder funktionell abgewandeltes Carboxyl bedeutet.

**22.** Verbindungen nach Anspruch 21, worin $R^{13}$ tert.-Butyldimethylsilyl, tert.-Butyldiphenylsilyl oder 2-Methoxyaethoxymethyl bedeutet.

**23.** Verbindungen nach einem der Ansprüche 6 bis 20 oder pharmazeutisch anwendbare Salze davon zur Anwendung als therapeutische Wirkstoffe.

**24.** Verfahren zur Herstellung von Verbindungen der in Anspruch 1 definierten Formel I und von Salzen davon, dadurch gekennzeichnet, dass man

a) eine Verbindung der allgemeinen Formel

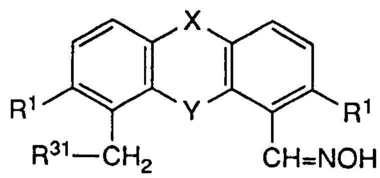

II

worin X,Y und $R^1$ die in Anspruch 1 angegebene Bedeutung besitzen und $R^{31}$ Carboxyl oder funktionell abgewandeltes Carboxyl bedeutet,

reduziert; oder

b) eine Verbindung der allgemeinen Formel

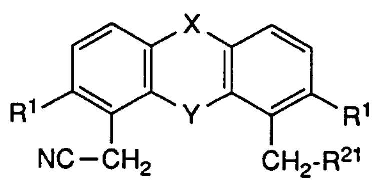

III

worin X,Y und $R^1$ die in Anspruch 1 angegebene Bedeutung besitzen und $R^{21}$ geschütztes Amino oder Amino bedeutet,

hydrolysiert; oder

c) in einer Verbindung der allgemeinen Formel

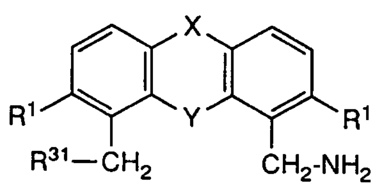

Ib

worin X,Y und $R^1$ die in Anspruch 1 angegebene und $R^{31}$ obige Bedeutung besitzen,

die Aminogruppe in eine geschützte Aminogruppe überführt; oder

d) in einer Verbindung der allgemeinen Formel

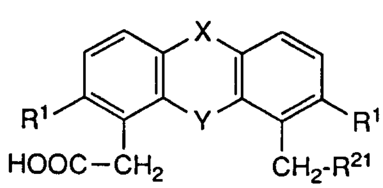

Ic

worin X,Y und $R^1$ die in Anspruch 1 angegebene und $R^{21}$ obige Bedeutung besitzen,

die Carboxylgruppe in eine funktionell abgewandelte Carboxylgruppe überführt; oder

e) in einer Verbindung der Formel

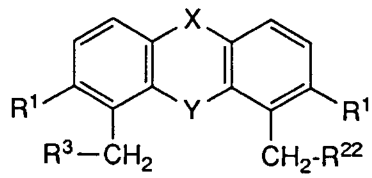

Id

worin X,Y,$R^1$ und $R^3$ die in Anspruch 1 angegebene Bedeutung besitzen und $R^{22}$ geschütztes Amino bedeutet,

die Schutzgruppe abspaltet; oder

f) in einer Verbindung der allgemeinen Formel

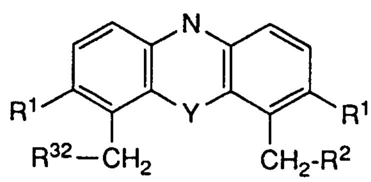

Ie

worin X,Y,$R^1$ und $R^2$ die in Anspruch 1 angegebene Bedeutung besitzen und $R^{32}$ funktionell abgewandeltes Carboxy bedeutet,

die funktionell abgewandelte Carboxylgruppe in die Carboxylgruppe überführt; oder

g) aus einer Verbindung der allgemeinen Formel

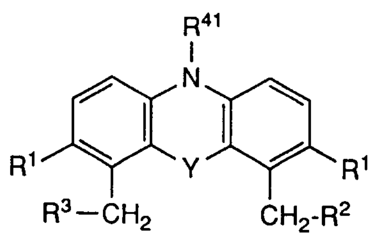

If

worin Y,$R^1$,$R^2$ und $R^3$ die in Anspruch 1 angegebene Bedeutung besitzen und $R^{41}$ eine abspaltbare Arakylgruppe bedeutet,

die abspaltbare Gruppe entfernt; oder

h) eine Verbindung der allgemeinen Formel

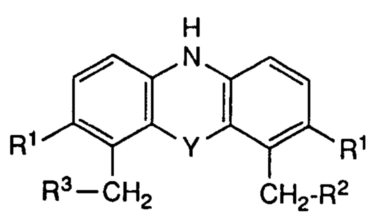

Ig

worin Y,$R^1$, $R^2$ und $R^3$ die in Anspruch 1 angegebene Bedeutung besitzen,

acyliert und erwünschtenfalls eine in der eingeführten Acylgruppe vorhandene Carboxylgruppe verestert; oder

i) eine Verbindung der allgemeinen Formel

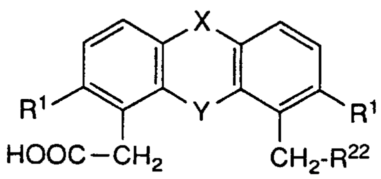

Ih

worin X,Y und $R^1$ die in Anspruch 1 angegebene und $R^{22}$ obige Bedeutung besitzen,

mit einer gegebenenfalls geschützten Aminosäure oder mit einer gegebenenfalls geschützten Kette von bis zu 20 Aminosäuren kuppelt; oder

j) eine Verbindung der allgemeinen Fomel

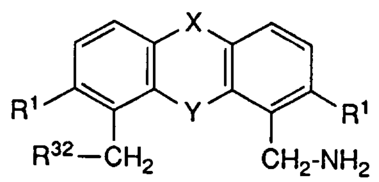

Ii

worin X,Y und $R^1$ die in Anspruch 1 angegebene und $R^{32}$ obige Bedeutung besitzen,

mit einer gegebenenfalls geschützten Aminosäure oder mit einer gegebenenfalls geschützten Kette von bis zu 20 Aminosäuren kuppelt; oder

k) eine Verbindung der allgemeinen Formel

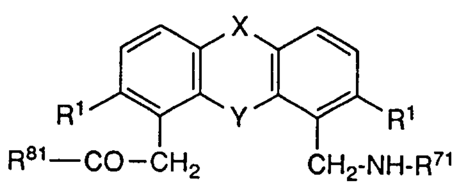

Ij

worin X,Y und $R^1$ die in Anspruch 1 angegebene Bedeutung besitzen und $R^{71}$ Wasserstoff, einen gegebenenfalls geschützten Rest einer Aminosäure oder eine gegebenenfalls geschützte Kette von Aminosäureresten und $R^{81}$ Wasserstoff, einen gegebenenfalls geschützten Rest einer Aminosäure oder eine gegebenenfalls geschütze Kette von Aminosäureresten bedeuten, wobei mindestens eines von $R^{71}$ und $R^{81}$ von Wasserstoff verschieden ist,

mit einer gegebenenfalls geschützten Aminosäure oder mit einer gegebenenfalls geschützten Kette von Aminosäuren kuppelt, wobei in den beiden Reaktionskomponenten insgesamt höchstens 20 Aminosäurereste vorhanden sind; oder

l) eine Verbindung der allgemeinen Formel

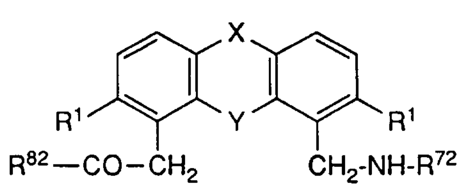

Ik

worin X,Y und $R^1$ die in Anspruch 1 angegebene Bedeutung besitzen und $R^{72}$ Wasserstoff, einen gegebenenfalls geschützten Rest einer Aminosäure oder eine gegebenenfalls geschützte Kette von bis zu 20 Aminosäureresten und $R^{82}$ Wasserstoff, einen gegebenenfalls geschützten Rest einer Aminosäure oder eine gegebenenfalls geschützte Kette von bis zu 20 Aminosäureresten bedeuten, wobei das Molekül mindestens einen und höchstens 20 Aminosäurereste enthält,

cyclisiert; oder

m) aus einer Verbindung der Formel I, welche mindestens einen geschützten Aminosäurerest enthält, die Schutzgruppe(n) abspaltet; oder

n) eine Verbindung der Formel I, welche ein basisches Zentrum enthält, mittels einer Säure bzw. eine Verbindung der Fomel I, welche ein saures Zentrum enthält, mittels einer Base in ein Salz überführt.

**25.** Arzneimittel, enthaltend eine Verbindung nach einem der Ansprüche 6 bis 20 oder ein pharmazeutisch anwendbares Salz davon und einen pharmazeutisch anwendbaren Hilfsstoff.

**26.** Verwendung von Verbindungen nach einem der Ansprüche 6 bis 20 oder von Salzen davon als "Research Tools" zur Ermittlung von biologisch aktiven Peptidsequenzen.

**27.** Verwendung von Verbindungen nach einem der Ansprüche 6 bis 20 oder von pharmazeutisch anwendbaren Salzen davon als Arzneimittel bzw. zur Herstellung von Arzneimitteln.

**28.** Verwendung von Verbindungen nach einem der Ansprüche 10 bis 18 zur Verhinderung der Entstehung von Blutplättchenthromben bzw. zur Herstellung entsprechender Arzneimittel.